# EUROPEAN PATENT SPECIFICATION

(11) **EP 1 689 752 B1**
(45) Date of publication and mention of the grant of the patent: **27.02.2008**
(21) Application number: 04798915.7
(22) Date of filing: 19.11.2004
(51) Int. Cl.: C07D 487/04, A61K 31/395, A61K 31/505, A61P 9/12

(54) **PYRAZOLOPYRIMIDINES**
PYRAZOLOPYRIMIDINE
PYRAZOLOPYRIMIDINES

(30) Priority: 24.11.2003 GB 0327323
(43) Date of publication of application: 16.08.2006
(73) Proprietor: Pfizer Limited, Sandwich, Kent CT13 9NJ (GB); Pfizer, Inc., New York, NY 10017 (US)
(72) Inventor: BELL,Andrew, Pfizer Global Research & Development, Sandwich, Kent CT13 9NJ (GB); BROWN, David, Pfizer Global Research & Development, Sandwich, Kent CT13 9NJ (GB); BULL, David, Pfizer Global Research & Development, Sandwich, Kent CT13 9NJ (GB); FOX, David, Pfizer Global Research & Development, Sandwich, Kent CT13 9NJ (GB); MARSH, Ian, Pfizer Global Research & Development, Sandwich, Kent CT13 9NJ (GB); MORRELL, Andrew, Pfizer Global Research & Developm, Sandwich, Kent CT13 9NJ (GB); PALMER, Michael, Pfizer Global Research & Developm, Sandwich, Kent CT13 9NJ (GB); WINSLOW, Carol, Pfizer Global Research & Developm, Sandwich, Kent CT13 9NJ (GB)
(74) Representative: Ruddock, Keith Stephen
(86) International application number: PCT/IB2004/003791
(87) International publication number: WO 2005/049617

(56) References cited:
- EP-A- 1 348 707
- WO-A-02/00660
- WO-A-02/102314
- WO-A-20/04096810
- US-A- 6 130 223
- DUMAITRE B ET AL: "SYNTHESIS AND CYCLIC GMP PHOSPHODIESTERASE INHIBITORY ACTIVITY OF A SERIES OF 6-PHENYLPYRAZOLO 3,4-DPYRIMIDONES" JOURNAL OF MEDICINAL CHEMISTRY, AMERICAN CHEMICAL SOCIETY. WASHINGTON, US, vol. 39, no. 8, 1996, pages 1635-1644, XP000651134 ISSN: 0022-2623

## Description

The present invention relates to a series of novel 5,7-diaminopyrazolo[4,3-*d*] pyrimidines, which are cyclic guanylate monophosphate (cGMP)-specific phosphodiesterase type 5 inhibitors (hereinafter referred to as PDE-5 inhibitors) that are useful in the treatment of hypertension and other disorders, to processes for their preparation, intermediates used in their preparation, to compositions containing them and the uses of said compounds and compositions.

### i) Hypertension

Blood pressure (BP) is defined by a number of haemodynamic parameters taken either in isolation or in combination. Systolic blood pressure (SBP) is the peak arterial pressure attained as the heart contracts. Diastolic blood pressure is the minimum arterial pressure attained as the heart relaxes. The difference between the SBP and the DBP is defined as the pulse pressure (PP).

Hypertension, or elevated BP, has been defined as a SBP of at least 140mmHg and/or a DBP of at least 90mmHg. By this definition, the prevalence of hypertension in developed countries is about 20% of the adult population, rising to about 60-70% of those aged 60 or more, although a significant fraction of these hypertensive subjects have normal BP when this is measured in a non-clinical setting. Some 60% of this older hypertensive population have isolated systolic hypertension (ISH), i.e. they have an elevated SBP and a normal DBP.
Hypertension is associated with an increased risk of stroke, myocardial infarction, atrial fibrillation, heart failure, peripheral vascular disease and renal impairment (Fagard, RH; Am. J. Geriatric Cardiology 11(1), 23-28, 2002; Brown, MJ and Haycock, S; Drugs 59(Suppl 2), 1-12, 2000).

The pathophysiology of hypertension is the subject of continuing debate. While it is generally agreed that hypertension is the result of an imbalance between cardiac output and peripheral vascular resistance, and that most hypertensive subjects have abnormal cardiac output and increased peripheral resistance there is uncertainty which parameter changes first (Beevers, G et al.; BMJ 322, 912-916, 2001).

Despite the large number of drugs available in various pharmacological categories, including diuretics, alpha-adrenergic antagonists, beta-adrenergic antagonists, calcium channel blockers, angiotensin converting enzyme (ACE) inhibitors and angiotensin receptor antagonists, the need for an effective treatment of hypertension is still not satisfied.

### ii) PDE5 inhibitors

Vascular endothelial cells secrete nitric oxide (NO). This acts on vascular smooth muscle cells and leads to the activation of guanylate cyclase and the accumulation of cyclic guanosine monophosphate (cGMP). The accumulation of cGMP causes the muscles to relax and the blood vessels to dilate. This dilation reduces vascular resistance and so leads to a reduction in blood pressure.

The cGMP is inactivated by hydrolysis to guanosine 5'-monophosphate (GMP) by a cGMP-specific phosphodiesterase. One important phosphodiesterase has been identified as Phosphodiesterase type 5 (PDE5). Inhibitors of PDE5 decrease the rate of hydrolysis of cGMP and so potentiate the actions of nitric oxide.

Inhibitors of PDE5 have been reported in several chemical classes, including: pyrazolo[4,3-d]pyrimidin-7-ones (e.g. published international patent applications WO 93/06104, WO 98/49166, WO 99/54333, WO 00/24745, WO 01/27112 and WO 01/27113); pyrazolo[3,4-d]pyrimidin-4-ones (e.g. published international patent application WO 93/07149); pyrazolo[4,3-d]pyrimidines (e.g. published international patent application WO 01/18004); quinazolin-4-ones (e.g. published international patent application WO 93/12095); pyrido[3,2-d]pyrimidin-4-ones (e.g. published international patent application WO 94/05661); purin-6-ones (e.g. published international patent application WO 94/00453); hexahydro-pyrazino[2',1':6,1]pyrido[3,4-b]indole-1,4-diones (e.g. published international application WO 95/19978) and imidazo[5,1-f][1,2,4]triazin-ones (e.g. published international application WO 99/24433).

Although they have been suggested as agents for the treatment of related conditions such as angina, PDE5 inhibitors have not yet been adopted as agents for the treatment of hypertension. PDE5 inhibitors are known for the treatment of male erectile dysfunction, e.g. sildenafil, tadalafil and vardenafil. There remains a demand for new PDE5 inhibitors, particularly with improved pharmacokinetic and pharmacodynamic properties. The compounds provided herein are potent inhibitors of PDE5 that have improved selectivity *in vitro* and/or an extended half-life *in vivo.*

WO 02/00660 and WO 01/18004 disclose pyrazolo[4,3-d]pyrimidines with a PDE-5 inhibiting effect, which can be used for treating disorders of the cardiovascular system.

According to a first aspect, the present invention provides compounds of formula (I) 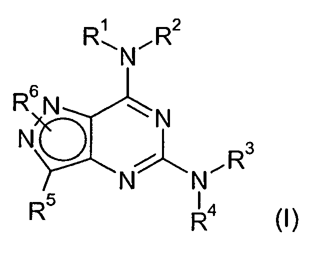 wherein
R¹ is a cyclic group selected from R^{A}, R^{B}, R^{C} and R^{D}, each of which is optionally substituted with one or more R⁷ groups;
R² is hydrogen or C₁-C₂ alkyl;
R³ and R⁴ are each independently C₁-C₈ alkyl, C₂-C₈ alkenyl, C₂-C₈ alkynyl or C₃-C₁₀ cycloalkyl, each of which is optionally substituted with one or more R⁸ groups, or R^{E}, which is optionally substituted with one or more R⁹ groups, or hydrogen;
or -NR³R⁴ forms R^{F}, which is optionally substituted with one or more R¹⁰ groups;
R⁵ is -Y-CONR¹⁵R¹⁶;
R⁶, which may be attached at N¹ or N², is C₁-C₆ alkyl, C₁-C₆ haloalkyl, C₂-C₆ alkenyl or C₂-C₆ alkynyl, each of which is optionally substituted by C₁-C₆ alkoxy, C₁-C₆ haloalkoxy or a cyclic group selected from R^{J}, R^{K}, R^{L} and R^{M}, or R⁶ is R^{N}, C₃-C₇ cycloalkyl or C₃-C₇ halocycloalkyl, each of which is optionally substituted by C₁-C₆ alkoxy or C₁-C₆ haloalkoxy, or R⁶ is hydrogen;
R⁷ is halo, C₁-C₆ alkyl, C₁-C₆ haloalkyl, C₂-C₆ alkenyl, C₂-C₆ alkynyl, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹², CONR¹²R¹³ or CN;
R⁸ is halo, phenyl, C₁-C₆ alkoxyphenyl, OR¹², OC(O)R¹², NO₂, NR¹²R¹³, NR¹²C(O)R¹³, NR¹²CO₂R¹⁴, C(O)R¹², CO₂R¹², CONR¹²R¹³, CN, R^{G} or R^{H}, the last two of which are optionally substituted with one or more R⁹ groups;
R⁹ is C₁-C₆ alkyl, C₁-C₆ haloalkyl or CO₂R¹²;
R¹⁰ is halo, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, C₁-C₆ alkyl or C₁-C₆ haloalkyl, the last two of which are optionally substituted by R¹¹;
R¹¹ is phenyl, NR¹²R¹³ or NR¹²CO₂R¹⁴;
R¹² and R¹³ are each independently hydrogen, C₁₋C₆alkyl or C₁-C₆ haloalkyl;
R¹⁴ is C₁₋C₆ alkyl or C₁-C₆ haloalkyl;
R¹⁵ and R¹⁶ are each independently selected from
   hydrogen,
   C₁-C₆ haloalkyl,
   C₁-C₆ alkyl optionally substituted with
   R¹⁷
   -NR¹⁸R¹⁹,
   -CO₂R²⁰,
   -CONR²¹R²²,
   R²³ or
   phenyl optionally substituted by
      halo,
      C₁-C₆ alkyl or
      R¹⁷
   C₃-C₇ cycloalkyl optionally substituted with
   C₁-C₆ alkyl,
   R¹⁷ or
   -NR¹⁸R¹⁹, and
   R²³;
or NR¹⁵R¹⁶ constitutes a 3- to 8-membered ring which may optionally include one or more further heteroatoms selected from nitrogen, oxygen and sulphur, and which may optionally be further substituted with R¹⁷, C₁-C₆ haloalkyl, -CO₂R¹⁰, -CONR²¹R²², oxo or C₁-C₆ alkyl optionally substituted by R¹⁷;
R¹⁷ is hydroxy, C₁-C₆ alkoxy, C₁-C₆ (haloalkyl)oxy or C₃-C₇ cycloalkyloxy;
R¹⁸ and R¹⁹ are each independently selected from hydrogen and C₁-C₆ alkyl;
or -NR¹⁸R¹⁹ constitutes an azetidine, pyrrolidine, piperidine or morpholine ring;
R²⁰ is hydrogen or C₁-C₆ alkyl;
R²¹ and R²² are each independently selected from hydrogen, C₁-C₆ alkyl, C₁-C₆ haloalkyl and C₃-C₇ cycloalkyl;
or -NR²¹R²² constitutes a 3- to 8-membered ring which may optionally include one or more further heteroatoms selected from nitrogen, oxygen and sulphur;
R²³ is a saturated 3- to 8-membered ring which includes at least one heteroatom selected from nitrogen, oxygen and sulphur, which ring may optionally be substituted by one or more C₁-C₆ alkyl groups, provided that the group R²³ is joined to the parent molecule by a covalent bond to a carbon atom of said ring;
R^{A} and R^{J} are each independently a C₃-C₁₀ cycloalkyl or C₃-C₁₀ cycloalkenyl group, each of which may be either monocyclic or, when there are an appropriate number of ring atoms, polycyclic and which may be fused to either
   (a) a monocyclic aromatic ring selected from a benzene ring and a 5- or 6-membered heteroaromatic ring containing up to three heteroatoms selected from nitrogen, oxygen and sulphur, or
   (b) a 5-, 6- or 7-membered heteroalicyclic ring containing up to three heteroatoms selected from nitrogen, oxygen and sulphur;
R^{B} and R^{K} are each independently a phenyl or naphthyl group, each of which may be fused to
   (a) a C₅-C₇ cycloalkyl or C₅-C₇ cycloalkenyl ring,
   (b) a 5-, 6- or 7-membered heteroalicyclic ring containing up to three heteroatoms selected from nitrogen, oxygen and sulphur, or
   (c) a 5- or 6-membered heteroaromatic ring containing up to three heteroatoms selected from nitrogen, oxygen and sulphur;
R^{C}, R^{L} and R^{N} are each independently a monocyclic or, when there are an appropriate number of ring atoms, polycyclic saturated or partly unsaturated ring system containing between 3 and 10 ring atoms, of which at least one is a heteroatom selected from nitrogen, oxygen and sulphur, which ring may be fused to a C₅-C₇ cycloalkyl or C₅-C₇ cycloalkenyl group or a monocyclic aromatic ring selected from a benzene ring and a 5- or 6-membered heteroaromatic ring containing up to three heteroatoms selected from nitrogen, oxygen and sulphur,
R^{D} and R^{M} are each independently a 5- or 6-membered heteroaromatic ring containing up to three heteroatoms independently selected from nitrogen, oxygen and sulphur, which ring may further be fused to
   (a) a second 5- or 6-membered heteroaromatic ring containing up to three heteroatoms selected from nitrogen, oxygen and sulphur,
   (b) C₆-C₇ cycloalkyl or C₅-C₇ cycloalkenyl ring;
   (c) a 5-, 6- or 7-membered heteroalicyclic ring containing up to three heteroatoms selected from nitrogen, oxygen and sulphur; or
   (d) a benzene ring;
R^{E}, R^{F} and R^{G} are each independently a monocyclic or, when there are an appropriate number of ring atoms, polycyclic saturated ring system containing between 3 and 10 ring atoms, of which at least one is a heteroatom selected from nitrogen, oxygen and sulphur;
R^{H} is a 5- or 6-membered heteroaromatic ring containing up to three heteroatoms independently selected from nitrogen, oxygen and sulphur, and
Y is a covalent bond, C₁-C₆ alkylenyl or C₃-C₇ cycloalkylbnyl;
a tautomer thereof or a pharmaceutically acceptable salt or solvate of said compound or tautomer.

As used herein, alkylenyl indicates an alkyl-*m*,*n*-diyl unit where m and n are the same or different, such as methylene (-CH₂-), ethylene (-CH₂CH₂-) and propane-1,2-diyl (-CH(CH₃)CH₂-).

As used herein, cycloalkylenyl indicates a cycloalkyl-*m,n*-diyl unit where m and n are the same or different, such as cyclopropane-1,1-diyl and cyclohexane-1,4-diyl.

Unless otherwise indicated, an alkyl or alkoxy group may be straight or branched and contain 1 to 8 carbon atoms, preferably 1 to 6 and particularly 1 to 4 carbon atoms. Examples of alkyl include methyl, ethyl, n-propyl, isopropyl, n-butyl, isobutyl, sec-butyl, pentyl and hexyl. Examples of alkoxy include methoxy, ethoxy, isopropoxy and n-butoxy.

Unless otherwise indicated, an alkenyl or alkynyl group may be straight or branched and contain 2 to 8 carbon atoms, preferably 2 to 6 and particularly 2 to 4 carbon atoms and may contain up to 3 double or triple bonds which may be conjugated. Examples of alkenyl and alkynyl include vinyl, allyl, butadienyl and propargyl.

Unless otherwise indicated, a cycloalkyl or cycloalkoxy group may contain 3 to 10 ring-atoms, may be either monocyclic or, when there are an appropriate number of ring atoms, polycyclic. Examples of cycloalkyl groups are cyclopropyl, cyclopentyl, cyclohexyl and adamantyl.

Unless otherwise indicated, a cycloalkenyl group may contain 3 to 10 ring-atoms, may be either monocyclic or, when there are an appropriate number of ring atoms, polycyclic and may contain up to 3 double bonds. Examples of cycloalkenyl groups are cyclopentenyl and cyclohexenyl.

Aryl includes phenyl, naphthyl, anthracenyl and phenanthrenyl.

Unless otherwise indicated, a heteroalicyclyl group contains 3 to 10 ring-atoms up to 4 of which may be hetero-atoms such as nitrogen, oxygen and sulfur, and may be saturated or partially unsaturated. Examples of heteroalicyclyl groups are oxiranyl, azetidinyl, tetrahydrofuranyl, thiolanyl, pyrrolidinyl, pyrrolinyl, imidazolidinyl, imidazolinyl, sulfolanyl, dioxolanyl, dihydropyranyl, tetrahydropyranyl, piperidinyl, pyrazolinyl, pyrazolidinyl, dioxanyl, morpholinyl, dithianyl, thiomorpholinyl, piperazinyl, azepinyl, oxazepinyl, thiazepinyl, thiazolinyl and diazapanyl.

Unless otherwise indicated, a heteroaryl group contains 3 to 10 ring-atoms up to 4 of which may be hetero-atoms such as nitrogen, oxygen and sulfur. Examples of heteroaryl groups are furyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyrimidinyl, pyrazinyl, pyridazinyl, tetrazolyl, triazinyl. In addition, the term heteroaryl includes fused heteroaryl groups, for example benzimidazolyl, benzoxazolyl, imidazopyridinyl, benzoxazinyl, benzothiazinyl, oxazolopyridinyl, benzofuranyl, quinolinyl, quinazolinyl, quinoxalinyl, benzothiazolyl, phthalimido, benzofuranyl, benzodiazepinyl, indolyl and isoindolyl.

Halo means fluoro, chloro, bromo or iodo.

Haloalkyl includes monohaloalkyl, polyhaloalkyl and perhaloalkyl, such as 2-bromoethyl, 2,2,2-trifluoroethyl, chlorodifluoromethyl and trichloromethyl. Haloalkoxy includes monohaloalkoxy, polyhaloalkoxy and perhaloalkoxy, such as 2-bromoethoxy, 2,2,2-trifluoroethoxy, chlorodifluoromethoxy and trichloromethoxy. Halocycloalkyl includes monohalocycloalkyl, polyhalocycloalkyl and perhalocycloalkyl.

Unless otherwise indicated, the term substituted means substituted by one or more defined groups. In the case where groups may be selected from a number of alternative groups, the selected groups may be the same or different.

In one preferred embodiment, R¹ is R^{A} which is optionally substituted with one or more R⁷ groups; and R^{A} is a C₃-C₁₀ cycloalkyl group, which may be either monocyclic or, when there are an appropriate number of ring atoms, polycyclic, which may be fused to either
(a) a monocyclic aromatic ring selected from a benzene ring and a 5- or 6-membered heteroaromatic ring containing up to three heteroatoms selected from nitrogen, oxygen and sulphur, or
(b) a 5-, 6- or 7-membered heteroalicyclic ring containing up to three heteroatoms selected from nitrogen, oxygen and sulphur.

Preferably, R^{A} is a monocyclic C₃-C₈ cycloalkyl group.

More preferably, R^{A} is a monocyclic C₅-C₇ cycloalkyl group.

Most preferably, R^{A} is cyclopentyl or cyclohexyl.

In another preferred embodiment, R¹ is R^{B}, which is optionally substituted with one or more R⁷ groups.

Preferably, R^{B} is phenyl.

In another preferred embodiment, R¹ is R^{C}, which is optionally substituted with one or more R⁷ groups.

Preferably, R^{C} is a monocyclic saturated or partly unsaturated ring system containing between 3 and 8 ring atoms, of which at least one is a heteroatom selected from nitrogen, oxygen and sulphur.

More preferably, R^{C} is a monocyclic saturated or partly unsaturated ring system containing between 5 and 7 ring atoms, of which at least one is a heteroatom selected from nitrogen, oxygen and sulphur.

Most preferably, R^{C} is a monocyclic saturated ring system containing between 5 and 7 ring atoms, of which at least one is a heteroatom selected from nitrogen, oxygen and sulphur.

In another preferred embodiment, R¹ is R^{D}, which is optionally substituted with one or more R⁷ groups.

Preferably, R^{D} is a 5- or 6-membered heteroaromatic ring containing up to three heteroatoms independently selected from nitrogen, oxygen and sulphur.

More preferably, R^{D} is a 5-membered heteroaromatic ring containing a heteroatom selected from nitrogen, oxygen and sulphur and optionally up to two further nitrogen atoms in the ring, or a 6-membered heteroaromatic ring including 1, 2 or 3 nitrogen atoms.

More preferably R^{D} is furanyl, thienyl, pyrrolyl, pyrazolyl, imidazolyl, isoxazolyl, oxazolyl, isothiazolyl, thiazolyl, oxadiazolyl, pyridyl, pyridazinyl, pyrimidyl or pyrazinyl.

Most preferably, R^{D} is pyrazolyl, imidazolyl, isoxazolyl, oxazolyl, oxadiazolyl, pyridyl, pyridazinyl, pyrimidyl or pyrazinyl.

Preferably, R⁷ is halo,C₁-C₆ alkyl, C₁-C₆ haloalkyl, OR¹² or CONR¹²R¹³.

More preferably, R⁷ is halo, C₁-C₃ alkyl, C₁-C₃ alkoxy, hydroxy or CONH(C₁-C₃ alkyl).

Most preferably, R⁷ is fluoro, methyl, ethyl, hydroxy, methoxy, propoxy or CONHMe.

Preferably, R² is hydrogen or methyl.

More preferably, R² is hydrogen.

Preferably, R³ is hydrogen, C₁-C₆ alkyl, which is optionally substituted with one or more R⁸ groups, or R^{E}, which is optionally substituted with one or more R⁹ groups; and wherein R^{E} is a monocyclic or, when there are an appropriate number of ring atoms, polycyclic saturated ring system containing between 3 and 7 ring atoms, of which at least one is a heteroatom selected from nitrogen, oxygen and sulphur.

More preferably, R³ is hydrogen, C₁-C₄ alkyl, which is optionally substituted with one or more R⁸ groups, or R^{E} which is optionally substituted with one or more R⁹ groups; and wherein R^{E} is a monocyclic saturated ring system containing between 3 and 7 ring atoms, of which at least one is a heteroatom selected from nitrogen, oxygen and sulphur.

In one preferred embodiment, R³ is R^{E}, which is optionally substituted with one or more R⁹ groups and wherein R^{E} is a monocyclic saturated ring system containing between 3 and 7 ring atoms containing one nitrogen atom.

More preferably, R^{E} is azetidinyl, pyrrolidinyl or piperidinyl.

In another preferred embodiment, R³ is C₁-C₄ alkyl, which is optionally substituted with one or more R⁸ groups and wherein R⁸ is halo, phenyl, C₁-C₆ alkoxyphenyl, OR¹², NR¹²R¹³, NR¹²CO₂R¹⁴, CO₂R¹², CONR¹²R¹³, R^{G} or R^{H}, the last two of which are optionally substituted with one or more R⁹ groups.

More preferably, R⁸ is hydroxy, methoxy, methoxyphenyl, NH₂, NHMe, NMe₂, NHCO₂^{t}Bu, NMeCO₂^{t}Bu, CO₂H, CONHMe, R^{G} or R^{H}, the last two of which are optionally substituted with one or more R⁹ groups.

In one preferred embodiment, R⁸ is R^{G}, which is optionally substituted with one or more R⁹ groups and wherein R^{G} is a monocyclic saturated ring system containing between 3 and 7 ring atoms, of which at least one is a heteroatom selected from nitrogen, oxygen and sulphur.

More preferably, R^{G} is a monocyclic saturated ring system containing between 3 and 7 ring atoms containing one nitrogen atom and optionally one oxygen atom.

Most preferably, R^{G} is pyrrolidinyl, piperidinyl or morpholinyl.

In another preferred embodiment, R⁸ is R^{H}, which is optionally substituted with one or more R⁹ groups and wherein R^{H} is a 5- or 6-membered heteroaromatic ring containing up to two nitrogen atoms.

More preferably, R^{H} is pyrazolyl.

Preferably, R⁹ is methyl or CO₂^{t}Bu.

In another preferred embodiment, R³ is hydrogen or C₁-C₄ alkyl, which is optionally substituted with one or more R⁸ groups, or R³ is azetidinyl, pyrrolidinyl or piperidinyl, each of which is optionally substituted with one or more R⁹ groups, wherein R⁸ is hydroxy, methoxy, methoxyphenyl, NH₂, NHMe, NMe₂, NHCO₂^{t}Bu, NMeCO₂^{t}Bu, CO₂H, CONHMe, pyrrolidinyl, piperidinyl, morpholinyl or pyrazolyl, the last four of which are optionally substituted with one or more R⁹ groups and wherein R⁹ is methyl or CO₂^{t}Bu.

In one preferred embodiment, R⁴ is hydrogen, C₁-C₆ alkyl, C₁-C₆ haloalkyl, C₂-C₆ alkenyl or C₂-C₆ alkynyl.

More preferably, R⁴ is hydrogen, C₁-C₆ alkyl or C₁-C₆ haloalkyl.

Most preferably, R⁴ is hydrogen, methyl or ethyl.

In another preferred embodiment, -NR³R⁴ forms R^{F}, which is optionally substituted with one or more R¹⁰ groups and wherein R^{F} is a monocyclic or, when there are an appropriate number of ring atoms, polycyclic saturated ring system containing between 3 and 10 ring atoms containing at least one nitrogen atom and optionally one other atom selected from oxygen and sulphur.

More preferably, R^{F} is a monocyclic or, when there are an appropriate number of ring atoms, polycyclic saturated ring system containing between 3 and 10 ring atoms containing one or two nitrogen atoms and optionally one other atom selected from oxygen and sulphur.

Most preferably, R^{F} is selected from azetidinyl, pyrrolidinyl, piperidinyl, piperazinyl, morpholinyl, 3-azabicyclo[3.1.0]hex-3-yl, homopiperazinyl, 2,5-diazabicyclo[4.3.0]non-2-yl, 3,8-diazabicyclo[3.2.1]oct-3-yl, 3,8-diazabicyclo[3.2.1]oct-8-yl, 1,4-diazabicyclo[4.3.0]non-4-yl and 1,4-diazabicyclo[3.2.2]non-4-yl.

Preferably R¹⁰ is halo, OR¹², NR¹²R¹³ NR¹²CO₂R¹⁴, CO₂R¹³, oxo, C₁-C₆ alkyl or C₁-C₆ haloalkyl, the last two of which are optionally substituted by R¹¹.

More preferably, R¹⁰ is halo, methyl, ethyl, isopropyl, hydroxy, methoxy, NH₂, NHMe, NMe₂, NHCO₂^{t}Bu, CO₂H CO₂^{t}Bu, oxo, benzyl, -CH₂NH₂, -CH₂NHMe, CH₂NMe₂ or -CH₂NMeCO₂'Bu.

Preferably R⁵ is -CONR¹⁵R¹⁶, i.e. a group -Y-CONR¹⁵R¹⁶ wherein Y is a covalent bond. Preferably R¹⁵ and R¹⁶ are each independently selected from hydrogen, C₁-C₆ alkyl optionally substituted with R¹⁷, -NR¹⁸R¹⁹, -CO₂R²⁰, -CONR²¹R²², R²³ or phenyl optionally substituted by halo, C₁-C₆ alkyl or R¹⁷, C₃-C₇ cycloalkyl and R²³, or NR¹⁵R¹⁶ constitutes a 5- to 7-membered ring which may optionally include one or more further heteroatoms selected from nitrogen and oxygen, and which may optionally be further substituted with R¹⁷, -CO₂R²⁰, -CONR²¹R²² or C₁-C₆ alkyl optionally substituted by R¹⁷. More preferably R¹⁵ and R¹⁶ are each independently selected from hydrogen and C₁-C₆ alkyl optionally substituted with R¹⁷ or -NR¹⁸R¹⁹.

Preferably, R¹⁷ is hydroxy, C₁-C₆ alkoxy or C₃-C₇ cycloalkyloxy;

Preferably, R²¹ and R²² are each independently selected from hydrogen, C₁-C₆ alkyl, and C₃-C₇ cycloalkyl, or -NR²¹R²² constitutes a 5- to 8-membered ring which may optionally include one or more further heteroatoms selected from nitrogen and oxygen.

Preferably, R²³ is a saturated 5- to 7-membered ring which includes at least one heteroatom selected from nitrogen and oxygen, which ring may optionally be substituted by one or more C₁-C₆ alkyl groups.

Preferably, R⁶ is positioned on N¹ to give the compound of formula (I^{A}):

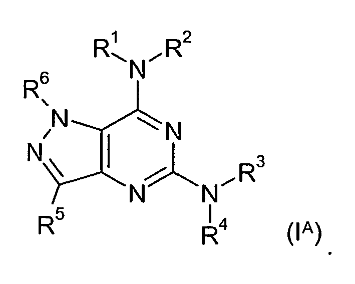

In an alternative embodiment of the present invention, R⁶ may be positioned on N² to give the compound of formula (I^{B}):

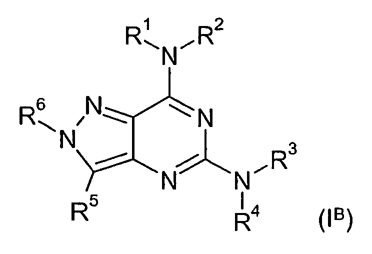

Preferably, R⁶ is C₁-C₆ alkyl or C₁-C₆ haloalkyl, each of which is optionally substituted by C₁-C₆ alkoxy, C₁-C₆ haloalkoxy or a cyclic group selected from R^{J}, R^{L} and R^{M}, or R⁶ is R^{N} or hydrogen;
R^{J} is a C₃-C₇ monocyclic cycloalkyl group;
R^{L} and R^{N} are each independently a monocyclic, saturated or partly unsaturated ring system containing between 4 and 7 ring atoms, of which at least one is a heteroatom selected from nitrogen, oxygen and sulphur; and
R^{M} is a 5- or 6-membered heteroaromatic ring containing up to three heteroatoms independently selected from nitrogen, oxygen and sulphur.

More preferably, R⁶ is C₁-C₄ alkyl or C₁-C₄ haloalkyl, each of which is optionally substituted by C₁-C₄ alkoxy, C₁-C₄ haloalkoxy or a cyclic group selected from R^{J}, R^{L} and R^{M}, or R⁶ is R^{N} or hydrogen;
R^{J} is cyclopropyl or cyclobutyl;
R^{L} and R^{N} are each independently a monocyclic saturated ring system containing either 5 or 6 ring atoms, of which at least one is a heteroatom selected from nitrogen, oxygen and sulphur; and
R^{M} is a 5- or 6-membered heteroaromatic ring containing a heteroatom selected from nitrogen, oxygen and sulphur.

More preferably, R⁶ is C₁-C₄ alkyl or C₁-C₄ haloalkyl, each of which is optionally substituted by C₁-C₄ alkoxy or a cyclic group selected from R^{J}, R^{L} and R^{M}, or R⁶ is R^{N} or hydrogen;
R^{J} is cyclopropyl or cyclobutyl;
R^{L} and R^{N} are each independently a monocyclic saturated ring system containing either 5 or 6 ring atoms containing one heteroatom selected from nitrogen, oxygen and sulphur; and
R^{M} is a 5- or 6-membered heteroaromatic ring containing one nitrogen atom.

More preferably, R⁶ is C₁-C₄ alkyl or C₁-C₄ haloalkyl, each of which is optionally substituted by C₁-C₄ alkoxy, cyclopropyl, cyclobutyl, tetrahydrofuranyl, tetrahydropyranyl or pyridinyl, or R⁶ is hydrogen or tetrahydropyranyl.

Most preferably, R⁶ is hydrogen, methyl, ethyl, isopropyl, isobutyl, methoxyethyl, methoxypropyl, ethoxyethyl, ethoxypropyl, propoxyethyl, 2,2,2-trifluoroethyl, tetrahydrofuranylmethyl, tetrahydropyranylmethyl, tetrahydropyranyl or pyridinylmethyl.

Preferred embodiments of compounds of formula (I) are those that incorporate two or more of the foregoing preferences.

Preferably R¹ is a cyclic group selected from R^{A}, R^{B}, R^{C} and R^{D}, each of which is optionally substituted with one or more R⁷ groups;
R² is hydrogen or C₁-C₂ alkyl;
R³ is hydrogen, C₁-C₄ alkyl, which is optionally substituted with one or more R⁸ groups, or R^{E}, which is optionally substituted with one or more R⁹ groups;
R⁴ is hydrogen, C₁-C₆ alkyl or C₁-C₆ haloalkyl;
or -NR³R⁴ forms R^{F}, which is optionally substituted with one or more R¹⁰ groups;
R⁶ is C₁-C₄ alkyl or C₁-C₄ haloalkyl, each of which is optionally substituted by C₁-C₄ alkoxy, C₁-C₄ haloalkoxy or a cyclic group selected from R^{J}, R^{L} and R^{M}, or R⁶ is R^{N} or hydrogen;
R⁷ is halo, C₁-C₆ alkyl, C₁-C₆ haloalkyl, C₂-C₆ alkenyl, C₂-C₆ alkynyl, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹², CONR¹²R¹³ or CN;
R⁸ is halo, phenyl, C₁-C₆ alkoxyphenyl, OR¹², OC(O)R¹², NO₂, NR¹²R¹³, NR¹²C(O)R¹³, NR¹²CO₂R¹⁴, C(O)R¹², CO₂R¹², CONR¹²R¹³, CN, R^{G} or R^{H}, the last two of which are optionally substituted with one or more R⁹ groups;
R⁹ is C₁-C₆ alkyl, C₁-C₆ haloalkyl or CO₂R¹²;
R¹⁰ is halo, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, C₁-C₆ alkyl or C₁-C₆ haloalkyl, the last two of which are optionally substituted by R¹¹;
R¹¹ is phenyl, NR¹²R¹³ or NR¹²CO₂R¹⁴;
R¹² and R¹³ are each independently hydrogen, C₁-C₆ alkyl or C₁-C₆ haloalkyl;
R¹⁴ is C₁-C₆alkyl or C₁-C₆ haloalkyl;
R^{A} is a monocyclic C₃-C₈ cycloalkyl group;
R^{B} is phenyl;
R^{C} is a monocyclic saturated or partly unsaturated ring system containing between 3 and 8 ring atoms, of which at least one is a heteroatom selected from nitrogen, oxygen and sulphur;
R^{D} is a 5- or 6-membered heteroaromatic ring containing up to three heteroatoms independently selected from nitrogen, oxygen and sulphur;
R^{E} is a monocyclic saturated ring system containing between 3 and 7 ring atoms, of which at least one is a heteroatom selected from nitrogen, oxygen and sulphur;
R^{F} and R^{G} are each independently a monocyclic or, when there are an appropriate number of ring atoms, polycyclic saturated ring system containing between 3 and 10 ring atoms, of which at least one is a heteroatom selected from nitrogen, oxygen and sulphur;
R^{H} is a 5- or 6-membered heteroaromatic ring containing up to three heteroatoms independently selected from nitrogen, oxygen and sulphur;
R^{J} is cyclopropyl or cyclobutyl;
R^{L} and R^{N} are each independently a monocyclic saturated ring system containing either 5 or 6 ring atoms, of which at least one is a heteroatom selected from nitrogen, oxygen and sulphur;
R^{M} is a 5- or 6-membered heteroaromatic ring containing a heteroatom selected from nitrogen, oxygen and sulphur; and
Y is a covalent bond.

More preferably, R¹ is a cyclic group selected from R^{A}, R^{B}, R^{C} and R^{D}, each of which is optionally substituted with one or more R⁷ groups;
R² is hydrogen or C₁-C₂ alkyl;
R³ is hydrogen, C₁-C₄ alkyl, which is optionally substituted with one or more R⁸ groups, or R^{E}, which is optionally substituted with one or more R⁹ groups;
R⁴ is hydrogen, C₁-C₆ alkyl or C₁-C₆ haloalkyl;
or -NR³R⁴ forms R^{F}, which is optionally substituted with one or more R¹⁰ groups;
R⁶ is C₁-C₄ alkyl or C₁-C₄ haloalkyl, each of which is optionally substituted by C₁-C₄ alkoxy, C₁-C₄ haloalkoxy or a cyclic group selected from R^{J}, R^{L} and R^{M}, or R⁶ is R^{N} or hydrogen;
R⁷is halo, C₁-C₆ alkyl, C₁-C₆ haloalkyl, OR¹² or CONR¹²R¹³;
R⁸ is halo, phenyl, C₁-C₆ alkoxyphenyl, OR¹², NR¹²R¹³, NR¹²CO₂R¹⁴, CO₂R¹², CONR¹²R¹³, R^{G} or R^{H}, the last two of which are optionally substituted with one or more R⁹ groups;
R⁹ is C₁-C₆ alkyl, C₁-C₆ haloalkyl or CO₂R¹²;
R¹⁰ is halo, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, C₁-C₆ alkyl or C₁-C₆ haloalkyl, the last two of which are optionally substituted by R¹¹;
R¹¹ is phenyl, NR¹²R¹³ or NR¹²CO₂R¹⁴;
R¹² and R¹³ are each independently hydrogen, C₁-C₆ alkyl or C₁-C₆ haloalkyl;
R¹⁴ is C₁-C₆ alkyl or C₁-C₆ haloalkyl;
R¹⁵ and R¹⁶ are each independently selected from hydrogen, C₁-C₆ alkyl optionally substituted with R¹⁷, -NR¹⁸R¹⁹, -CO₂R²⁰, -CONR²¹R²², R²³ or phenyl optionally substituted by halo, C₁-C₆ alkyl or R¹⁷, C₃-C₇ cycloalkyl and R²³, or NR¹⁵R¹⁶ constitutes a 5- to 7-membered ring which may optionally include one or more further heteroatoms selected from nitrogen and oxygen, and which may optionally be further substituted with R¹⁷, -CO₂R²⁰, -CONR²¹R²² or C₁-C₆ alkyl optionally substituted by R¹⁷;
R¹⁷ is hydroxy, C₁-C₆ alkoxy or C₃-C₇ cycloalkyloxy;
R²¹ and R²² are each independently selected from hydrogen, C₁-C₆ alkyl, and C₃-C₇ cycloalkyl, or -NR²¹R²² constitutes a 5- to 8-membered ring which may optionally include one or more further heteroatoms selected from nitrogen and oxygen;
R²³ is a saturated 5- to 7-membered ring which includes at least one heteroatom selected from nitrogen and oxygen, which ring may optionally be substituted by one or more C₁-C₆ alkyl groups;
R^{A} is a monocyclic C₅-C₇ cycloalkyl group;
R^{B} is phenyl;
R^{C} is a monocyclic saturated ring system containing between 5 and 7 ring atoms, of which at least one is a heteroatom selected from nitrogen, oxygen and sulphur;
R^{D} is a 5-membered heteroaromatic ring containing a heteroatom selected from nitrogen, oxygen and sulphur and optionally up to two further nitrogen atoms in the ring, or a 6-membered heteroaromatic ring including 1, 2 or 3 nitrogen atoms;
R^{E} is a monocyclic saturated ring system containing between 3 and 7 ring atoms containing one nitrogen atom;
R^{F} is a monocyclic or, when there are an appropriate number of ring atoms, polycyclic saturated ring system containing between 3 and 10 ring atoms containing at least one nitrogen atom and optionally one other atom selected from oxygen and sulphur;
R^{G} is a monocyclic saturated ring system containing between 3 and 7 ring atoms, of which at least one is a heteroatom selected from nitrogen, oxygen and sulphur;
R^{H} is a 5- or 6-membered heteroaromatic ring containing up to two nitrogen atoms;
R^{L} and R^{N} are each independently a monocyclic saturated ring system containing either 5 or 6 ring atoms, of which at least one is a heteroatom selected from nitrogen, oxygen and sulphur;
R^{M} is a 5- or 6-membered heteroaromatic ring containing a heteroatom selected from nitrogen, oxygen and sulphur; and
Y is a covalent bond.

Most preferred compounds are:
1-(2-ethoxyethyl)-*N*-ethyl-5-(ethylamino)-7-(4-methylpyridin-2-ylamino)-1H-pyrazolo[4,3-*d*]pyrimidine-3-carboxamide,
5-(dimethylamino)-1-(2-ethoxyethyl)-*N*-methyl-7-(4-methylpyridin-2-ylamino)-1H-pyrazolo[4,3-*d*]pyrimidine-3-carboxamide,
5-(dimethylamino)-1-(2-ethoxyethyl)-*N*-(2-(methylamino)ethyl)-7-(4-methylpyridin-2-ylamino)-1*H*-pyrazolo[4,3-*d*]pyrimidine-3-carboxamide,
5-(dimethylamino)-*N*-(2-(dimethylamino)ethyl)-1-(2-ethoxyethyl)-7-(4-methylpyridin-2-ylamino)-1*H*-pyrazolo[4,3-*d*]pyrimidine-3-carboxamide,
5-(dimethylamino)-1-(2-ethoxyethyl)-7-(4-methylpyridin-2-ylamino)-*N*-(piperidin-4-yl)-1*H*-pyrazolo[4,3-*d*]pyrimidine-3-carboxamide,
5-(dimethylamino)-1-(2-ethoxyethyl)-*N*-(2-methoxyethyl)-7-(4-methylpyridin-2-yl-amino)-1*H*-pyrazolo[4,3-*d*]pyrimidine-3-carboxamide,
(2*R*)-2-{[5-(dimethylamino)-1-(2-ethoxyethyl)-7-(4-methylpyridin-2-ylamino)-1*H-*pyrazolo[4,3-*d*]pyrimidine-3-carbonyl]amino}propionicacid,
3-{[5-(dimethylamino)-1-(2-ethoxyethyl)-7-(4-methylpyridin-2-ylamino)-1*H-*pyrazolo[4,3-*d*]pyrimidine-3-carbonyl]amino}propionicacid,
1-(2-ethoxyethyl)-*N*-methyl-7-(4-methylpyridin-2-ylamino)-5-(piperazin-1-yl)-1*H-*pyrazolo[4,3-*d*]pyrimidine-3-carboxamide,
1-(2-ethoxyethyl)-*N*-methyl-5-((3*R*)-3-methylpiperazin-1-yl)-7-(4-methylpyridin-2-yl-amino)-1H-pyrazolo[4,3-*d*]pyrimidine-3-carboxamide,
1-(2-ethoxyethyl)-N-ethyl-5-((3*R*)-3-methylpiperazin-1-yl)-7-(4-methylpyridin-2-yl-amino-1*H*-pyrazolo[4,3-*d*]pyrimidine-3-carboxamide,
1-(2-ethoxyethyl)-5-(ethylamino)-*N*-methyl-7-(4-methylpyridin-2-ylamino)-1*H-*pyrazolo[4,3-*d*]pyrimidine-3-carboxamide,
1-(2-ethoxyethyl)-*N*-(2-methoxyethyl)-5-(methylamino)-7-(4-methylpyridin-2-ylamino)-1*H*-pyrazolo[4.3-d]pyrimidine-3-carboxamide,
5-(dimethylamino)-1-(2-ethoxyethyl)-*N*-(2-hydroxyethyl)-7-(4-methylpyridin-2-yl-amino)-1*H*-pyrazolo(4,3-*d*]pyrimidine-3-carboxamide,
1-(2-ethoxyethyl)-5-(ethylamino)-*N*-(2-methoxyethyl)-7-(4-methylpyridin-2-ylamino)-1*H*-pyrazolo[4,3-*d*]pyrimidine-3-carboxamide,
1-(2-ethoxyethyl)-5-(*N*-(2-hydroxyethyl)-*N*-methylamino)-N-methyl-7-(4-methyl-pyridin-2-ylamino)-1*H*-pyrazolo[4,3-*d*]pyrimidine-3-carboxamide,
1-(2-ethoxyethyl)-5-((2-methoxyethyl}amino)-*N*-methyl-7-{4-methylpyridin-2-ylamino)-1*H*-pyrazolo[4,3-d]pyrimidine-3-carboxamide,
7-(cyclohexylamino)-1-(2-ethoxyethyl)-*N*-methyl-5-(3*R*)-3-methylpiperazjn-1-(yl)-1*H-*pyrazolo[4,3-*d*]pyrimidine-3-carboxamide,and
1-(2-ethoxyelhyl)-*N*-methyl-5-[N-methyl-*N*-((3*S*)-1-methylpyrrolidin-3-yl)amino]-7-(4-methylpyridin-2-ylamino)-1*H*-pyrazolo[4,3-*d*]pyrimidine-3-carboxamide
and tautomers thereof and pharmaceutically acceptable salts and solvates of said compounds or tautomers.

Pharmaceutically acceptable salts of the compounds of formula (I) include the add addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, Weinheim, Germany, 2002).

A pharmaceutically acceptable salt of a compound of formula (I) may be readily prepared by mixing together solutions of the compound of formula (I) and the desired acid or base, as appropriate. The salt may precipitate from solution and be collected by filtration or may be recovered by evaporation of the solvent. The degree of ionisation in the salt may vary from completely ionised to almost non-ionised.

The compounds of the invention may exist in both unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8), 1269-1288 by Haleblian (August 1975).

Hereinafter all references to compounds of formula (I) include references to salts, solvates and complexes thereof and to solvates and complexes of salts thereof.

The compounds of the invention include compounds of formula (I) as hereinbefore defined, and isomers thereof (including optical, geometric and tautomeric isomers) as hereinafter defined and isotopically-labeled compounds of formula (1).

Compounds of formula (I) containing one or more asymmetric carbon atoms can exist as two or more stereoisomers. Where a compound of formula (I) contains an alkenyl or alkenylene group, geometric cis/trans (or Z/E) isomers are possible. Where the compound contains, for example, a keto or oxime group or an aromatic moiety, tautomeric isomerism ('lautomerism') can occur. It follows that a single compound may exhibit more than one type of isomerism.

Included within the scope of the present invention are all stereoisomers, geometric isomers and tautomeric forms of the compounds of formula (I), including compounds exhibiting more than one type of isomerism, and mixtures of one or more thereof. Also included are acid addition or base salts wherein the counterion is optically active, for example, D-lactate or L-lysine, or racemic, for example, DL-tartrate or DL-arginine.

*Cis*/*trans* isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture.

Stereoisomeric conglomerates may be separated by conventional techniques known to those skilled in the art - see, for example, "Stereochemistry of Organic Compounds" by E L Eliel (Wiley, New York, 1994).

The present invention includes all pharmaceutically acceptable isotopically-labelled compounds of formula (I) wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula (I), for example, those incorporating a radioactive isotope, are useful in drug and/or substrate tissue distribution studies. The radioactive isotopes tritium, *i.e.* ³H, and carbon-14, *i.e.* ¹⁴C*,* are particularly useful for this purpose in view of their ease of incorporation and ready means of detection.

Substitution with heavier isotopes such as deuterium, *i.e.* ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

Pharmaceutically acceptable solvates in accordance with the invention include those wherein the solvent of crystallization may be isotopically substituted, e.g. D₂O, d₆-acetone, d₆-DMSO.

Compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

The compounds of formula (I) are inhibitors of PDE5. Accordingly, in a further aspect the present invention provides for the use of a compound of formula (I), or a tautomer, salt or solvate thereof, as a pharmaceutical agent, and particularly as a therapeutic agent for the treatment of a condition where inhibition of PDE5 is known, or can be shown, to produce a beneficial effect.

The term "treatment" includes palliative, curative and prophylactic treatment.

Conditions suitable for treatment with the compounds of the invention include hypertension (including essential hypertension, pulmonary hypertension, secondary hypertension, isolated systolic hypertension, hypertension associated with diabetes, hypertension associated with atherosclerosis, and renovascular hypertension), congestive heart failure, angina (including stable, unstable and variant (Prinzmetal) angina), stroke, coronary artery disease, congestive heart failure, conditions of reduced blood vessel patency (such as post-percutaneous coronary angioplasty), peripheral vascular disease, atherosclerosis, nitrate-induced tolerance, nitrate tolerance, diabetes, impaired glucose tolerance, metabolic syndrome, obesity, sexual dysfunction (including male erectile disorder, impotence, female sexual arousal disorder, clitoral dysfunction, female hypoactive sexual desire disorder, female sexual pain disorder, female sexual orgasmic dysfunction and sexual dysfunction due to spinal cord injury), premature labour, pre-eclampsia, dysmenorrhea, polycystic ovary syndrome, benign prostatic hyperplasia, bladder outlet obstruction, incontinence, chronic obstructive pulmonary disease, acute respiratory failure, bronchitis, chronic asthma, allergic asthma, allergic rhinitis, gut motility disorders (including irritable bowel syndrome), Kawasaki's syndrome, multiple sclerosis, Alzheimer's disease, psoriasis, skin necrosis, scarring, fibrosis, pain (particularly neuropathic pain), cancer, metastasis, baldness, nutcracker oesophagus, anal fissure and haemorrhoids.

In a further aspect, the present invention provides for the use of a compound of formula (I), or a tautomer, salt or solvate thereof, for the manufacture of a medicament for the treatment of such a condition.

The compounds of the present invention may be used alone or in combination with other therapeutic agents. When used in combination with another therapeutic agent the administration of the two agents may be simultaneous or sequential. Simultaneous administration includes the administration of a single dosage form that comprises both agents and the administration of the two agents in separate dosage forms at substantially the same time. Sequential administration includes the administration of the two agents according to different schedules provided that there is an overlap in the periods during which the treatment is provided. Suitable agents with which the compounds of formula (I) can be co-administered include aspirin, angiotensin II receptor antagonists (such as losartan, candesartan, telmisartan, valsartan, irbesartan and eprosartan), calcium channel blockers (such as amlodipine), beta-blockers (i.e. beta-adrenergic receptor antagonists such as sotalol, proporanolol, timolol, antenolol, carvedilol and metoprolol), Cl1027, CCR5 receptor antagonists, imidazolines, sGCa's (soluble guanylate cyclase activators) antihypertensive agents, diuretics (such as hydrochlorothiazide, torsemide, chlorothiazide, chlorthalidone and amiloride), alpha adrenergic antagonists (such as doxazosin), ACE (angiotensin converting enzyme) inhibitors (such as quinapril, enalapril, ramipril and lisinopril), aldosterone receptor antagonists (such as eplerenone and spironolactone), neutral endopeptidase inhibitors, antidiabetic agents (such as insulin, sulfonylureas (such as glyburide, glipizide and glimepiride), glitazones (such as rosiglitazone and pioglitazone) and metformin), cholesterol lowering agents (such as atorvastatin, pravastatin, lovastatin, simvastatin, clofibrate and rosuvastatin), and alpha-2-delta ligands (such as gabapentin, pregabalin, [(1R,5R,65)-6-(aminomethyl)bicyclo[3.2.0]hept-6-yl]acetic acid, 3-(1-aminomethyl-cyclohexylmethyl)-4H-[1,2,4]oxadiazol-5-one, C-[1-(1 H-tetrazol-5-ylmethyl)-cycloheptyl]-methylamine, (3S,4S)-(1-aminomethyl-3,4-dimethyl-cyclopentyl)-acetic acid, (1α,3α,5α)-(3-amino-methyl-bicyclo[3.2.0]hept-3-yl)-acetic acid, (3S,5R)-3-aminomethyl-5-methyl-octanoic acid, (3S,5R)-3-amino-5-methyl-heptanoic acid, (3S,5R)-3-amino-5-methyl-nonanoic acid and (3S,5R)-3-amino-5-methyl-octanoic acid).

The compounds of formula (I) may be administered alone or in combination with one or more other compounds of the invention or in combination with one or more other drugs (or as any combination thereof). Generally, they will be administered as a formulation in association with one or more pharmaceutically acceptable excipients. The term "excipient" is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films (including muco-adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001).

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 wt% to 5 wt% of the tablet, and glidants may comprise from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 wt% to 10 wt%, preferably from 0.5 wt% to 3 wt% of the tablet.

Other possible ingredients include anti-oxidants, colourants, flavouring agents, preservatives and taste-masking agents.

Exemplary tablets contain up to about 80% drug, from about 10 wt% to about 90 wt% binder, from about 0 wt% to about 85 wt% diluent, from about 2 wt% to about 10 wt% disintegrant, and from about 0.25 wt% to about 10 wt% lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in "Pharmaceutical Dosage Forms: Tablets, Vol. 1", by H. Lieberman and L. Lachman, Marcel Dekker, N.Y., N.Y., 1980 (ISBN 0-8247-6918-X).

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Verma et al, Pharmaceutical Technology On-line, 25(2), 1-14 (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (e.g. Powderject^{™}, Bioject^{™}, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or HPMC), blisters and cartridges for use in an inhaler or insufflator may be formulated to contain a powder mix of the compound of the invention, a suitable powder base such as lactose or starch and a performance modifier such as *I*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 10mg of the compound of the invention per actuation and the actuation volume may vary from 1µl to 100µl. A typical formulation may comprise a compound of formula (I), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, poly(DL-lactic-coglycolic acid (PGLA). Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 1µg to 20mg of the compound of formula (I). The overall daily dose will typically be in the range 1µg to 80mg which may be administered in a single dose or, more usually, as divided doses throughout the day.

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i.e*. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula ... in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the range 0.1 mg to 500mg depending, of course, on the mode of administration. For example, oral administration may require a total daily dose of from 0.1 mg to 500mg, while an intravenous dose may only require from 0.01 mg to 50mg. The total daily dose may be administered in single or divided doses.

These dosages are based on an average human subject having a weight of about 65kg to 70kg. The physician will readily be able to determine doses for subjects whose weight falls outside this range, such as infants and the elderly.

Compounds of the invention may be prepared, in known manner in a variety of ways. In the following reaction schemes and hereafter, unless otherwise stated R¹ to R⁶ are as defined in the first aspect. These processes form further aspects of the invention.
a) Compounds of formula (I) can be prepared from the corresponding monochlorides of formula (II) by reaction with HNR³R⁴ as illustrated in Scheme 1. 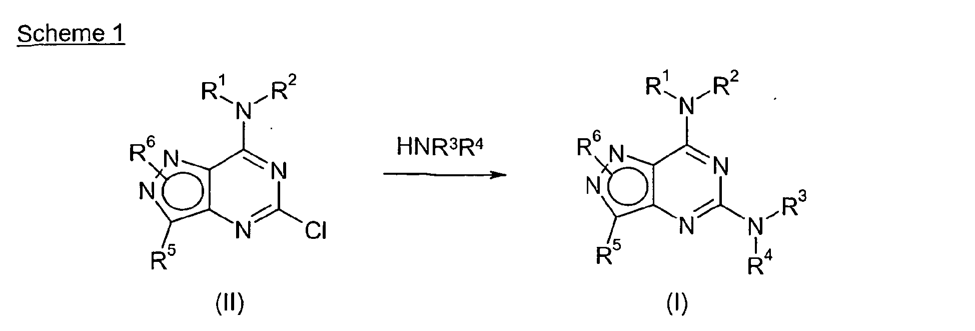
   A solution of the monochloride (II) and the amine HNR³R⁴ in a suitable dipolar aprotic solvent are stirred at elevated temperature for between 1 and 24 hours. Suitable solvents include dimethylsulfoxide, dimethylformamide and N-methylpyrrolidinone. An excess of a tertiary amine such as N-ethyldiisopropylamine, N-methylmorpholine or triethylamine, and/or a fluoride source such as caesium fluoride or tetraethylammonium fluoride may optionally be included. It is sometimes necessary to perform the reaction at elevated pressure in a closed vessel, particularly when the amine HNR³R⁴ or the solvent is volatile.
   Preferably, the monochloride is treated with 3-5 equivalents of the amine HNR³R⁴ and 3-5 equivalents of N-ethyldiisopropylamine in dimethylsulfoxide or N-methylpyrrolidinone, optionally in the presence of caesium fluoride or tetraethylammonium fluoride, at 80-125°C for 12-18 hours.
   It will be appreciated that any functional groups in HNR³R⁴, and particularly any primary or secondary amine groups, may need to be protected in order to allow this reaction to proceed successfully. In such a case, or when there is a functional group in another part of the structure of (I) that is protected, such as an amine group in R¹ or R⁵, the final step of the synthesis will be a deprotection step. For example, if there is an amine group protected by a BOC group, then treatment with acid (such as hydrogen chloride in dioxan or trifluoroacetic acid) will be used. If benzyloxycarbonyl is the preferred amine protecting group, the unmasking step can be a hydrogenolysis. Similarly, any carboxylic acids protected as esters can be deprotected by appropriate methods, such as treatment with trifluoroacetic acid (for *tert-*butyl esters) or hydrogenolysis (for benzyl esters).
b) Compounds of formula (II) can be prepared from the corresponding acids of formula (III) by reaction with HNR¹⁵R¹⁶ as illustrated in Scheme 2. 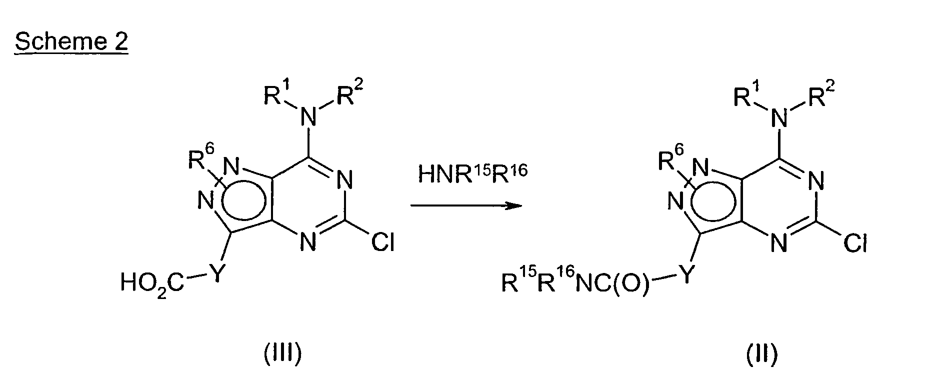
   A solution of the acid (III) and the amine HNR¹⁵R¹⁶ in a suitable solvent is treated with a condensing agent, optionally in the presence of 1-hydroxybenzotriazole (HOBT) (or 1-hydroxy-7-azabenzotriazole (HOAT)) and a tertiary amine base such as triethylamine, N-ethyldiisopropylamine or 4-(dimethylamino)pyridine, at a temperature of between 0°C and the boiling point of the solvent. Suitable solvents include acetonitrile, dichloromethane, dimethylformamide, ethyl acetate, N-methylpyrrolidinone, tetrahydrofuran and mixtures thereof. Suitable condensing agents include: 1,1'-carbonyldiimidazole, carbodiimides such as dicyclohexylcarbodiimide (DCC) and 1-(3-dimethylaminopropyl)-1-ethylcarbodiimide . (WSCDI); uronium salts such as O-(benzotriazol-1-yl)-1,1,3,3-tetramethyluronium hexafluorophosphate (HBTU) and O-(7-azabenzotriazol-1-yl)-1,1,3,3-tetramethyluronium hexafluorophosphate (HATU); phosphonium salts such as 1-benzotriazolyloxytris(dimethylamino)phosphonium hexafluorophosphate (BOP) and 1-benzotriazolyloxytris(pyrrolidino)phosphonium hexafluorophosphate (PyBOP); diphenylphosphinic chloride (Dpp-Cl) and bis(2-oxo-3-oxazolidinyl)phosphinic chloride (BOP-Cl).
   Alternatively the acid may be converted to a more reactive derivative such as the acid chloride, for example by treatment with thionyl chloride or oxalyl chloride. The reactive derivative is then reacted with the amine HNR¹⁵R¹⁶ in a suitable solvent, in the presence of a tertiary amine base such as triethylamine, N-ethyldiisopropylamine or 4-(dimethylamino)pyridine. Suitable solvents include dichloromethane and dimethylformamide.
   The transformations of Schemes 2 and 1 may conveniently be carried out sequentially without isolation of the intermediate of formula (II). Thus the compounds of formula (III) may be treated with an amine HNR¹⁵R¹⁶ at room temperature in the presence of 1,1'-carbonyldiimidazole, then a second amine HNR³R⁴ may be added and the mixture heated to 120°C so as to provide the compounds of formula (I) directly.
c) Compounds of formula (III) can be prepared from the corresponding esters of formula (IV) wherein R^{A} is an alkyl group (particularly a methyl, ethyl, or *tert*-butyl group) or a benzyl group, as illustrated in Scheme 3. 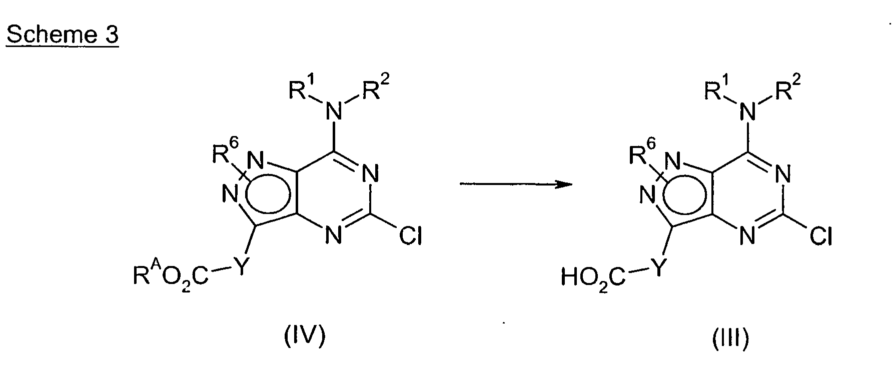
   When R^{A} is methyl or ethyl the conversion may conveniently be accomplished by treating the compound of formula (IV) with an alkaline metal hydroxide such as lithium, sodium or potassium hydroxide in a suitable solvent at a temperature of between about 10°C and the boiling point of the solvent. Suitable solvents include water, methanol, ethanol and mixtures of water with methanol, ethanol, tetrahydrofuran and dioxan. When R^{A} is *tert*-butyl the conversion may be accomplished by treating the compound of formula (IV) with an acid such as hydrogen chloride or trifluoroacetic acid in a suitable solvent at a temperature of between 0°C and ambient temperature. Suitable solvents include dioxan and dichloromethane. When R^{A} is benzyl the conversion may conveniently be accomplished by treating the compound of formula (IV) with an alkaline metal hydroxide as discussed above, or by hydrogenolysis using molecular hydrogen or a suitable hydrogen donor such as ammonium formate in the presence of a transition metal or transition metal salt catalyst such as palladium-on-carbon, in a suitable solvent, such as methanol.
d) Compounds of formula (IV) can generally be prepared from the corresponding dichlorides of formula (V) by reaction with HNR¹R² as illustrated in Scheme 4. 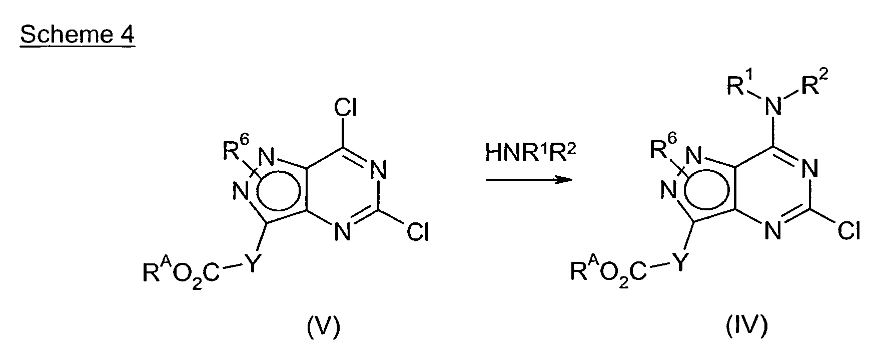
   A solution of the dichloride (V), the amine HNR¹R² and an excess of a tertiary amine such as N-ethyldiisopropylamine, N-methylmorpholine or triethylamine in a suitable dipolar aprotic solvent are stirred at ambient or elevated temperature for between 1 and 24 hours. Suitable solvents include dichloromethane, dimethylsulfoxide, dimethylformamide, tetrahydrofuran and N-methylpyrrolidinone. It will be appreciated that any functional groups in HNR¹R², and particularly any primary or secondary amine groups, may need to be protected in order to allow this reaction to proceed successfully. Preferably, the monochloride is treated with 3-5 equivalents of the amine HNR¹R² and optionally 3-5 equivalents of N-ethyldiisopropylamine in dimethylsulfoxide or a mixture of dimethylsulfoxide and N-methylpyrrolidinone at 30-90°C for 1-18 hours.
   Alternatively, a solution of the amine HNR¹R² in a suitable solvent is treated with butyllithium or sodium hexamethyldisilazide at low temperature, and the dichloride is added to the resulting solution. Suitable solvents include tetrahydrofuran, dioxan and N-methylpyrrolidinone.
   In certain cases, particularly when Y is a covalent bond and the amine HNR¹R² is only weakly nucleophilic, the direct transformation of compounds of formula (V) into compounds of formula (IV) gives unsatisfactory results and a more indirect alternative route may be employed. This route is discussed in part z) below.
e) Compounds of formula (V) can be prepared from the corresponding pyrazolopyrimidinediones formula (VI) as illustrated in Scheme 5. 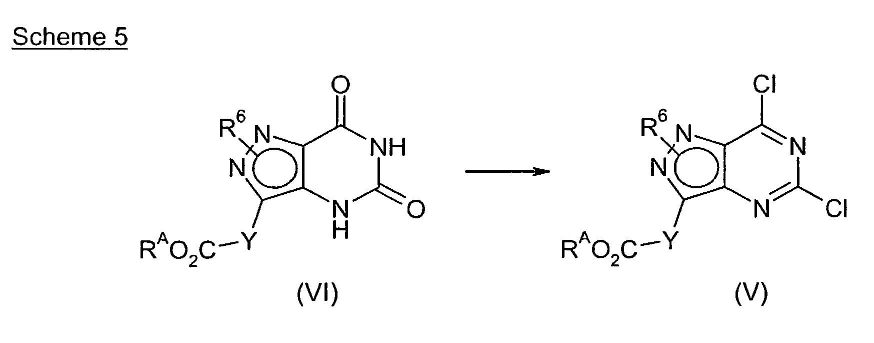
   The dione is treated with a large excess of a suitable chlorinating reagent such as phosphorus oxychloride (POCl₃) or phenylphosphonyl dichloride (PhP(O)Cl₂) in the presence of a tertiary amine such as N-ethyldiisopropylamine, N-methylmorpholine, triethylamine or N,N-dimethylaniline at elevated temperature for 8-48 hours. Dimethylformamide can optionally be added as a catalyst. Alternatively, the dione is treated with POCl₃ or PhP(O)Cl₂ in a suitable solvent in the presence of a tetraalkylammonium chloride, such as tetraethylammonium chloride, at elevated temperature. Suitable solvents include acetonitrile and propionitrile. Preferably, the dione is treated with 10-30 equivalents of POCl₃ and 3-5 equivalents of tetraethylammonium chloride in propionitrile at reflux for 4-18 hours.
f) Compounds of formula (VI) can be prepared from the corresponding aminoamides of formula (VII) as illustrated in Scheme 6. 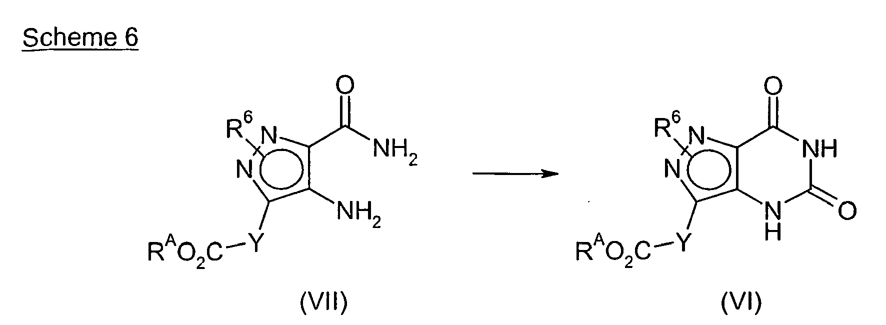
   A solution of the pyrazolecarboxamide (VII) and phosgene or an equivalent thereof, such as 1,1'-carbonyldiimidazole, trichloromethyl chloroformate or bis(trichloromethyl) carbonate, in a suitable solvent is stirred at a temperature of between ambient temperature and the boiling point of the solvent, optionally at elevated pressure, for between 2 and 18 hours. Suitable solvents include acetonitrile, dichloromethane and dimethylformamide. Preferably, a solution of the dione and 1 equivalent of carbonyl diimidazole in dimethylformamide is stirred at 70°C to 90°C for 18 hours.
g) Compounds of formula (VII) can be prepared from the corresponding nitroamides of formula (VIII) as illustrated in Scheme 7. 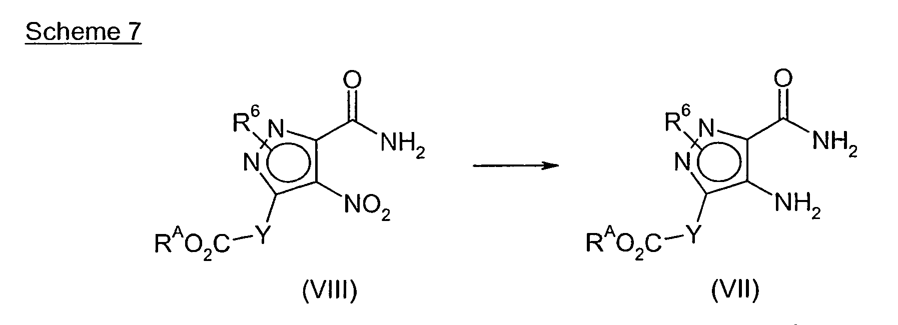
   Reduction of the nitro group can be achieved by, for example, transfer or catalytic hydrogenation, or by a dissolving metal reduction.
   For transfer hydrogenation, the nitro compound is reacted with a suitable hydrogen donor, such as ammonium formate or cyclohexene, in a polar solvent, such as tetrahydrofuran, methanol or ethanol, in the presence of a transition metal or transition metal salt catalyst, such as palladium or palladium(II) hydroxide, optionally at elevated temperature and pressure.
   For catalytic hydrogenation, a solution of the nitro compound in a polar solvent, such as tetrahydrofuran, methanol or ethanol, is stirred under a hydrogen atmosphere in the presence of a transition metal or transition metal salt catalyst, such as palladium or palladium(II) hydroxide, optionally at elevated pressure. The catalyst may be in solution (homogeneous catalysis) or in suspension (heterogeneous catalysis).
   For dissolving metal reduction, the nitro compound is treated with a suitable reactive metal, such as zinc or tin, in the presence of an acid such as acetic acid or hydrochloric acid. Other reducing agents, such as tin(II) chloride, may also be used.
h) Compounds of formula (VIII) can be prepared from the corresponding nitroesters of formula (IX) as illustrated in Scheme 8. 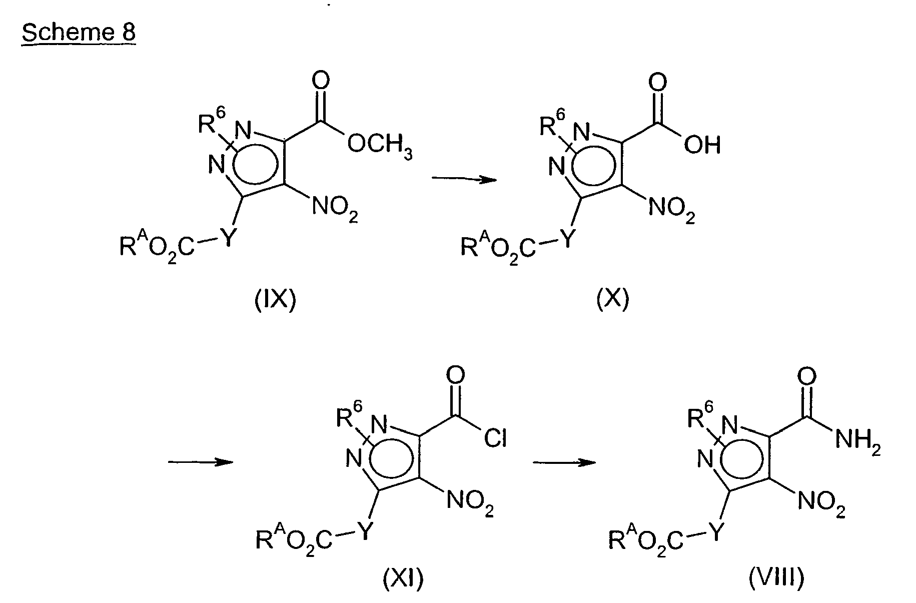
   The methyl ester of the compounds of formula (IX) can be hydrolysed under basic conditions as described in part c) above. For some embodiments of Y, the choice of R^{A} will be limited to those that form esters that are resistant to alkaline hydrolysis, such as branched alkyl groups. The acid (X) is then converted to the corresponding acid chloride (XI) by treatment with oxalyl chloride and dimethylformamide in a suitable solvent such as dichloromethane, or with thionyl chloride. Finally, a solution of the acid chloride in a suitable solvent such as dichloromethane, tetrahydrofuran or dioxan is treated with gaseous ammonia or aqueous ammonia to provide the amide of formula (VIII).
   In the embodiments (IX^{A}) in which Y is a covalent bond and R^{A} is a methyl group, the use of one equivalent of metal hydroxide leads to the chemoselective hydrolysis of the ester group adjacent to the R⁶ substituent (Chambers, D et al., J. Org. Chem. 50, 4736-4738, 1985), as illustrated in Scheme 8A. 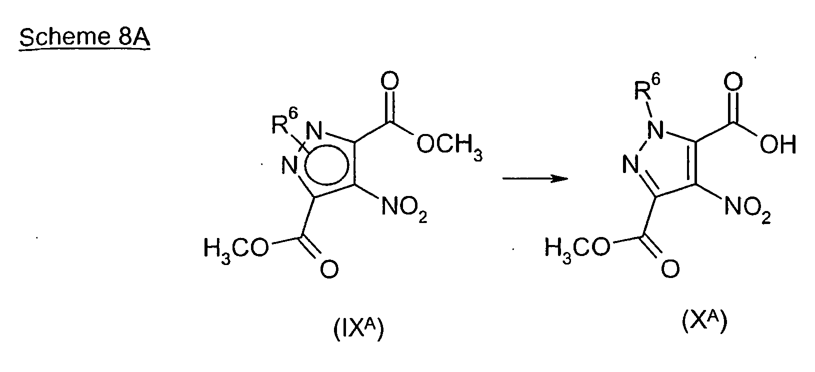
i) Compounds of formula (IX^{B}), wherein R^{6A} is any group according to R⁶ except hydrogen, i.e. compounds of formula (IX) except those wherein R⁶ is hydrogen, can be prepared from the corresponding esters of formula (IX^{C}), i.e. compounds of formula (IX) wherein R⁶ is hydrogen, as illustrated in Scheme 9. 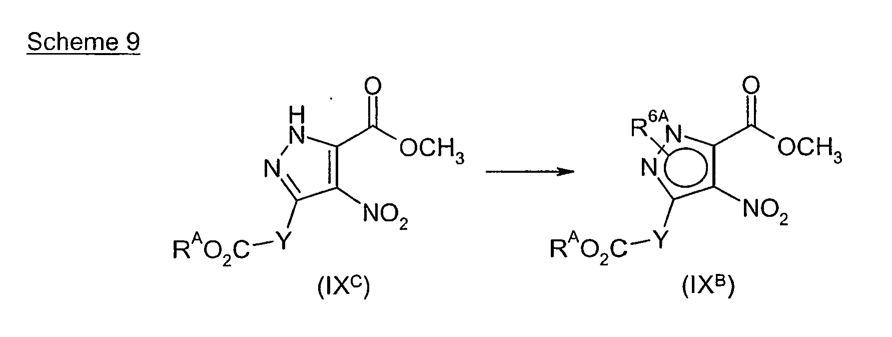
   The compound of formula (IX^{C}) is treated with a base such as an alkaline metal carbonate or bicarbonate, for example potassium carbonate or caesium carbonate, or a tertiary amine, for example triethylamine, diisopropylethylamine or pyridine, and the appropriate chloride (R^{6A}-Cl), bromide (R^{6A}-Br), iodide (R^{6A}-I), mesylate (R^{6A}-OSO₂CH₃) or tosylate (R^{6A}-OSO₂Tol) in a suitable solvent at a temperature of between -70°C and 100°C. Suitable solvents include ethers such as tetrahydrofuran and dioxan, dimethylformamide and acetonitrile. Stronger bases such as sodium hydride, potassium *tert*-butoxide and sodium or potassium hexamethyldisilazide may also be used. Alternatively, the transformation may be achieved using the Mitsunobu reaction, in which a solution of the compound of formula (lX^{C}) and the appropriate alcohol R^{6A}-OH in a suitable solvent is treated with triphenylphosphine and a dialkyl azodicarboxylate such as diethyl azodicarboxylate or diisopropyl azodicarboxylate. A preferred solvent is tetrahydrofuran. The reaction is performed at a temperature of between -10°C and ambient temperature.
   When the reaction gives a mixture of the N¹- and N²-alkylated products, these can be separated using standard techniques.
j) The compound of formula (IX^{C}) wherein R^{A} is methyl and Y is a covalent bond is described in published international patent application WO00/24745 (see preparation 2, page 48). Other compounds of formula (IX), and particularly compounds of formula (IX^{C}), can be prepared in two steps from the diacids of formula (XII), as illustrated in Scheme 10. 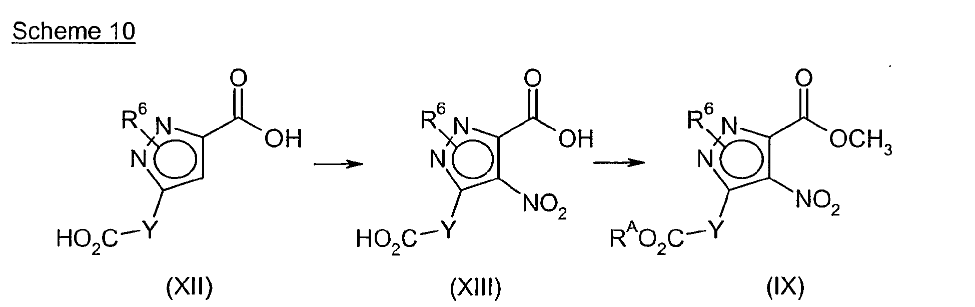
   In the first step, the compounds of formula (XII) are treated with a nitrating agent such as nitric acid or a mixture of nitric acid and sulphuric acid to provide the compounds of formula (XIII). In the second step, the two carboxylic acid groups are esterified. When R^{A} is methyl, this is conveniently achieved in a single operation. When R^{A} is other than methyl, two sub-steps are necessary, and the order in which the two groups are esterified will depend on the nature of Y and R⁶. Suitable conditions for forming esters are well known in the art. When R^{A} is methyl, a preferred method is to treat the diacid with thionyl chloride so as to form the bis-chloride and then react this with methanol.
k) Certain compounds of formula (XII) are commercially available or are described in the literature, in particular those wherein Y is a covalent bond.
   Compounds of formula (XII) that are not items of commerce can be prepared as illustrated in Schemes 11 to 13, in which R^{A} is as defined in part j) above. 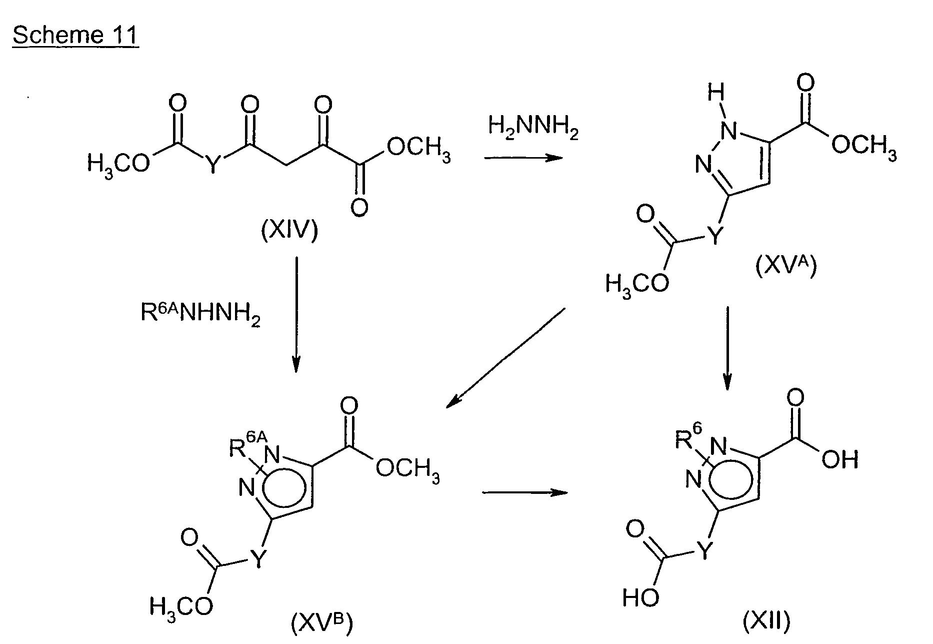
   The method illustrated in Scheme 11 is the Knorr pyrazole synthesis. A 1,3-diketone of formula (XIV) is reacted with hydrazine to give a pyrazole of formula (XV^{A}), or with a substituted hydrazine R^{6A}-NHNH2 to give a pyrazole of formula (XV^{B}).
   Pyrazoles of formula (XV^{B}) may also be obtained by N-alkylation of the corresponding pyrazoles of formula (XV^{A}) following the methods described in part i) above. Hydrolysis of the ester groups as described in part c) above then provides the compounds of formula (XII).
   Compounds of formula (XIV) can be prepared from the corresponding methyl ketones of formula (XVI) using a crossed Claisen condensation as illustrated in Scheme 12. 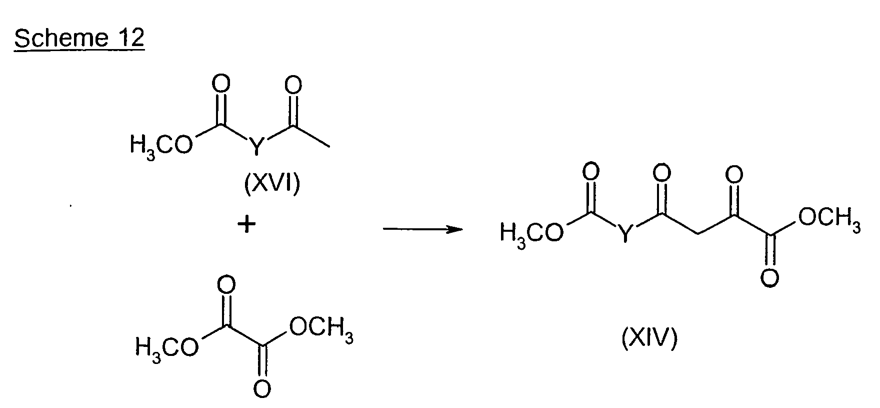
   A methyl ketone of formula (XVI) is reacted with dimethyl oxalate in a suitable solvent in the presence of a suitable base. Suitable solvents include ethers, such as tetrahydrofuran. Suitable bases include sodium hydride, potassium *tert*-butoxide and lithium diisopropylamide. Alternatively, sodium methoxide may be used as the base and methanol as the solvent. 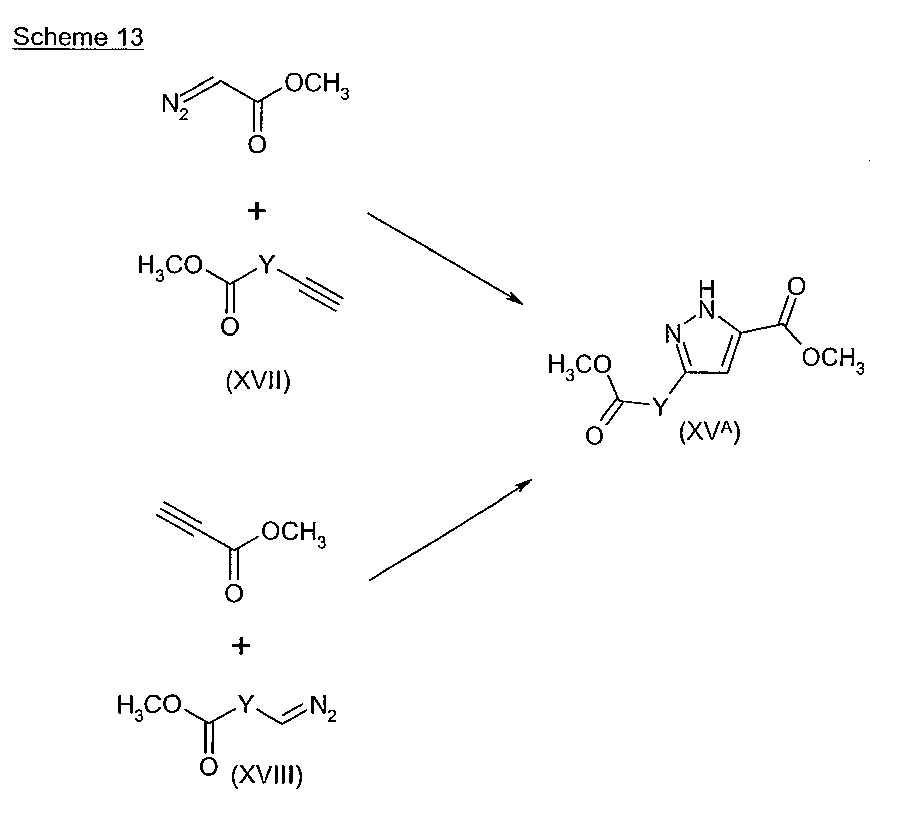
   The method illustrated in Scheme 13 is the Pechmann pyrazole synthesis. A diazo compound and an acetylene are combined to produce a pyrazole of formula (XV^{A}). When Y is other than a covalent bond two variants of the method can be considered. An acetylene of formula (XVII) can be combined with methyl diazoacetate, or a diazo compound of formula (XVIII) can be combined with methyl propiolate. The initial reaction product (XV^{A}) may be carried forward as described above.
   As an alternative to the steps described in parts a) to c) above, compounds of formula (IV) may be elaborated to give compounds of formula (I) by the following method.
l) The monochlorides of formula (IV) can be converted to diamines of formula (XIX) as illustrated in Scheme 14. 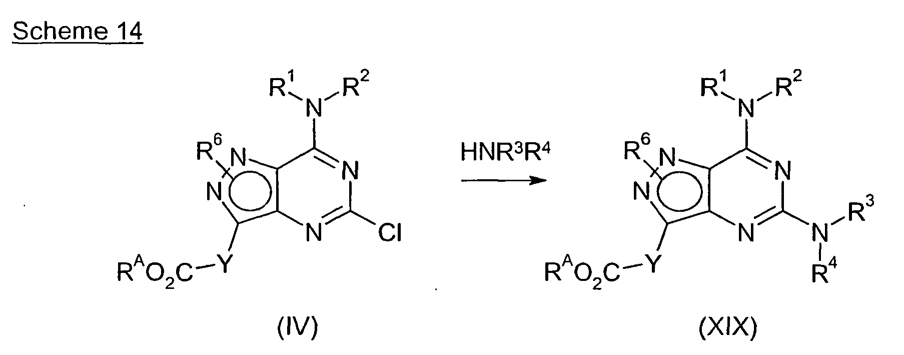 The transformation can be achieved using the methods described in part a) above.
m) The esters of formula (XIX) can be converted to acids of formula (XX) as illustrated in Scheme 15. 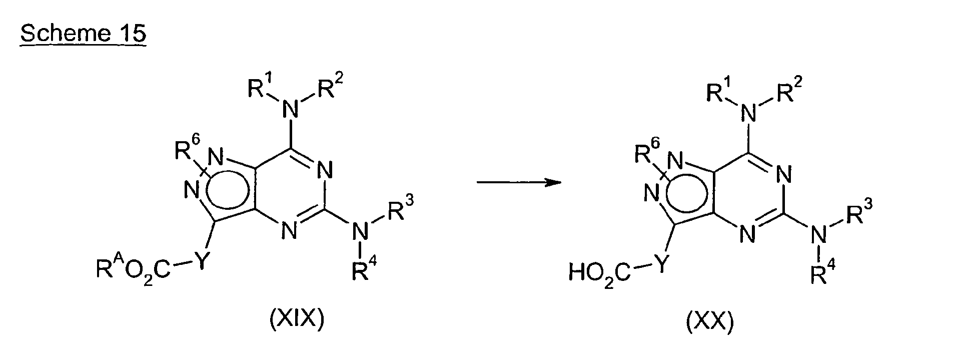
   The transformation can be achieved using the methods described in part c) above.
n) The acids of formula (XX) can be converted to compounds of formula (I) as illustrated in Scheme 16. 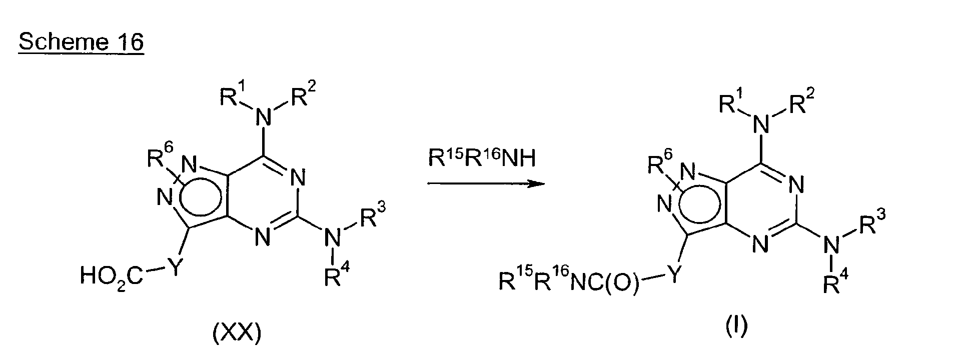
   The transformation can be achieved using the methods described in part b) above.
o) In some embodiments of the compounds of formula (I), the group R⁶ may not be compatible with the synthetic methods described above. An alternative in these circumstances is to introduce the R⁶ group at a late stage, as illustrated in Scheme 17. 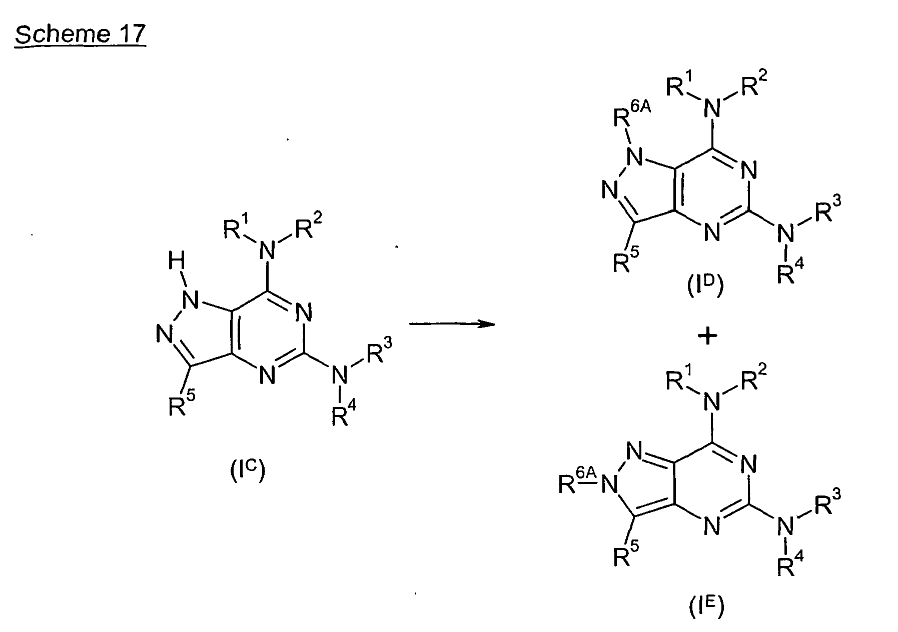
   A compound of formula (I^{C}), i.e. a compound of formula (I) wherein R⁶ is hydrogen, can be alkylated using the methods described in part j) above. The reaction will generally give a mixture of the N¹-alkylated compound (1°) and the N²-isomer (I^{E}). These can be separated and purified by conventional methods. The use of more reactive alkylating agents tends to promote alkylation at the N² position.
   It will be appreciated that the alkylation reaction to introduce R^{6A} might also be carried out at other stages in the synthetic sequence.
   In addition to the methods described above, certain compounds of general formulae (III) and (IV) may be prepared by elaboration of the substituent at the C³ position of the pyrazolopyrimidine, as further illustrated below. It will be appreciated that the synthetic transformations discussed may also be used in the elaboration of the C³-substituent of compounds at any other stage of the synthetic sequence.
p) Compounds of formula (IV^{A}), i.e compounds of formula (IV) wherein Y is CH₂, may be prepared from the corresponding compounds of formula (III^{A}), i.e. compounds of formula (III) wherein Y is a covalent bond, by a one-carbon homologation method such as the Arndt-Eistert reaction illustrated in Scheme 18. 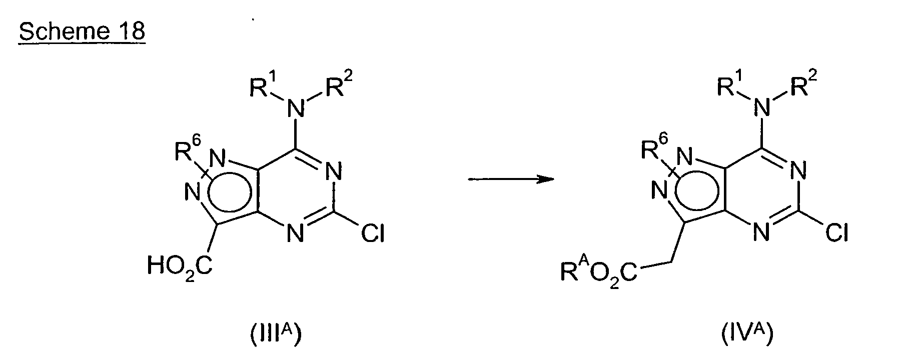
   The carboxylic acid is converted to a reactive intermediate such as the acid chloride (by reaction with oxalyl chloride) or a mixed anhydride (by reaction with isobutyl chloroformate). The intermediate is reacted with diazomethane to provide an α-diazoketone. This is treated with silver oxide in the presence of R^{A}-OH to give the homologated ester of formula (IV^{A}).
q) Compounds of formula (III^{B}), i.e. compounds of formula (III) wherein Y is CH₂, may be prepared from the corresponding nitriles of formula (XXI) by the method illustrated in Scheme 19. 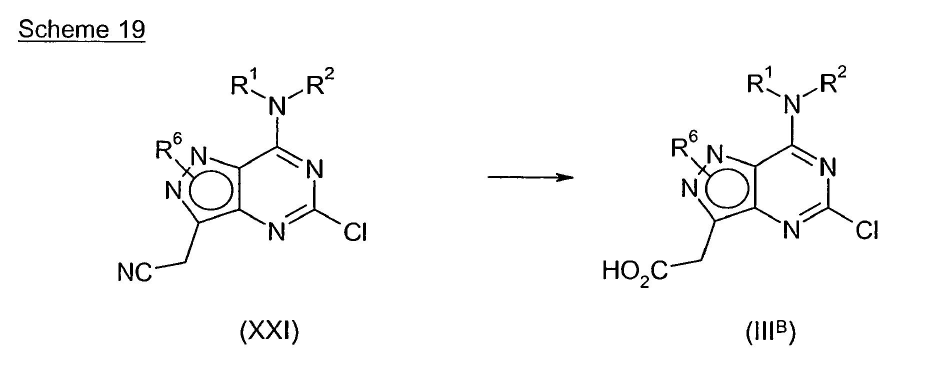
   The nitrile can be hydrolysed by treatment with aqueous mineral acids, such as hydrochloric acid, optionally at elevated temperature.
r) Compounds of formula (XXI) can be prepared from the corresponding chlorides of formula (XXII) by the method illustrated in Scheme 20. 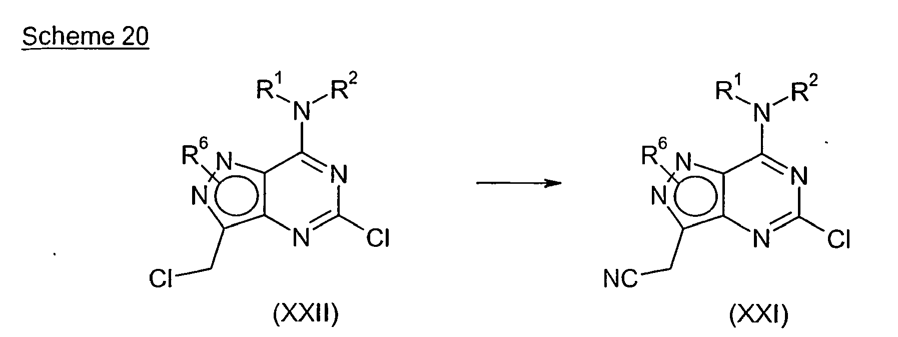
   The chloride is treated with a metal cyanide, such as sodium cyanide or potassium cyanide in a suitable solvent, such as dimethylsulfoxide, dimethylformamide or ethanol.
s) Compounds of formula (XXII) can be prepared from the corresponding alcohols of formula (XXIII) by the method illustrated in Scheme 21. 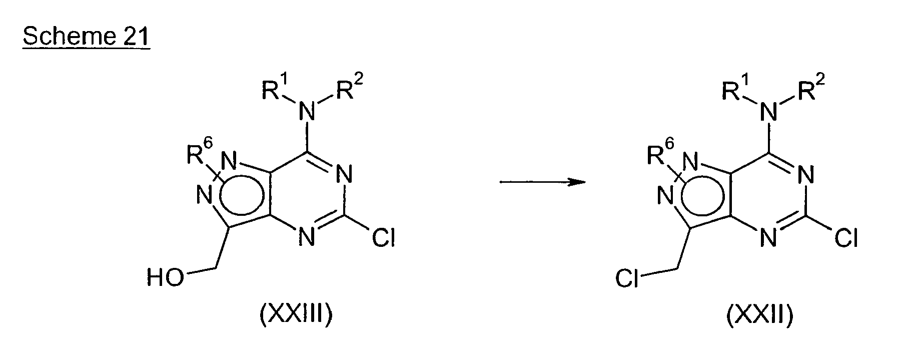
   The alcohol is treated with thionyl chloride or with a mixture of triphenylphosphine and either *N*-chlorosuccinimide or tetrachloromethane.
t) Compounds of formula (XXIII) can be prepared from the corresponding esters of formula (IV^{B}), i.e. compounds according to formula (IV) wherein Y is a covalent bond, or from the corresponding acids of formula (III^{A}) by the method illustrated in Scheme 22. 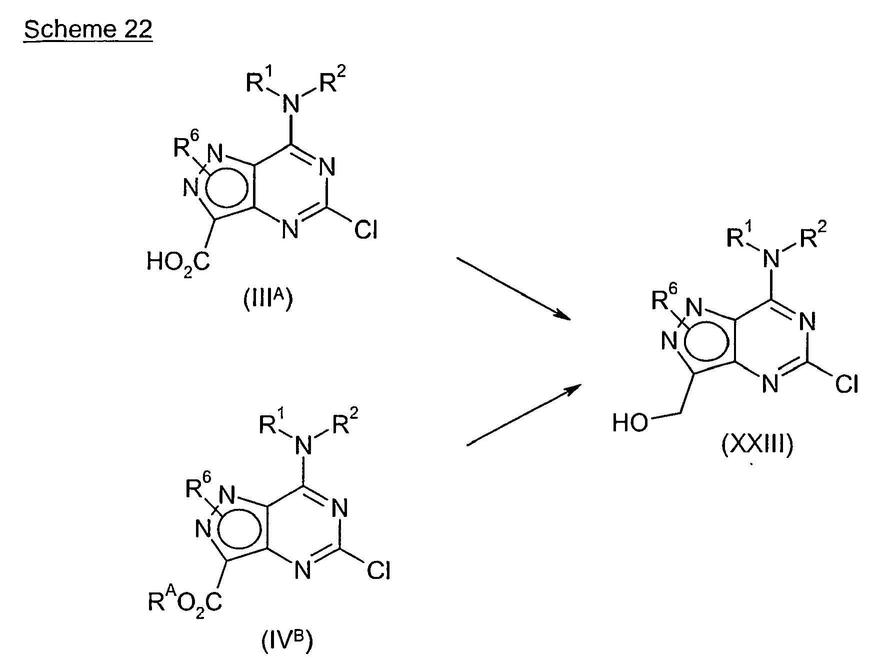
   The acids of formula (III^{A}) and the esters of formula (IV^{B}) can be reduced to the alcohols of formula (XXIII) by treatment with lithium aluminium hydride in a suitable solvent at a temperature of between 0° and the boiling point of the solvent. Suitable solvents include ethers such as tetrahydrofuran. The acids can also be reduced by treatment with isobutyl chloroformate and a tertiary amine base to provide a mixed anhydride, followed by reaction with sodium borohydride. The esters can also be reduced by treatment with diisobutylaluminium hydride or lithium borohydride.
u) Compounds of formula (IV^{C}), i.e. compounds of formula (IV) wherein Y is CH₂CH₂, can be prepared from the corresponding acrylate ester of formula (XXIV) by the method illustrated in Scheme 23. 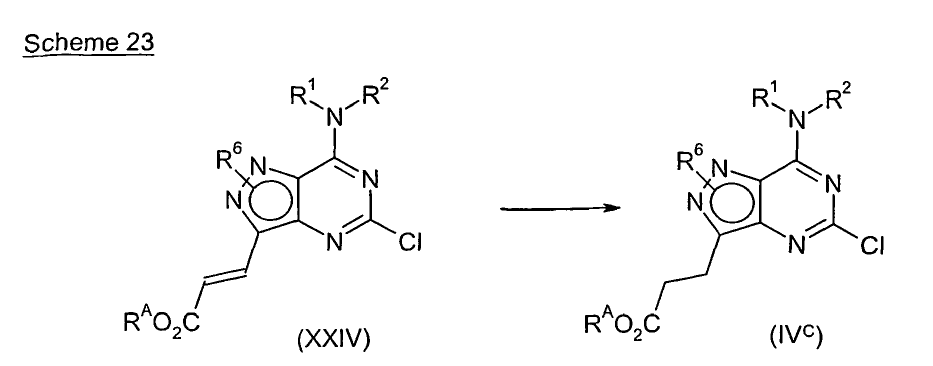
   The reduction of the carbon-carbon double bond of (XXIV) to give the compounds of formula (lV^{C}) can be accomplished by catalytic hydrogenation using molecular hydrogen in the presence of a transition metal catalyst such as palladium, platinum or nickel. When R^{A} is benzyl the conditions can be chosen such that only the double bond is reduced or reduction is accompanied by hydrogenolytic cleavage of the ester to give the carboxylic acid.
   The acrylates of formula (XXIV) can also be treated with alkylcopper reagents to give analogues of the compounds of formula (IV^{c}) in which an alkyl substituent is introduced on the carbon atom adjacent to the pyrazolopyrimidine ring system, or with a sulphonium ylid or a carbene equivalent to give a 2-(pyrazolopyrimidinyl)-cyclopropane-1-carboxylate derivative.
v) Compounds of formula (XXIV) can be prepared from the corresponding aldehydes of formula (XXV) by the method illustrated in Scheme 24. 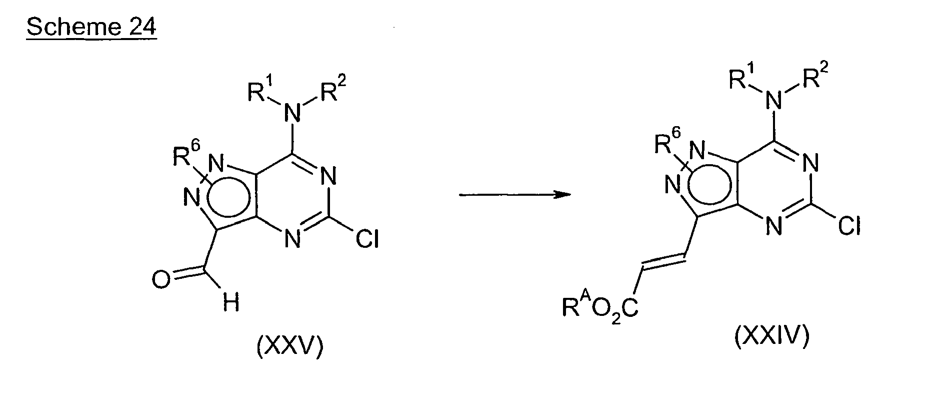
   The aldehyde of formula (XXV) can be converted to the acrylate ester of formula (XXIV) by reaction with a phosphorus reagent following the protocols of the Wittig, Horner or Wadsworth-Horner-Emmons reactions. The reagent is prepared by treating a triphenylphosphonium salt Ph₃P⁺CH₂CO₂R^{A}.X⁻ (Wittig), a phosphine oxide Ph₂P(O)CH₂CO₂R^{A} (Horner), or a phosphonate (EtO)₂P(O)CH₂CO₂R^{A} (Wadsworth-Horner-Emmons), with a base such as butyllithium, a lithium dialkylamide or an alkaline metal alkoxide, in a suitable solvent such as tetrahydrofuran.
   The method is not limited to the preparation of α-unsubstituted acrylate esters. The use of an alkyl-substituted phosphorus reagent such as Ph₃P⁺CH(R)CO₂R^{A}.X⁻ or the equivalent phosphine oxide or phosphonate, wherein R is alkyl, gives access to the corresponding α-alkyl acrylate derivative.
   The conversion of the aldehydes of formula (XXV) to acrylate esters of formula (XXIV) can also be achieved by reaction with a malonate derivative following the method of the Knoevenagel condensation.
w) Compounds of formula (XXV) can be prepared from the esters of formula (IV^{B}) or more preferably from the corresponding alcohols of formula (XXIII) by the methods illustrated in Scheme 25. 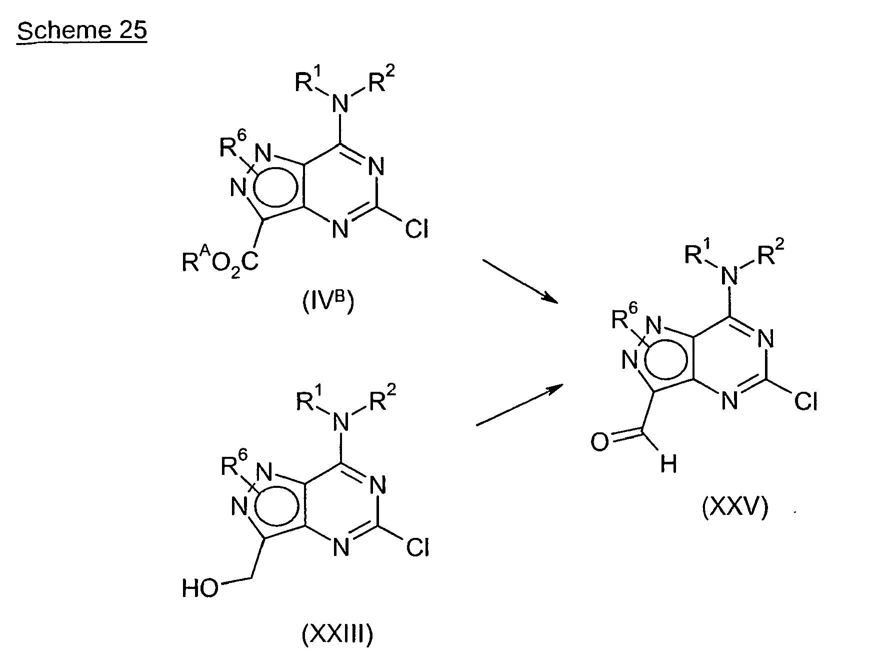
   The reduction of the esters of formula (IV^{B}) can be achieved using diisobutylaluminium hydride (DIBAL) in a suitable solvent at a temperature of less than 0°C, preferably less than -60°C. Suitable solvents include hydrocarbons such as pentane, hexane and toluene, ethers such as tetrahydrofuran, and mixtures thereof.
   The oxidation of the alcohols of formula (XXIII) can be achieved using a chromium(VI) reagent such as pyridinium chlorochromate, a hypervalent iodine reagent such as the Dess-Martin periodinane, or a combination of tetra-n-propylammonium perruthenate and N-methylmorpholine-N-oxide in a suitable solvent at a temperature of between 0°C and ambient temperature. Suitable solvents include dichloromethane.
x) The aldehydes of formula (XXV) may be converted to esters of formula (IV^{A}) as illustrated in Scheme 26 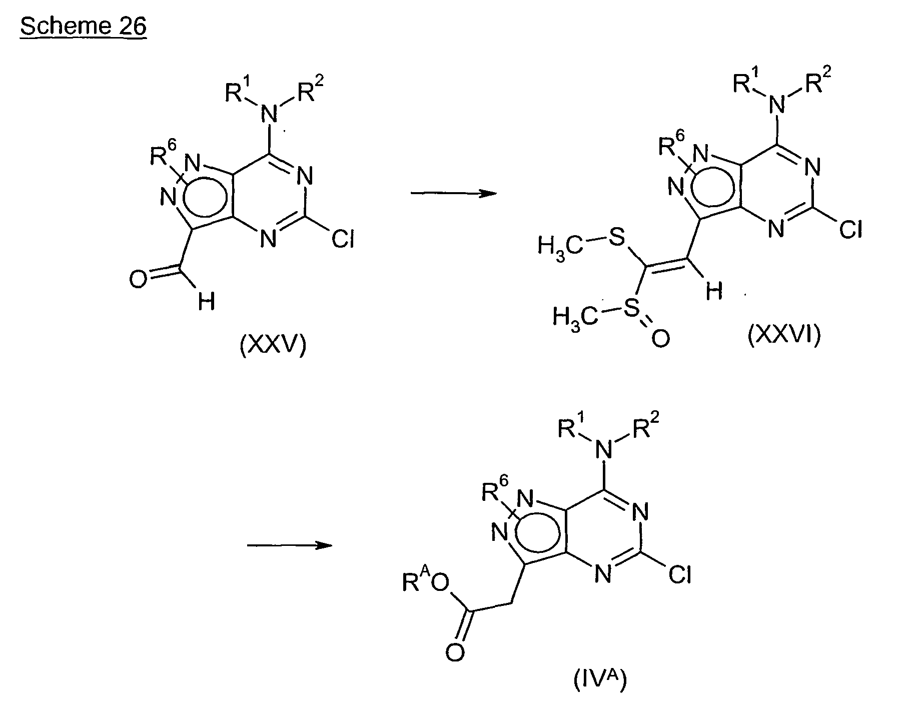
   The aldehyde is treated with methyl methylmercaptomethyl sulfoxide (CH₃SCH₂S(O)CH₃) and triton B in tetrahydrofuran to give intermediate (XXVI) which is treated with the appropriate alcohol R^{A}OH and acetyl chloride to provide the ester of formula (IV^{A}). This method is particularly useful when R^{A} is methyl.
y) Compounds of formula (IV^{c}) can also be prepared from the corresponding chlorides of formula (XXII) by the method illustrated in Scheme 27. 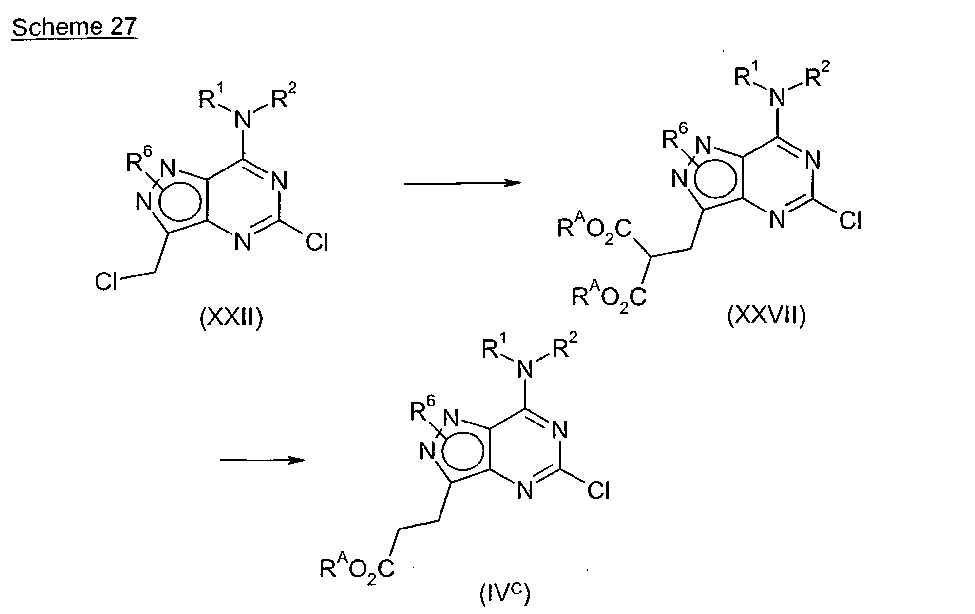
   The chloride of formula (XXII) is reacted with a dialkyl malonate (R^{A}O₂C)₂CH₂ and a base in a suitable solvent. Typically, the base is an alkaline metal alkoxide such as sodium ethoxide or potassium *tert*-butoxide, and the solvent is an alcohol such as ethanol or an ether such as tetrahydrofuran. Preferably the base and the solvent are chosen such as to minimise transesterification with the malonate reagent and the intermediate (XXVII). For example, when the reagent is diethyl malonate the base is preferably sodium ethoxide and the solvent is ethanol. The intermediate (XXVII) is then decarboxylated to give the product (IV^{C}). This can be achieved by selective hydrolysis using one equivalent of an alkaline metal hydroxide, such as sodium hydroxide, followed by acidification, or by any other method known in the art.
   The method is not limited to symmetrical malonates. For example, the use of *tert-*butyl methyl malonate would give an intermediate (XXVII) in which one R^{A} is methyl and the other is *tert*-butyl. By choosing the appropriate conditions, decarboxylation could then be controlled to give a product (IV^{C}) in which R^{A} was either *tert*-butyl or methyl.
   The method can be extended to substituted malonates (R^{A}O₂C)₂CHR, where R is an alkyl group. This gives access to compounds analogous to (IV^{c}) in which the group R is a substituent on the carbon atom adjacent to the R^{A}O₂C group. These compounds can also be prepared by alkylating the intermediate (XXVII) with R-Br or R-I in the presence of an alkaline metal alkoxide base.
z) As mentioned in part d) above, the reaction of compounds of formula (V^{A}), i.e. compounds of formula (V) wherein Y is a covalent bond, with weakly nucleophilic amines HNR¹R² is sometimes not high yielding. An alternative route is illustrated in Schemes 28A and 28B. 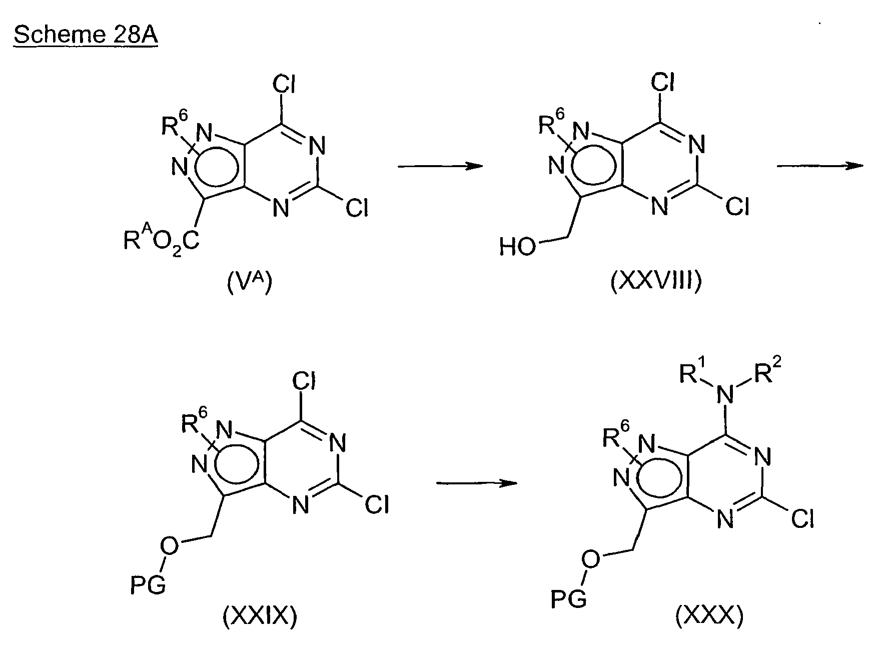
   The esters of formula (V^{A}) can be reduced to the alcohols of formula (XXVIII) according to the methods described in part t) above. A preferred method is reduction with diisobutylaluminium hydride at a temperature of between -20°C and 0°C. The primary alcohol is then protected to give compounds of formula (XXIX), wherein PG is an alcohol protecting group. A preferred protecting group is a trialkylsilyl group, particularly a *tert*-butyldimethylsilyl group. The compounds of formula (XXIX) are then reacted with an amine HNR¹R² according to the methods described in part d) above to give compounds of formula (XXX). 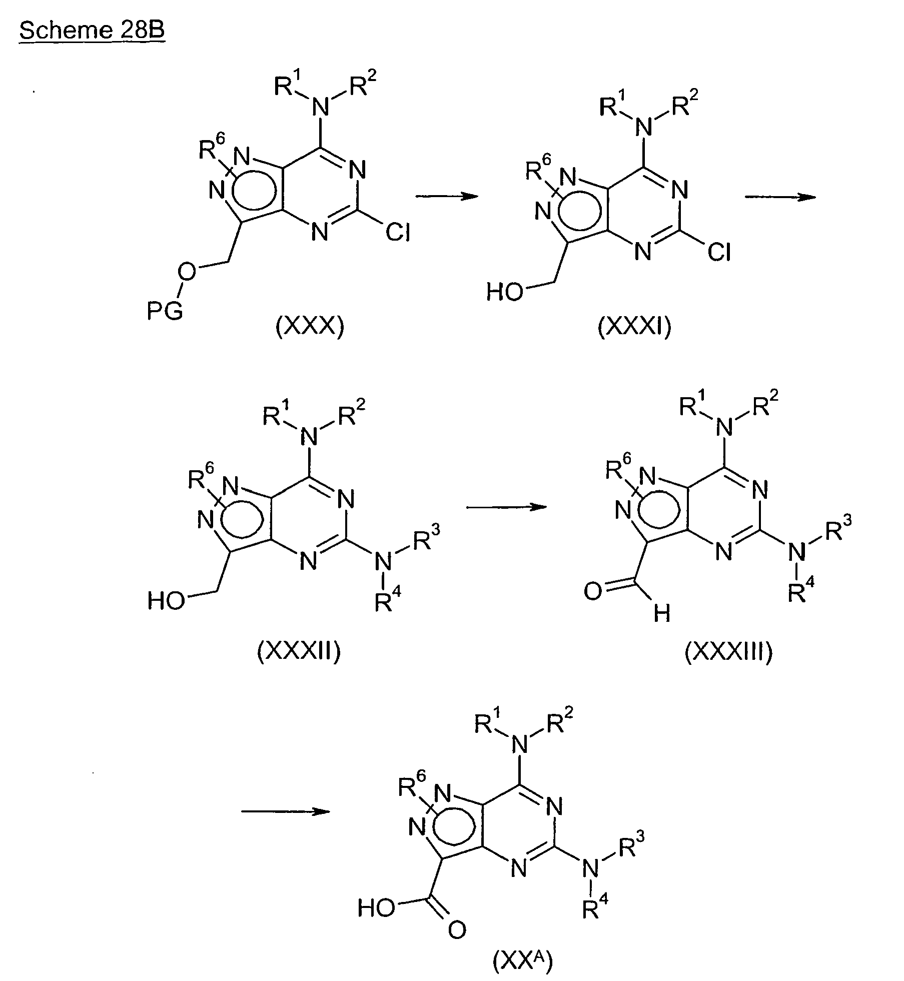
   The compounds of formula (XXX) are deprotected to provide the primary alcohols of formula (XXXI) using appropriate conditions. When PG is a trialkylsilyl group it may be removed by treatment with a fluoride salt, such as tetrabutylammonium fluoride. The -NR³R⁴ group is then introduced according to the methods described in part a) above to provide compounds of formula (XXXII). The primary alcohol is oxidised as described in part w) above to provide the aldehydes of formula (XXXIII). A preferred oxidising agent is the Dess-Martin periodinane. Finally the aldehydes of formula (XXXIII) are oxidised to provide the acids of formula (XX^{A}), i.e. compounds of formula (XX) wherein Y is a covalent bond. Suitable oxidising agents include potassium permanganate, Jones' reagent and sodium chlorite. A preferred method is to treat the aldehydes with sodium chlorite, sodium dihydrogenphosphate and 2-methyl-2-butene in *tert*-butanol at room temperature for about 1 hour.

The following compounds form further aspects of the present invention:
a compound of formula (II) 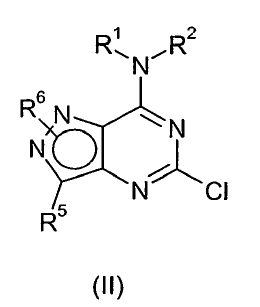
wherein R¹, R², R⁵ and R⁶ are as defined above; and
a compound of formula (III) 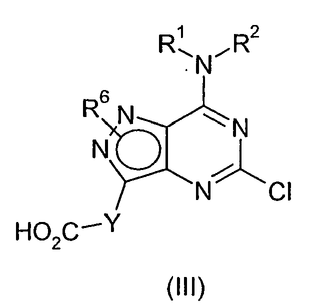 wherein R¹, R², R⁶ and Y are as defined above.

The invention is further illustrated by the following, non-limiting examples. Melting points were determined on a Gallenkamp melting point apparatus using glass capillary tubes and are uncorrected. Unless otherwise indicated all reactions were carried out under a nitrogen atmosphere, using commercially available anhydrous solvents. '0.88 Ammonia' refers to commercially-available aqueous ammonia solution of about 0.88 specific gravity. Thin-layer chromatography was performed on glass-backed pre-coated Merck silica gel (60 F254) plates, and silica gel column chromatography was carried out using 40-63µm silica gel (Merck silica gel 60). Ion exchange chromatography was performed using with the specified ion exchange resin which had been pre-washed with deionised water. Proton NMR spectra were measured on a Varian Inova 300, Varian Inova 400, or Varian Mercury 400 spectrometer in the solvents specified. In the NMR spectra, only non-exchangeable protons which appeared distinct from the solvent peaks are reported. Low resolution mass spectra were recorded on either a Fisons Trio 1000, using thermospray positive ionisation, or a Finnigan Navigator, using electrospray positive or negative ionisation. High resolution mass spectra were recorded on a Bruker Apex II FT-MS using electrospray positive ionisation. Combustion analyses were conducted by Exeter Analytical UK. Ltd., Uxbridge, Middlesex. Optical rotations were determined at 25°C using a Perkin Elmer 341 polarimeter using the solvents and concentrations specified. Example compounds designated as (+) or (-) optical isomers are assigned based on the sign of optical rotation when determined in a suitable solvent.

### Abbreviations, Definitions and Glossary

- AcOH: acetic acid
- Ambertyst^{®} 15: Ion exchange resin, available from Aldrich Chemical Company
- APCI: Atmospheric Pressure Chemical lonisation
- Arbocel^{™}: Filtration agent, from J. Rettenmaier & Sohne, Germany
- atm: Pressure in atmospheres (1 atm = 760 Torr = 101.3 kPa)
- Biotage^{™}: Chromatography performed using Flash 75 silica gel cartridge, from Biotage, UK
- BOC: *tert*-butoxycarbonyl
- br: Broad
- *c*: Concentration used for optical rotation measurements in g per 100 ml (1 mg/ml is *c* 0.10)
- cat: Catalytic
- CBz: benzyloxycarbonyl
- CDI: N,N'-carbonyldiimidazole
- d: Doublet
- DCC: N,N'-dicyclohexylcarbodiimide
- DCM: dichloromethane
- dd: Doublet of doublets
- DEAD: diethyl azodicarboxylate
- Degussa^{®} 101: 10 wt% palladium on activated carbon, Degussa type E101 available from Aldrich Chemical Company
- Dess-Martin periodinane: 1,1,1-triacetoxy-1,1-dihydro-1,2-benziodoxol-3(1H)-one
- Develosil: Supplied by Phenomenex - manufactured by Nomura Chemical
- Combi-RP C₃₀: Co. Composed of spherical silica particles (size 3 µm or 5 µm)
- hplc column: which have a chemically bonded surface of C30 chains. These particles are packed into stainless steel columns of dimensions 2 cm internal diameter and 25 cm long.
- DIAD: diisopropyl azodicarboxylate
- DIBAL: diisobutylaluminium hydride
- DMAP: 4-dimethylaminopyridine
- DMF: N,N-dimethylformamide
- DMSO: dimethyl sulphoxide
- Dowex^{®}: Ion exchange resin, from Aldrich Chemical Company
- ee: Enantiomeric excess
- Et₃N: triethylamine
- EtOAc: ethyl acetate
- EtOH: ethanol
- HOAT: 1-hydroxy-7-azabenzotriazole
- HOBT: 1-hydroxybenzotriazole hydrate
- HRMS: High Resolution Mass Spectrocopy (electrospray ionisation positive scan)
- Hünig's base: N-ethyldiisopropylamine
- Hyflo^{™}: Hyflo supercel^{®}, from Aldrich Chemical Company
- KHMDS: potassium bis(trimethylsilyl)amide
- liq: Liquid
- LRMS: Low Resolution Mass Spectroscopy (electrospray or thermospray ionisation positive scan)
- LRMS (ES⁻): Low Resolution Mass Spectroscopy (electrospray ionisation negative scan)
- m: Multiplet
- m/z: Mass spectrum peak
- MCI^{™} gel: High porous polymer, CHP20P 75-150µm, from Mitsubishi Chemical Corporation
- MeOH: methanol
- Mukaiyama's reagent: 2-chloro-1-methylpyridinium iodide
- NaHMDS: sodium bis(trimethylsilyl)amide
- NMM: N-methylmorpholine
- NMO: 4-methylmorpholine N-oxide
- NMP: 1-methyl-2-pyrrolidinone
- Phenomenex: Supplied by Phenomenex. Composed of spherical silica particles
- Luna C18 hplc: (size 5 µm or 10 µm) which have a chemically bonded surface of
- column: C18 chains. These particles are packed into a stainless steel column of dimensions 2.1cm internal diameter and 25 cm long.
- psi: Pounds per square inch (1 psi = 6.9 kPa)
- PyBOP®: Benzotriazol-1-yloxytris(pyrrolidino)phosphonium hexafluorophosphate
- PyBrOP®: bromo-tris-pyrrolidino-phosphonium hexafluorophosphate
- q: Quartet
- R_{f}: Retention factor on TLC
- s: Singlet
- Sep-Pak^{®}: Reverse phase C₁₈ silica gel cartridge, Waters Corporation
- t: Triplet
- TBDMS-Cl: *tert*-butyldimethylchlorosilane
- TFA: trifluoroacetic acid
- THF: tetrahydrofuran
- TLC: Thin Layer Chromatography
- TMS-Cl: chlorotrimethylsilane
- WSCDI: 1-(3-dimethylaminopropyl)-3-ethylcarbodiimide hydrochloride
- δ: Chemical shift

The following Examples illustrate the preparation of the compounds of the formula (I):-

### Preparation 1

### Dimethyl 1-(2-ethoxyethyl)-4-nitro-1H-pyrazole-3,5-dicarboxylate

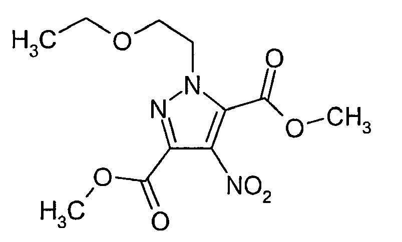

Potassium carbonate (1.32g, 9.57mmol) and 2-ethoxyethyl bromide (1.18mL, 9.57mmol) were added to a solution of dimethyl 4-nitro-1H-pyrazole-3,5-dicarboxylate (EP 1241170, pg. 50, preparation 10) (2g, 9.57mmol) in N,N-dimethylformamide (35mL) and the reaction mixture was stirred at room temperature for 18 hours. The reaction mixture was concentrated *in vacuo* and the residue was partitioned between ethyl acetate (200mL) and water (100mL). The organic phase was dried over magnesium sulphate and concentrated *in vacuo.* The crude product was purified by column chromatography on silica eluting with pentane:ethyl acetate 100:0 to 70:30 in 10% increments to yield the title product, 1.63g.
¹H-NMR (CDCl₃, 400MHz) δ: 1.07 (t, 3H), 3.41 (m, 2H), 3.73 (t, 2H), 3.89 (s, 3H), 3.94 (s, 3H), 4.76 (t, 2H). MS APCI+ m/z 302 [MH]⁺

### Preparation 2

### Dimethyl 1-methyl-4-nitro-1H-pyrazole-3,5-dicarboxylate

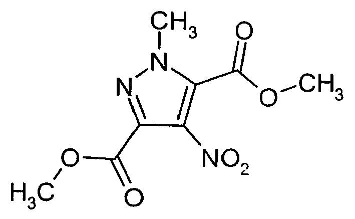

A solution of dimethyl 4-nitro-1*H*-pyrazole-3,5-dicarboxylate (EP 1241170, pg. 50, preparation 10) (30g, 0.131mol) in N,N-dimethylformamide (250mL) was treated with caesium carbonate (42.66g, 0.130mol). The reaction mixture was stirred at room temperature for 1 hour and then treated with dimethyl sulphate (12.39mL, 0.130mol). The reaction mixture was stirred at room temperature for 18 hours and was then concentrated *in vacuo.* The residue was partitioned between dichloromethane (550mL) and water (550mL) and the aqueous phase was washed with dichloromethane (2x450mL). The combined organic phases were dried over magnesium sulphate and concentrated *in vacuo* to yield the title product as a white solid, 28.51g.
¹H-NMR (DMSO-D₆, 400MHz) δ: 3.83 (m, 6H), 4.12 (s, 3H). MS APCI+ m/z 244 [MH]⁺

### Preparation 3

### Dimethyl 1-isobutyl-4-nitro-1H-pyrazole-3,5-dicarboxylate

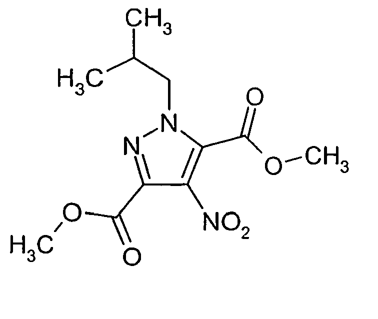

Dimethyl 4-nitro-1*H*-pyrazole-3,5-dicarboxylate (EP 1241170, pg. 50, preparation 10) (12.5g, 54.6mmol), 2-methyl-1-propanol (4.95g, 60mmol) and triphenylphosphine (15.72g, 60mmol) were dissolved in tetrahydrofuran (150mL) and the reaction mixture was cooled to 0°C in an ice bath. The reaction mixture was treated with diisopropyl azodicarboxylate (12.12g, 60mmol), allowed to return to room temperature and then stirred at room temperature for 18 hours. The reaction mixture was concentrated *in vacuo* and the residue was dissolved in pentane:ethyl acetate 3:1 (300mL). The solids formed were filtered off and the organic layer separated and adsorbed onto silica. This was purified by column chromatography on silica gel eluting with pentane:ethyl acetate 9:1 to yield the title product.
¹H-NMR (CDCl₃, 400MHz) δ: 0.93 (d, 6H), 2.26 (m, 1 H), 3.93 (2xs, 6H), 4.41 (m, 2H). MS APCl+ m/z 286 [MH]⁺

### Preparation 4

### Dimethyl 4-nitro-1-(2-propoxyethyl)-1H-pyrazole-3,5-dicarboxylate

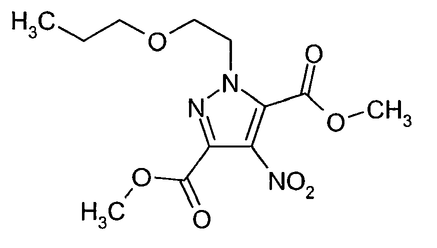

Dimethyl 4-nitro-1*H*-pyrazole-3,5-dicarboxylate (EP 1241170, pg. 50, preparation 10) (15g, 60mmol), 2-propoxyethanol (8.2mL, 70mmol) and triphenylphosphine (18.9g, 70mmol) were dissolved in tetrahydrofuran (150mL) and the reaction mixture cooled to 0°C. The reaction mixture was treated with diisopropyl azodicarboxylate (14.2mL, 70mmol) and the reaction mixture stirred at 0°C for 3 hours before being allowed to warm to room temperature. The reaction mixture was concentrated *in vacuo* and the residue purified by column chromatography on silica gel eluting with ethyl acetate:pentane 15:85 to yield the title product.
¹H-NMR (CD₃OD, 400MHz) δ: 0.82 (t, 3H), 1.47 (m, 2H), 3.34 (t, 2H), 3.78 (t, 2H), 3.91 (m, 6H), 4.76 (t, 2H). MS APCI+ m/z 316 [MH]⁺

### Preparation 5

### 1-(2-Ethoxyethyl)-4-nitro-1H-pyrazole-3,5-dicarboxylic acid 3-methyl ester

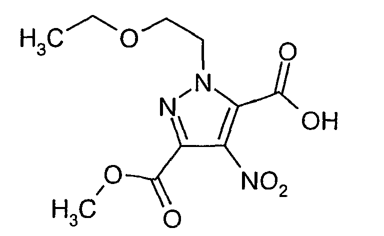

The di-ester of preparation 1 (1.63g, 5.4mmol) was added to a solution of potassium hydroxide (300mg, 5.9mmol) in methanol (20mL) and the reaction mixture stirred at room temperature for 18 hours. The reaction mixture was concentrated *in vacuo* and the residue dissolved in water (100mL) and washed with ether. The aqueous phase was acidified with 2M hydrochloric acid and extracted with dichloromethane (3x100mL). The organic phases were combined, dried over magnesium sulphate and concentrated *in vacuo* to yield the title product, 1.34g.
¹H-NMR (CD₃OD, 400MHz) δ : 1.07 (t, 3H), 3.47 (m, 2H), 3.80 (t, 2H), 3.88 (s, 3H), 4.77 (t, 2H). MS APCI+ m/z 288 [MH]⁺

### Preparations 6-8

The following compounds were prepared by a method similar to that described for preparation 5 using the appropriate di-ester.

| | | |
|---|---|---|
| 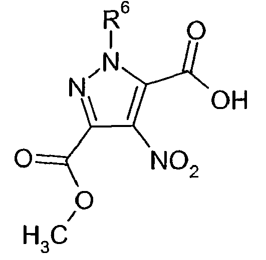 | | |

| No. | R⁶ | Data |
|---|---|---|
| 6 | -CH₂CH(CH₃)₂ | ¹H-NMR (CDCl₃, 400MHz) δ: 0.92 (d, 6H), 2.27 (m, 1H), 3.99 (s, 3H), 4.42 (m, 2H). MS APCI+ m/z 272 [MH]⁺ |
| 7 | -CH₃ | ¹H-NMR (CDCl₃, 400MHz) δ: 3.91 (s, 3H), 4.22 (s, 3H), 8.10 (m, 1 H). MS APCI+ m/z 230 [MH]⁺ |
| 8 | -(CH₂)₂O(CH₂)₂CH₃ | ¹H-NMR (CD₃OD, 400MHz) δ: 0.83 (t, 3H), 1.49 (m, 2H), 3.36 (t, 2H), 3.80 (t, 2H), 3.90 (s, 3H), 4.78 (t, 2H). MS APCI+ m/z 302, [MH]⁺, |

### Preparation 9

### Methyl 5-carbamoyl-1-(2-ethoxyethyl)-4-nitro-1H-pyrazole-3-carboxylate

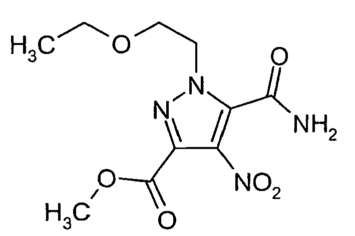

Oxalyl chloride (15.7mL, 190mmol) was added steadily to a solution of the carboxylic acid of preparation 5 (17.1 g, 59.5mmol) in dichloromethane (300mL). N,N-dimethylformamide (46µL, 6mmol) was then added and the reaction mixture stirred for 2 hours. The reaction mixture was concentrated *in vacuo* and the residue azeotroped from dichloromethane (3x200mL). The product was dissolved in tetrahydrofuran (300mL), cooled in ice, treated with 0.88 ammonia (200mL) and stirred for 18 hours at room temperature. The reaction mixture was concentrated in *vacuo* and the residue partitioned between water (200mL) and ethyl acetate. The organics were dried over magnesium sulphate and concentrated *in vacuo* to yield the crude product which triturated in ether to yield the title product, 8.2g.
¹H-NMR (DMSO-D₆, 400MHz) δ: 1.03 (t, 3H), 3.38 (m, 2H), 3.70 (t, 2H), 3.86 (s, 3H), 4.36 (t, 2H), 8.30 (m, 1 H), 8.46 (m, 1 H). MS APCI+ m/z 287 [MH]⁺

### Preparations 10-12

The following compounds were prepared by a method similar to that described for preparation 9 using the appropriate carboxylic acid.

| | | |
|---|---|---|
| 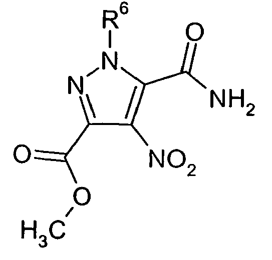 | | |

| No. | R⁶ | Data |
|---|---|---|
| 10 | -CH₂CH(CH₃)₂ | ¹H-NMR (CDCl₃, 400MHz) δ: 0.91 (d, 6H), 2.27 (m, 1 H), 3.98 (s, 3H), 4.36 (m, 2H), 7.23 (m, 2H). MS APCI+ m/z 271 [MH]⁺ |
| 11 | -CH₃ | ¹H-NMR (DMSO-D₆, 400MHz) δ: 3.84 (s, 3H), 3.86 (s, 3H), 8.38 (m, 1 H), 8.50 (m, 1 H). MS APCI+ m/z 229 [MH]⁺ |
| 12 | -(CH₂)₂O(CH₂)₂CH₃ | ¹H-NMR (DMSO-D₆, 400MHz) δ: 0.81 (t, 3H), 1.45 (m, 2H), 3.32 (t, 2H), 3.74 (t, 2H), 3.90 (s, 3H), 4.40 (t, 2H), 8.33 (s, 1 H), 8.48 (s, 1 H). MS APCI+ m/z 301 [MH]⁺ |

### Preparation 13

### Methyl 4-amino-5-carbamoyl-1-(2-ethoxyethyl)-1H-pyrazole-3-carboxylate

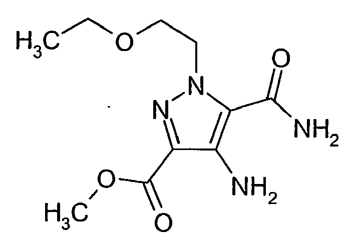

Palladium(II) hydroxide on carbon (1g) was added to a solution of the nitro compound of preparation 9 (8.2g, 28.6mmol) in methanol (300mL). Ammonium formate (8.8g, 0.14mol) was added portionwise to the reaction mixture over 20 minutes and the reaction mixture then stirred at reflux for 2 hours. The reaction mixture was cooled to room temperature and filtered to remove catalyst. The filtrate was concentrated *in vacuo* and azeotroped with toluene to yield the title product, 7.3g.
¹H-NMR (DMSO-D₆, 400MHz) δ: 1.04 (t, 3H), 3.32 (m, 2H), 3.66 (t, 2H), 3.78 (s, 3H), 4.49 (t, 2H), 5.12 (m, 2H), 7.50 (m, 2H). MS APCI+ m/z 257 [MH]⁺

### Preparations 14-16

The following compounds were prepared by a method similar to that described for preparation 13 using the appropriate ester.

| | | |
|---|---|---|
| 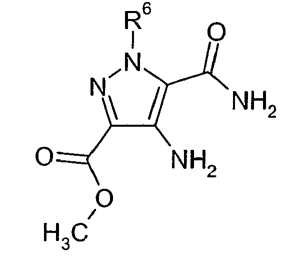 | | |

| No. | R⁶ | Data |
|---|---|---|
| 14 | -CH₂CH(CH₃)₂ | ¹H-NMR (CDCl₃, 400MHz) δ: 0.87 (d, 6H), 2.22 (m, 1 H), 3.97 (s, 3H), 4.40 (m, 2H), 4.44 (m, 2H), 6.02 (m, 2H). MS APCI+ m/z 241 [MH]⁺ |
| 15 | -CH₃ | ¹H-NMR (DMSO-D₆, 400MHz) δ: 3.69 (s, 3H), 3.92 (s, 3H), 5.17 (m, 2H), 7.37 (brm, 2H). MS APCI+ m/z 199 [MH]⁺ |
| 16 | -(CH₂)₂O(CH₂)₂CH₃ | ¹H-NMR (CD₃OD, 400MHz) δ: 0.84 (t, 3H), 1.51 (m, 2H), 3.40 (t, 2H), 3.83 (t, 2H), 3.89 (s, 3H), 4.56 (t, 2H). MS APCI+ m/z 271, [MH]⁺, |

### Preparation 17

### Methyl 1-(2-ethoxyethyl)-5,7-dioxo-4,5,6,7-tetrahydro-1H-pyrazolo[4,3-d]pyrimidine-3-carboxylate

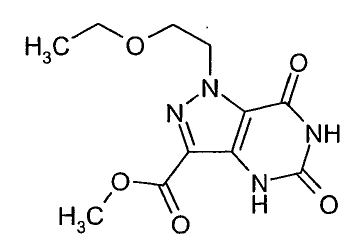

N,N'-Carbonyldiimidazole (5.54g, 34.2mmol) was added to a solution of the amide of preparation 13 (7.3g, 28.5mol) in N,N-dimethylformamide (250mL) and the reaction mixture stirred at room temperature for 1 hour and then at 90°C for 18 hours. The reaction mixture was allowed to cool to room temperature and concentrated in *vacuo.* The residue was sonicated in acetone (200mL) and concentrated *in vacuo* to yield the title product, 5.3g.
¹H-NMR (DMSO-D₆, 400MHz) δ: 0.99 (t, 3H), 3.37 (m, 2H), 3.77 (t, 2H), 3.82 (s, 3H), 4.64 (t, 2H). MS ES- m/z 281 [M-H]⁻

### Preparations 18-20

The following compounds were prepared by a method similar to that described for preparation 17 using the appropriate ester.

| | | |
|---|---|---|
| 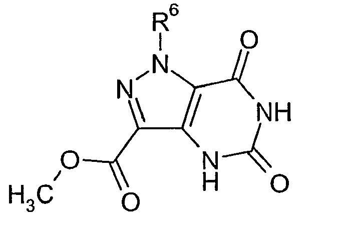 | | |

| No. | R⁶ | Data |
|---|---|---|
| 18 | -CH₂CH(CH₃)₂ | ¹H-NMR (DMSO-D₆, 400MHz) δ: 0.82 (d, 6H), 2.16 (m, 1 H), 3.83 (s, 3H), 4.32 (m, 2H), 10.75 (m, 1 H), 11.34 (m, 1 H). MS APCI+ m/z 267 [MH]⁺ |
| 19 | -CH₃ | ¹H-NMR (DMSO-D₆, 400MHz) δ: 3.80 (s, 3H), 4.08 (s, 3H). MS APCI- m/z 223 [M-H]⁻ |
| 20 | -(CH₂)₂O(CH₂)₂CH₃ | ¹H-NMR (DMSO-D₆, 400MHz) δ: 0.72 (t, 3H), 1.37 (m, 2H), 3.28 (t, 2H), 3.76 (t, 2H), 3.82 (s, 3H), 4.64 (t,2H),10.77(s,1H),11.37(s,1H). MS APCI+ m/z 295, [M-H]⁻ |

### Preparation 21

### Methyl 5,7-dichloro-1-(2-ethoxyethyl)-1H-pyrazolo[4,3-d]pyrimidine-3-carboxylate

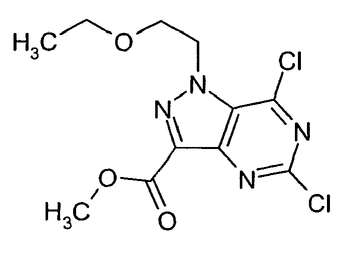

Phosphorous oxychloride (6.5mL, 70mmol) and tetraethylammonium chloride (3.47g, 21 mmol) were added to a solution of the dione of preparation 17 (1.97g, 7mmol) in propionitrile (28mL) and the reaction mixture refluxed for 4 hours. Additional phosphorous oxychloride (2.5mL) was added and the reaction mixture was then stirred at reflux for 18 hours. The reaction mixture was concentrated in *vacuo* and the residue re-dissolved in propionitrile (50mL) and phosphorous oxychloride (6.5mL) and stirred at reflux for a further 18 hours. The reaction mixture was then concentrated *in vacuo* and the residue partitioned between dichloromethane (300mL) and water (50mL). The organics were dried over magnesium sulphate and concentrated *in vacuo.* The crude product was purified by column chromatography on silica, eluting with ethyl acetate:pentane 0:100 to 25:75 to yield the title product, 1.98g.
¹H-NMR (CDCl₃, 400MHz) δ: 1.03 (t, 3H), 3.40 (m, 2H), 3.87 (t, 2H), 4.06 (s, 3H), 4.98 (t, 2H). MS APCI+ m/z 319 [MH]⁺

### Preparations 22-24

The following compounds were prepared by a method similar to that described for preparation 21 using the appropriate ester.

| | | |
|---|---|---|
| 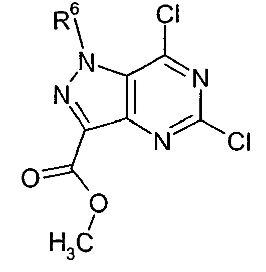 | | |

| No. | R⁶ | Data |
|---|---|---|
| 22 | -CH₂CH(CH₃)₂ | ¹H-NMR (CDCl₃, 400MHz) δ: 0.95 (d, 6H), 2.38 (m, 1 H), 4.08 (s, 3H), 4.61 (m, 2H). MS APCI + m/z 303 [MH]⁺ |
| 23 | -CH₃ | ¹H-NMR (CDCl₃, 400MHz) δ: 4.05 (s, 3H), 4.49 (s, 3H). MS APCl+ m/z 261 [MH]⁺ |
| 24 | -(CH₂)₂O(CH₂)₂CH₃ | ¹H-NMR (DMSO-D₆, 400MHz) δ: 0.65 (t, 3H), 1.33 (m, 2H), 3.26 (t, 2H), 3.82 (t, 2H), 3.93 (s, 3H), 4.94 (t, 2H). MS APCl+ m/z 333, [MH]⁺ |

### Preparation 25

### Methyl 5-chloro-1-(2-ethoxyethyl)-7-(4-methylpyridin-2-ylamino)-1H-pyrazolo[4,3-d]pyrimidine-3-carboxylate

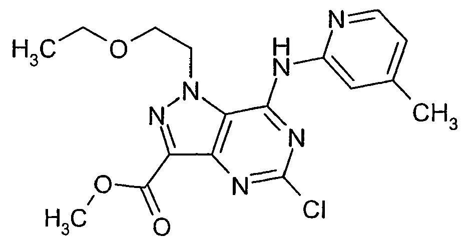

2-Amino-4-methylpyridine (1.34g, 12.4mmol) was added to a solution of the dichloro compound of preparation 21 (1.98, 6.2mmol) in dimethyl sulphoxide (10mL) and the reaction mixture stirred at 35°C for 5 hours. The reaction mixture was partitioned between dichloromethane (300mL) and water (500mL) and the organics washed with water (3x100mL), dried over magnesium sulphate and concentrated *in vacuo.* The crude product was purified by column chromatography on silica, eluting with dichloromethane:acetonitrile 98:2. Appropriate fractions were concentrated *in vacuo,* triturated with ether (50mL), filtered and the solid dried to yield the title product, 1.2g. ¹H-NMR (CDCl₃, 400MHz) δ: 1.06 (t, 3H), 2.49 (s, 3H), 3.62 (m, 2H), 4.00 (t, 2H), 4.06 (s, 3H), 5.05 (m, 2H), 6.98 (m, 1 H), 8.16 (m, 1 H), 8.50 (m, 1 H). MS APCI+ m/z 391 [MH]⁺

### Preparations 26-31

The following compounds were prepared by a method similar to that described for preparation 25 using the appropriate HNR¹R² amine and chloro compound.

| | | | | |
|---|---|---|---|---|
| 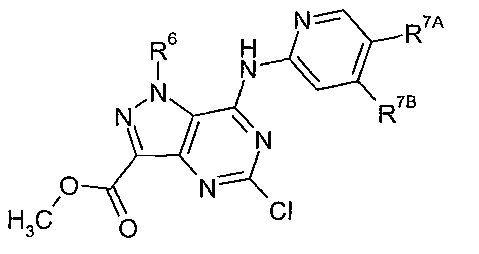 | | | | |

| No. | R⁶ | R^{7A} | R^{7B} | Data |
|---|---|---|---|---|
| 26 | -CH₂CH(CH₃)₂ | H | -CH₃ | ¹H-NMR (DMSO-D₆, 400MHz) δ: 0.84 (d, 6H), 2.22 (m, 1 H), 2.39 (s, 3H), 3.86 (s, 3H), 4.67 (m, 2H), 6.92 (m, 1 H), 7.60 (m, 1H), 8.08 (m, 1 H). MS APCl+ m/z 375 [MH]⁺ |
| 27 | -CH₃ | H | -CH₃ | ¹H-NMR (DMSO-D₆, 400MHz) δ: 2.40 (s, 3H), 3.84 (s, 3H), 4.40 (s, 3H), 6.95 (m, 1H), 7.68 (m, 1 H), 8.15 (m, 1 H). MS APCI+ m/z 333 [MH]⁺ |
| 28 | -(CH₂)₂O(CH₂)₂CH₃ | H | -CH₃ | ¹H-NMR (CDCl₃, 400MHz) δ: 0.80(t, 3H), 1.45(m, 2H), 2.55(s, 3H), 3.45(t, 2H), 3.85(t, 2H), 4.05(s, 3H), 4.86(t, 2H), 7.05(m, 1 H), 8.16(m, 1 H), 8.49(m, 1H). MS APCI+ m/z 405 [MH]⁺ |
| 29 | -(CH₂)₂OCH₂CH₃ | -CH₃ | H | ¹H-NMR (DMSO-D₆, 400MHz) δ : 1.01 (t, 3H), 2.26 (s, 3H), 3.52 (m, 2H), 3.88 (m, 5H), 4.96 (m, 2H), 7.76 (m, 1 H), 8.03 (m, 1 H), 8.20 (m, 1 H). MS APCI+ m/z 391 [MH]⁺ |
| 30 | -(CH₂)₂OCH₂CH₃ | H | H | ¹H-NMR (CDCl₃, 400MHz) δ: 1.15 (t, 3H), 3.63 (m, 2H), 4.01 (t, 2H), 4.08 (s, 3H), 4.97 (m, 2H), 7.17 (t, 2H), 7.86 (t, 1 H), 8.36 (m, 1H), 8.56 (m, 1 H). MS APCI+ m/z 377 [MH]⁺ |

### Preparation 31

### Methyl 5-chloro-7-(cyclopentylamino)-1-(2-ethoxyethyl)-1H-pyrazolo[4,3-d]pyrimidine-3-carboxylate

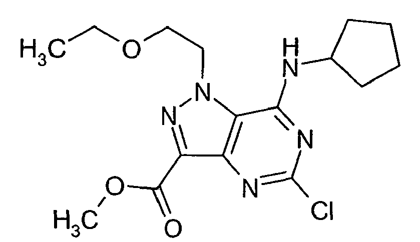

Cyclopentylamine (4.64mL, 47mmol) was added dropwise to an ice-cooled solution of the dichloro compound of preparation 21 (3.0g, 9.4mmol) in dimethylsulphoxide (8mL). Once addition was complete, the reaction was stirred for a further 10 minutes at room temperature. The reaction mixture was diluted with dichloromethane, and the mixture washed with water (x2). The solution was dried over magnesium sulphate and concentrated *in vacuo.* The residue was purified by column chromatography on silica gel using an elution gradient of ethyl acetate:pentane (25:75 to 50:50) to give the title compound as a white solid, 2.3g.
¹H-NMR (CDCl₃, 400MHz) δ: 1.17 (t, 3H), 1.50 (m, 2H), 1.73 (m, 4H), 2.21(m, 2H), 3.56(q, 2H), 3.93(t, 2H), 4.04(s, 3H), 4.50(m, 1 H), 4.70(t, 2H), 7.35(br, d, 1 H). MS ES+ m/z 382 [MH]⁺.

### Preparation 32

### Methyl 5-chloro-7-(cyclohexylamino)-1-(2-ethoxyethyl)-1H-pyrazolo[4,3-d]pyrimidine-3-carboxylate

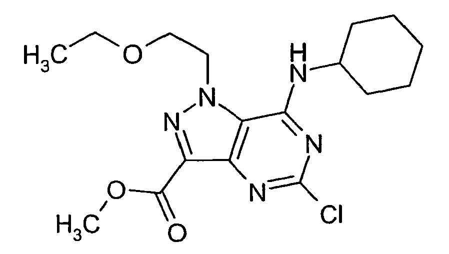

The dichloro compound of preparation 21 (2.50g, 7.84mmol) was dissolved in tetrahydrofuran (10mL) and the solution treated dropwise with a solution of cyclohexylamine (4.48mL, 39.20mmol) in tetrahydrofuran (10mL) whilst being cooled in an ice bath. The reaction mixture was stirred for 15 minutes at room temperature. The reaction mixture was diluted with water (50mL) and ethyl acetate (50mL) and stirred for 1 hour. The ethyl acetate layer was separated, washed with water, dried over magnesium sulphate and concentrated *in vacuo.* The residue was triturated with ether to yield 2.25g of the desired product.
¹H-NMR (CDCl₃, 400MHz) δ: 1.18 (t, 3H), 1.27 (m, 2H), 1.47 (m, 2H), 1.53-1.75 (m, 2H), 1.78 (m, 2H), 2.12 (m, 2H), 3.76 (m, 2H), 3.92 (t, 2H), 4.00 (s, 3H), 4.12 (m, 1 H), 4.70 (t, 2H), 7.20 (d, 1 H). MS ES+ m/z 382 [MH]⁺

### Preparation 33

### 5-Chloro-1-isobutyl-7-(4-methylpyridin-2-ylamino)-1H-pyrazolo[4,3-d]pyrimidine-3-carboxylic acid

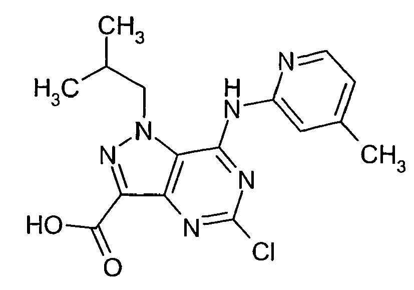

The ester of preparation 26 (1.00g, 2.67mmol) and 1 M aqueous sodium hydroxide solution (5.34mL, 5.34mmol) were dissolved in dioxane (25mL) and the reaction mixture stirred under nitrogen for 4 hours at room temperature. The reaction mixture was concentrated *in vacuo* and the residue dissolved in water (10mL) and acidified with 1 M citric acid solution. The precipitate formed was filtered off and dried in an oven at 55°C for 18 hours to yield the title product.
¹H-NMR (DMSO-D₆, 400MHz) δ: 0.83 (d, 6H), 2.21 (m, 1 H), 2.41 (s, 3H), 4.65 (m, 2H), 6.93 (m, 1 H), 7.60 (m, 1 H), 8.08 (m, 1 H). MS APCI+ m/z 361 [MH]⁺

### Preparations 34-38

The following compounds were prepared by a method similar to that described for preparation 33 using the appropriate ester.

| | | | | |
|---|---|---|---|---|
| 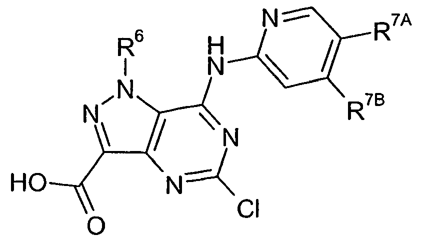 | | | | |

| No. | R⁶ | R^{7A} | R^{7B} | Data |
|---|---|---|---|---|
| 34 | -(CH₂)₂OCH₂CH₃ | H | -CH₃ | ¹H-NMR (DMSO-D₆, 400MHz) δ: 1.00 (t, 3H), 2.37 (s, 3H), 3.47 (m, 2H), 3.84 (m, 2H), 4.91 (m, 2H), 6.94 (m, 1 H), 7.82 (m, 1 H), 8.17 (m, 1 H). MS APCl+ m/z 377 [MH]⁺ |
| 35 | -CH₃ | H | -CH₃ | ¹H-NMR (DMSO-D₆, 400MHz) δ: 2.37 (s, 3H), 4.35 (s, 3H), 6.93 (m, 1H), 7.68 (m, 1 H), 8.12 (d, 1H). MS ES-m/z 317 [M-H]⁻ |
| 36 | -(CH₂)₂OCH₂CH₃ | -CH₃ | H | ¹H-NMR (DMSO-D₆, 400MHz) δ: 1.03 (t, 3H), 2.30 (s, 3H), 3.56 (m, 2H), 3.86 (m, 2H), 4.88 (m, 2H), 7.77 (m, 1 H), 8.08 (m, 1 H), 8.17 (m, 1 H). MS ES- m/z 375 [M-H]⁻ |
| 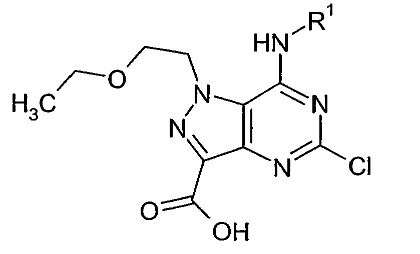 | | | | |
| No. | R¹ | Data | | |
| 37 | 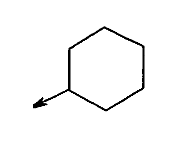 | ¹H-NMR (CDCl₃, 400MHz) δ: 1.00 (t, 3H), 1.18 (m, 1 H), 1.38 (m, 4H), 1.62 (m, 1H), 1.74 (m, 2H), 1.96 (m, 2H), 3.40 (t, 2H), 3.72 (m, 2H), 4.03 (m, 1 H), 4.73 (m, 2H), 7.26(d, 1H). MS ES- m/z 352 [M-H]⁻ | | |
| 38 | 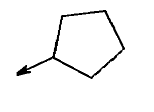 | ¹H-NMR (DMSO-D₆, 400MHz) δ: 0.99(t, 3H), 1.59(m, 4H), 1.72(m, 2H), 2.03(m, 2H), 3.40(q, 2H), 3.74(t, 2H), 4.41 (m, 1 H), 4.74(t, 2H), 7.35(d, 1 H) | | |

### Preparation 39

### 5-Chloro-1-(2-ethoxyethyl)-N-methyl-7-(4-methylpyridin-2-ylamino)-1H-pyrazolo[4,3-d]pyrimidine-3-carboxamide

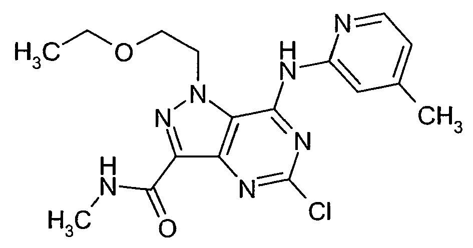

The carboxylic acid of preparation 34 (753mg, 2.0mmol) was added to a solution of 1-hydroxybenzotriazole hydrate (297mg, 2.20mmol), 1-(3-dimethylaminopropyl)-3-ethylcarbodiimide hydrochloride (421 mg, 2.2mmol) and N-ethyldiisopropylamine (383µL, 2.2mmol) in N,N-dimethylformamide (10mL) and the mixture stirred for 10 minutes at room temperature. An 8M solution of methylamine in ethanol (380µL, 3.0mmol) was added and the reaction mixture stirred at room temperature for 18 hours. The reaction mixture was concentrated *in vacuo* and the residue taken up in dichloromethane (100mL), washed with water (100mL), sodium hydrogencarbonate solution (50mL) and 1 M citric acid solution (50mL), dried over magnesium sulphate and concentrated *in vacuo.* The residue was purified by column chromatography on silica gel eluting with dichloromethane:methanol 100:0 to 97:3 to yield the title product, 450mg.
¹H-NMR (CDCl₃, 400MHz) δ: 1.18 (t, 3H), 2.39 (s, 3H), 3.00 (s, 3H), 3.62 (m, 2H), 3.96 (t 2H), 4.77 (t, 2H) 4.99 (m, 1 H), 6.83 (d, 1 H), 8.17 (d, 1 H), 8.24 (s, 1 H). MS APCI+ m/z 390 [MH]⁺

### Preparation 40

### 5-Chloro-1-isobutyl-N-methyl-7-(4-methylpyridin-2-ylamino)l-1H-pyrazolo[4,3-d]pyrimidine-3-carboxamide

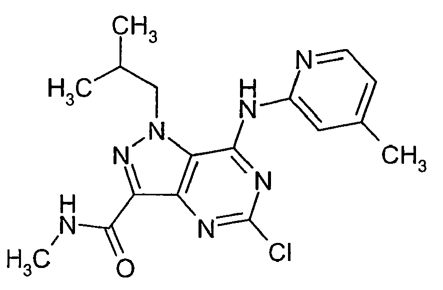

A solution of the carboxylic acid of preparation 33 (360mg, 1.0mmol) in N,N-dimethylformamide (5mL) was treated with 1-hydroxybenzotriazole hydrate (149mg, 1.10mmol), N-ethyldiisopropylamine (260µL, 1.5mmol) and 1-(3-dimethylaminopropyl)-3-ethylcarbodiimide hydrochloride (215mg, 1.10mmol) and the reaction mixture stirred for 20 minutes at room temperature. An 8M solution of methylamine in ethanol (38µL, 1.10mmol) was added and the reaction mixture stirred at room temperature for 48 hours. The reaction mixture was concentrated in *vacuo,* partitioned between ethyl acetate (50mL) and water (50mL) and the organics separated and washed with water (2x50mL). The organic layer was dried over magnesium sulphate and concentrated *in vacuo* and the residue was purified by column chromatography on silica gel eluting with dichloromethane:methanol 100:0 to 98:2 to yield the title product as a yellow solid, 360mg.
¹H-NMR (CDCl₃, 400MHz) δ: Rotamers 0.91 (2xd, 6H), 2.38+2.47 (2xs, 3H), 2.43 (m, 1 H), 2.97+3.11 (2xd, 3H), 4.71+4.76 (2xm, 2H), 7.42-7.65 (m, 3H). MS APCI+ m/z 374 [MH]⁺

### Preparation 41

### 5-Chloro-N-(2-(dimethylamino)ethyl)-1-isobutyl-7-(4-methylpyridin-2-ylamino)-1H-pyrazolo[4,3-d]pyrimidine-3-carboxamide

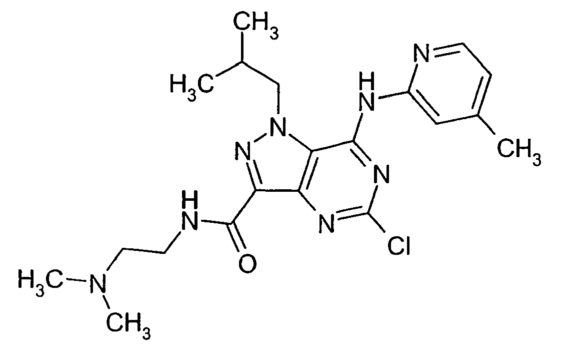

The title compound was prepared by a method similar to that described for preparation 40 using N,N-dimethylethylenediamine and the acid of preparation 33 to yield the product as a yellow solid in 88% yield.
¹H-NMR (CDCl₃, 400MHz) δ: 0.90 (d, 6H), 2.36-2.44 (m, 9H), 2.55 (m, 1 H), 2.66 (m, 2H), 3.71 (m, 2H), 4.71 (m, 2H), 6.65 (m, 1 H), 7.73 (m, 1 H), 8.35 (m, 1 H). MS APCI+ m/z 431 [MH]⁺

### Preparation 42

### 5-Chloro-1-(2-ethoxyethyl)-N-(2-hydroxyethyl)-7-(4-methylpyridin-2-ylamino)-1H-pyrazolo[4,3-d]pyrimidine-3-carboxamide

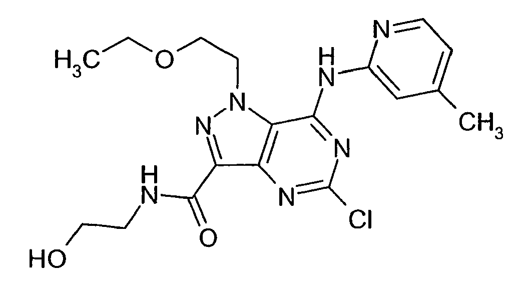

The title compound was prepared by a method similar to that described for preparation 40 using 2-aminoethanol and the acid of preparation 34.
¹H-NMR (CD₃OD, 400MHz) δ: 1.10 (m, 3H), 2.41 (s, 3H), 3.51-3.64 (m, 4H), 3.77 (m, 2H), 3.96 (m, 2H), 4.90 (m, 2H), 6.96 (m, 1 H), 7.56 (m, 1 H), 8.12 (m, 1H). MS APCI+ m/z 420 [MH]⁺

### Preparation 43

### 5-Chloro-1-(2-ethoxyethyl)-N-(2-methoxyethyl)-7-(4-methylpyridin-2-ylamino)-1H-pyrazolo[4,3-d]pyrimidine-3-carboxamide

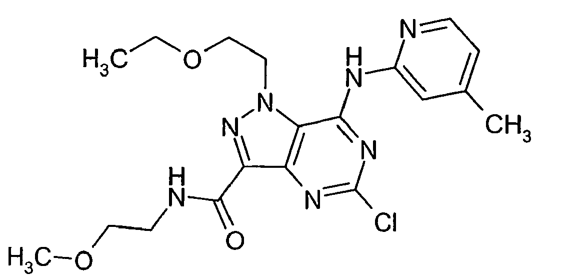

The title compound was prepared by a method similar to that described for preparation 40 using 2-methoxyethylamine and the acid of preparation 34.
¹H-NMR (CDCl₃, 400MHz) δ: 1.07 (t, 3H), 2.46 (s, 3H), 3.43 (s, 3H), 3.62 (m, 4H), 3.84 (m, 2H), 3.99 (m, 2H), 4.95 (m, 2H), 6.97 (m, 1 H), 7.23 (m, 1 H), 8.18 (m, 1 H),

### Preparation 44

### tert-Butyl (2-{[5-chloro-7-(cyclopentylamino)-1-(2-ethoxyethyl)-1H-pyrazolo[4,3 d]pyrimidine-3-carbonyl]amino}ethyl)carbamate

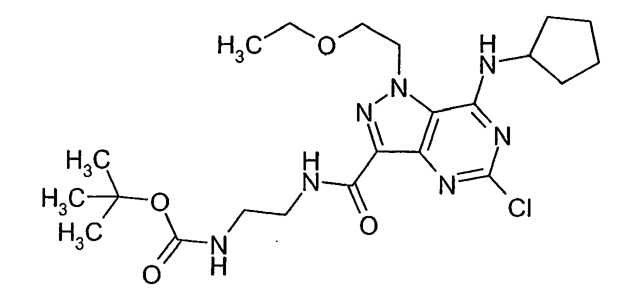

The acid of preparation 38 (353.8mg, 0.80mmol) was suspended in N,N-dimethylformamide (6mL) and the solution treated with N,N'-carbonyldiimidazole (208mg, 1.28mmol) and stirred at room temperature for 1 hour. The solution was treated with (2-amino-ethyl)-carbamic acid *tert*-butyl ester (160.2mg, 1.28mmol) and the reaction mixture stirred at room temperature for 5 hours. The reaction mixture was concentrated *in vacuo* and the residue dissolved in dichloromethane and washed with water (x2) and brine. The solution was dried over magnesium sulphate and concentrated *in vacuo.* The residue was purified by column chromatography on silica gel eluting with ethyl acetate to yield the title product as a colourless oil.
¹H-NMR (CD₃OD, 400MHz) δ : 1.12 (t, 3H), 1.39 (s, 9H), 1.62 (m, 2H), 1.72 (m, 2H), 1.82 (m, 2H), 2.19 (m, 2H), 3.31 (m, 2H), 3.55 (m, 4H), 3.89 (t, 2H), 4.50 (m, 1H), 4.76 (t, 2H)

### Preparations 45-49

The following compounds were prepared by a method similar to that described for preparation 44 using the appropriate acid and NHR¹⁵R¹⁶ amine.

| | | |
|---|---|---|
| 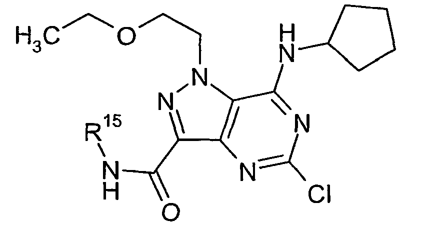 | | |

| No. | R¹⁵ | Data |
|---|---|---|
| 45 | 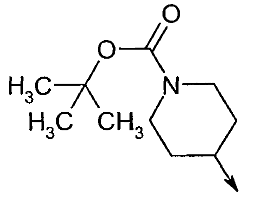 | ¹H-NMR (CD₃OD, 400MHz) δ: 1.12 (t, 3H), 1.48 (s, 9H), 1.52-1.73 (m, 6H), 1.82 (m, 2H), 2.01 (m, 2H), 2.18 (m, 2H), 3.11 (t, 2H), 3.54 (m, 2H), 3.89 (t, 2H), 3.98 (m, 2H), 4.17 (m, 1 H), 4.50 (m, 1 H), 4.76 (t, 2H) |
| 46 | -(CH₂)₂N(CH₃)₂ | ¹H-NMR (CD₃OD, 400MHz) δ: 1.12 (t, 3H), 1.63 (m, 2H), 1.71 (m, 2H), 1.82 (m, 2H), 2.19 (m, 2H), 2.33 (s, 6H), 2.62 (t, 2H), 3.54 (m, 2H), 3.62 (t, 2H), 3.89 (t, 2H), 4.50 (m, 1 H), 4.75 (t, 2H) |
| 47 | -(CH₂)₂OCH₃ | ¹H-NMR (CDCl₃, 400MHz) δ: 1.12 (t, 3H), 1.62 (m, 2H), 1.70 (m, 2H), 1.80 (m, 2H), 2.18 (m, 2H), 3.40 (s, 3H), 3.55 (m, 2H), 3.60 (m, 2H), 3.64 (t, 2H), 3.88 (m, 2H), 4.48 (m, 1 H), 4.75 (m, 2H), 7.98 (s, 1 H), 8.60 (m, 1 H). MS ES+ m/z 433 [MNa]⁺ |
| 48 | -CH₃ | ¹H-NMR (CDCl₃, 400MHz) δ: 1.12 (t, 3H), 1.63 (m, 2H), 1.71 (m, 2H), 1.82 (m, 2H), 2.20 (m, 2H), 3.00 (s, 3H), 3.53 (m, 2H), 3.88 (m, 2H), 3.64 (t, 2H), 4.50 (m, 1H), 4.73 (t, 2H), 7.48 (d, 1H). MS ES+ m/z 389 [MNa]⁺ |
| | | |
| No. | 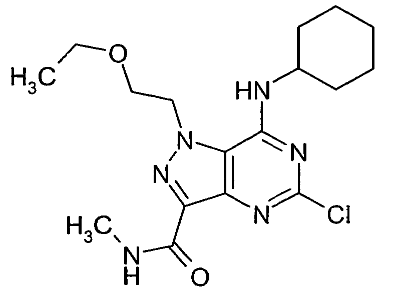 | Data |
| 49 | | ¹H-NMR (CDCl₃, 400MHz) δ: 1.17 (t, 3H), 1.20-1.35 (m, 3H), 1.41-1.55 (m, 2H), 1.65-1.84 (m, 3H), 2.11 (m, 2H), 3.10 (d, 3H), 3.56 (m, 2H), 3.92 (t, 2H), 4.16 (m, 1 H), 4.68 (t, 2H), 7.24 (m, 1 H), 8.14 (m, 1H). MS ES+ m/z 381 [MH]⁺ |

| | | |
|---|---|---|
| • Preparations 47 and 48 were not purified by column chromatography • Preparations 48 and 49 used an 8M solution of methylamine in ethanol to provide the HNR¹⁵R¹⁶ amine. | | |

### Preparation 50

### [5,7-Dichloro-1-(2-ethoxvethyl)-1H-pyrazolo[4,3-d]pyrimidin-3-yl]methanol

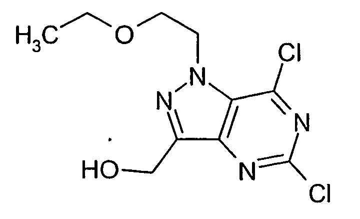

The dichloro compound of preparation 21 (2.4g, 7.52mmol) was dissolved in tetrahydrofuran (60mL) and the reaction mixture cooled to -78°C. DIBAL (37.6mL, 37.6mmol) in tetrahydrofuran (20mL) was added dropwise over 10 minutes and the reaction mixture stirred at -78°C for 10 minutes an then at -10°C for 1 hour. The reaction mixture was cooled to -78°C, quenched with ammonium chloride solution (25mL) and allowed to return to room temperature. The reaction mixture was diluted with dichloromethane (200mL) and water (100mL) and the solution filtered through ArBOCel®, washing through with dichloromethane (3x100mL). The organic phase was separated, dried over magnesium sulphate and concentrated *in vacuo.* The crude product was purified by column chromatography on silica gel eluting with dichloromethane:methanol 99:1 to yield the title product, 1.67g.
¹H-NMR (CDCl₃, 400MHz) δ: 1.08 (t, 3H), 3.42 (m, 2H), 3.80 (m, 2H), 4.90 (m, 2H), 5.10 (s, 2H). MS APCl+ m/z 291 [MH]⁺

### Preparation 51

### 3-(tert-Butyldimethylsilyloxymethyl)-5,7-dichloro-1-(2-ethoxyethyl)-1H-pyrazolo[4,3-d]pyrimidine

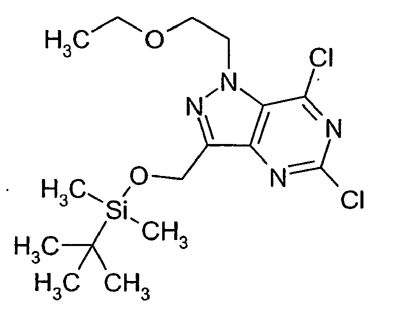

The alcohol of preparation 50 (1.32g, 4.53mmol) was dissolved in dichloromethane (25mL) and the solution treated with imidazole (339mg, 4.98mmol) and then *tert-*butyldimethylsilyl chloride (750mg, 4.98mmol). The reaction mixture was then stirred at room temperature for 18 hours. The reaction mixture was diluted with dichloromethane (200mL) and washed with 10% potassium carbonate solution (100mL). The organic phase was dried over sodium sulphate and concentrated in *vacuo.* The crude product was purified by column chromatography on silica gel eluting with dichloromethane:methanol 99:1 to yield the title product, 1.56g.
¹H-NMR (CDCl₃, 400MHz) δ: 0.00 (s, 6H), 0.78 (s, 9H), 0.93 (t, 3H), 3.29 (q, 2H), 3.71 (t, 2H), 4.72 (m, 2H), 4.94 (s, 2H). MS APCI+ m/z 405[MH]⁺

### Preparation 52

### N-[3-(tert-Butyldimethylsilyloxymethyl)-5-chloro-1-(2-ethoxyethyl)-1H-pyrazolo[4,3-d]pyrimidin-7-yl]pyrimidin-4-ylamine

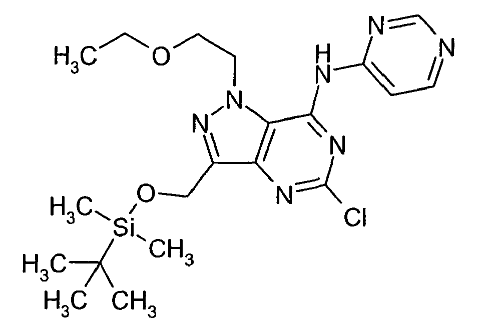

Pyrimidin-4-ylamine (1.10g, 11.55mmol) was dissolved in tetrahydrofuran (30mL) and the solution treated with sodium hexamethyldisilazide (2.12g, 11.55mmol) and stirred at room temperature for 20 minutes. The reaction mixture was treated with a solution of the dichloro compound of preparation 51 (1.56g, 3.85mmol) in tetrahydrofuran (10mL) and the reaction mixture stirred for 90 minutes at room temperature. The reaction mixture was quenched with ammonium chloride solution (100mL) and extracted with dichloromethane (200mL). The organic phase was separated, dried over magnesium sulphate and concentrated *in vacuo.* The crude product was purified by column chromatography on silica gel eluting with dichloromethane:methanol 97:3 to yield the title product, 830mg.
¹H-NMR (CDCl₃, 400MHz) δ: 0.00 (s, 6H), 0.77 (s, 9H), 1.08 (t, 3H), 3.54 (q, 2H), 3.80(m, 2H), 4.63 (m, 2H), 4.90 (s, 2H), 8.33 (d, 1 H), 8.51 (d, 1 H), 8.77 (s, 1 H). MS APCI+ m/z 464 [MH]⁺

### Preparation 53

### N-[3-(tert-Butyidimethylsilyloxymethyl)-5-chloro-1-(2-ethoxyethyl)-1H-pyrazolo[4,3-d]pyrimidin-7-yl]pyrazin-2-ylamine

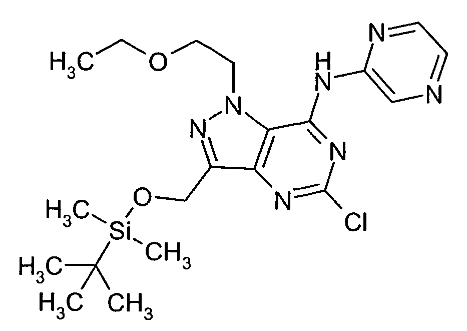

The title compound was prepared by a method similar to that described for preparation 52 using aminopyrazine.
¹H-NMR (CDCl₃, 400MHz) δ: 0.18 (s, 6H), 0.93 (s, 9H), 1.21 (t, 3H), 3.65 (m, 2H), 3.97 (m, 2H), 4.80 (m, 2H), 5.06 (m, 2H), 8.30 (m, 2H), 9.77 (m, 1 H), 10.17 (m, 1 H)

### Preparation 54

### [5-Chloro-1-(2-ethoxyethyl)-7-(pyrimidin-4-ylamino)-1H-pyrazolo[4 3-d]pyrimidin-3-yl]methanol

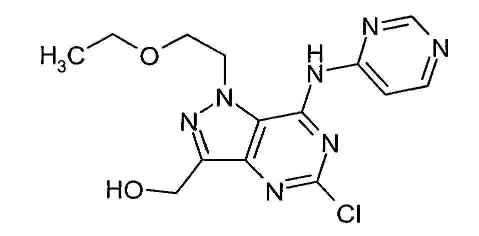

The protected alcohol of preparation 52 (2.0g, 1.76mmol) was dissolved in tetrahydrofuran (40mL) and the solution treated with a 1M solution of tetrabutylammonium fluoride in tetrahydrofuran (8.63mL). The reaction mixture was stirred for 90 minutes at room temperature and was then treated with additional tetrabutylammonium fluoride solution (4.32mL) and stirred for another hour. The reaction mixture was diluted with water (50mL) and the aqueous extracted with ethyl acetate (3x50mL). The combined organics were dried over magnesium sulphate and concentrated *in vacuo.* The crude product was purified by column chromatography on silica gel eluting with dichloromethane:methanol 99:1 to 95:5 to yield the title product, 1.25g.
¹H-NMR (CDCl₃, 400MHz) δ: 1.26 (t, 3H), 3.70 (q, 2H), 3.97 (m, 2H), 4.76 (m, 2H), 5.10 (s, 2H), 8.51 (d, 1H), 8.72 (d, 1H), 8.99(s, 1 H). MS APCl+ m/z 350 [MH]⁺

### Preparation 55

### [5-Chloro-1-(2-ethoxyethyl)-7-(pyrazin-2-ylamino)-1H-pyrazolo[4,3-d]pyrimidin-3-yl]methanol

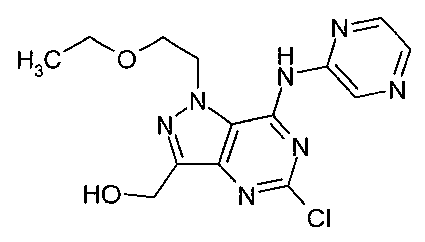

The title compound was prepared by a method similar to that described for preparation 54 using the protected alcohol of preparation 53.
¹H-NMR (CDCl₃, 400MHz) δ : 1.22 (t, 3H), 3.66 (m, 2H) 3.98 (m, 2H), 4.80 (m, 2H), 5.08 (s, 2H), 8.34 (m, 2H), 9.80 (m, 1 H), 10.22 (m, 1 H)

### Preparation 56

### 5-Chloro-1-(2-ethoxyethyl)-7-(pyrazin-2-ylamino)-1H-pyrazolo[4,3-d]pyrimidine-3-carbaldehyde

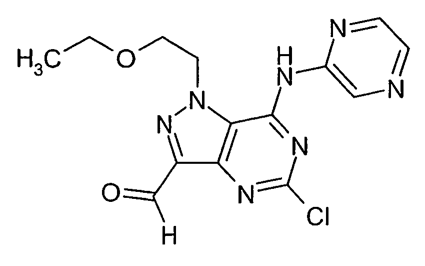

The alcohol of preparation 55 (251 mg, 0.72mmol) was dissolved in dichloromethane (12mL) and the solution cooled to 0°C in an ice bath. Dess Martin periodinane (456mg, 1.08mmol) was added and the reaction mixture stirred at room temperature for 2 hours. The reaction mixture was treated with a saturated solution of sodium thiosulphate in water (7.8mL) and then with saturated sodium hydrogencarbonate solution (7.8mL) and ether (7.8mL). The reaction mixture was stirred at room temperature for 15 minutes, the organic phase separated and the aqueous extracted with dichloromethane (x3). The organics were combined, dried over sodium sulphate and concentrated *in vacuo.* The crude product was purified by column chromatography on silica gel eluting with dichloromethane:methanol 99:1 to yield the title product, 200mg.
¹H-NMR (CDCl₃, 400MHz) δ: 1.22 (t, 3H), 3.69 (m, 2H), 4.06 (m, 2H), 4.92 (m, 2H), 7.22 (m, 1 H), 8.32 (m, 1 H), 8.40 (m, 1 H), 9.77 (m, 1H), 10.35 (m, 1 H)

### Preparation 57

### 5-Chloro-1-(2-ethoxyethyl)-7-(pyrimidin-4-ylamino)-1H-pyrazolo[4,3-d]pyrimidine-3-carbaldehyde

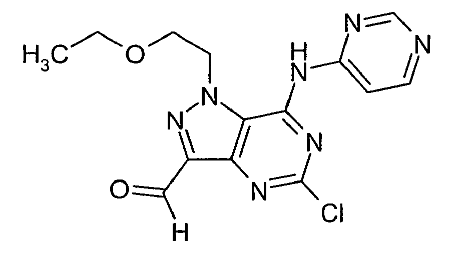

This compound was prepared by a method similar to that described for preparation 56 using the alcohol of preparation 54.
¹H-NMR (CDCl₃, 400MHz) δ: 1.23 (t, 3H), 3.72 (m, 2H), 4.06 (t, 2H), 4.93 (m, 2H), 8.36 (m, 1 H), 8.40 (m, 1 H), 9.77 (m, 1 H), 10.37 (m, 1 H)

### Preparation 58

### 5-Chloro-1-(2-ethoxyethyl)-7-(pyrimidin-4-ylamino)-1H-pyrazolo[4,3-d]pyrimidine-3-carboxylic acid

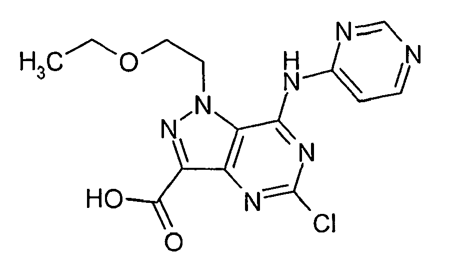

The aldehyde of preparation 57 (220mg, 0.63mmol) was dissolved in *tert*-butanol (40mL) and the solution treated with a 2M solution of 2-methyl-2-butene in tetrahydrofuran (44mL). The solution was stirred at room temperature and then treated dropwise with a solution of sodium chlorite (683mg, 7.59mmol) and sodium dihydrogen orthophosphate (699mg, 5.82mmol) in water (8mL) over 5 minutes. The reaction mixture was stirred at room temperature for 30 minutes. Water (40mL) and dichloromethane (40mL) were added to the reaction mixture and the phases separated. The aqueous layer was extracted with dichloromethane (2x40mL) and the aqueous was then acidified to pH 3 and extracted once more with dichloromethane (2x40mL). These organics were combined, dried over magnesium sulphate and concentrated *in vacuo.* The crude product was purified by column chromatography on silica gel eluting with first dichloromethane:methanol 97:3 and then dichloromethane:methanol:acetic acid 85:15:1 to yield the title product, 194mg. ¹H-NMR (CD₃OD, 400MHz) δ: 1.20 (t, 3H), 3.68 (m, 2H), 4.01 (t, 2H), 4.92 (t, 2H), 8.42 (m, 1 H), 8.68 (m, 1 H), 8.87 (m, 1 H). MS APCI+ m/z 364 [MH]⁺

### Preparation 59

### 5-Chloro-1-(2-ethoxyethyl)-7-(pyrazin-2-ylamino)-1H-pyrazolo[4,3-d]pyrimidine-3-carboxylic acid

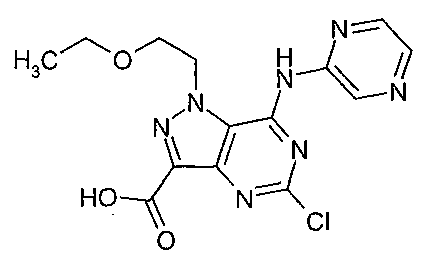

The title compound was prepared by a method similar to that described for preparation 58 using the aldehyde of preparation 56.
¹H-NMR (CD₃OD, 400MHz) δ: 1.20 (m, 3H), 3.65 (m, 2H), 3.99 (m, 2H), 4.96 (m, 2H), 8.36 (m, 1H), 8.42 (m, 1H), 9.60 (m, 1 H). MS APCI+ m/z 364 [MH]⁺

### Preparation 60

### tert-Butyl (3S)-3-(tert-butoxycarbonlamino)pyrrolidine-1-carboxylate

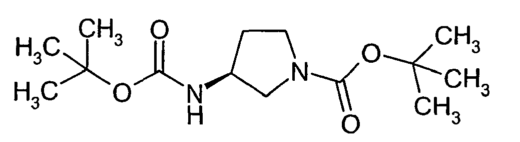

(3*S*)-3-(*tert*-Butoxycarbonylamino)pyrrolidine (1 g, 5.37mmol) and triethylamine (1.38mL, 10.00mmol) were dissolved in dichloromethane (15mL) and the solution stirred at room temperature for 10 minutes. The solution was then treated with di-*tert*-butyl dicarbonate (1.75g, 8.00mmol) and the reaction mixture stirred at room temperature for 18 hours. The reaction mixture was concentrated *in vacuo* and the residue purified by column chromatography on silica gel eluting with pentane:ethyl acetate 80:20 to yield the title product as a white solid, 1.25g.
¹H-NMR (CDCl₃, 400MHz) δ: 1.39 (s, 18H), 1.81 (m, 1 H), 2.15 (m, 1 H), 3.13 (m, 1 H), 3.40 (m, 2H), 3.58 (m, 1H), 4.17 (m, 1 H), 4.62 (m, 1 H). MS ES+ m/z 309 [MNa]⁺

### Preparation 61

### tert-Butyl (3R)-3-(tert-butoxycarbonylamino)pyrrolidine-1-carboxylate

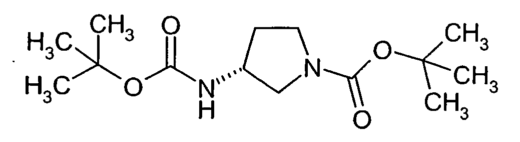

This compound was prepared by a method similar to that described for preparation 60 using (3*R*)-3-(*tert*-butoxycarbonylamino)pyrrolidine.
¹H-NMR (CDCl₃, 400MHz) δ: 1.37 (s, 18H), 1.79 (m, 1 H), 2.15 (m, 1 H), 3.13 (m, 1 H), 3.40 (m, 2H), 3.58 (m, 1 H), 4.16 (m, 1 H), 4.62 (m, 1 H). MS ES+ m/z 309 [MNa]⁺

### Preparation 62

### (3S)-N,1-Dimethyl-3-pyrrolidinylamine

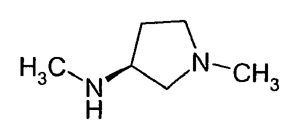

A solution of lithium aluminiumhydride (17mL, 16.89mmol) in tetrahydrofuran (10mL) was added dropwise to a stirring solution of the pyrrolidine of preparation 60 (600mg, 2.09mmol) in tetrahydrofuran (10mL) at 0°C. The reaction mixture was allowed to warm to room temperature and then heated to reflux for 5 hours. The reaction mixture was cooled to 0°C with an ice bath and then quenched by addition of sodium sulphate solution. The reaction mixture was diluted with ethyl acetate (100mL) and the aqueous washed with additional ethyl acetate. The combined organics were dried over magnesium sulphate and concentrated *in vacuo* to yield the title product, 60mg.
¹H-NMR (CD₃OD, 400MHz) δ: 2.25-2.46 (m, 4H), 2.75 (s, 3H), 3.02 (s, 3H), 3.73-4.08 (m, 4H), MS APCI+ m/z 115 [MH]⁺

### Preparation 63

### (3R)-N,1-Dimethyl-3-pyrrolidinylamine

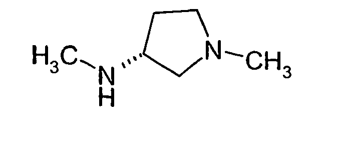

This compound was prepared by a method similar to that described for preparation 62 using the pyrrolidine of preparation 61.
¹H-NMR (CD₃OD, 400MHz) δ: 2.23-2.47 (m, 4H), 2.75 (s, 3H), 2.99 (s, 3H), 3.74-4.06 (m, 4H). MS APCI+ m/z 115 [MH]⁺

### Preparation 64

### tert-Butyl (3S)-(1-methyl-3-pyrrolidinyl)carbamate

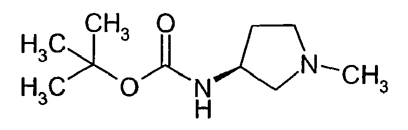

(3*S*)-3-(*tert*-Butoxycarbonylamino)pyrrolidine (2.0g, 10.75mmol) was dissolved in dichloromethane (100mL) and the solution treated with a 37% aqueous solution of formaldehyde (3.5mL, 43mmol). The solution was stirred for 30 minutes at room temperature and then treated with sodium triacetoxyborohydride (4.53g, 21.1 mmol) over 15 minutes. The reaction mixture was stirred at room temperature for 18 hours. The reaction mixture was diluted with sodium hydrogencarbonate solution (100mL) and the two phases separated. The organic layer was dried over magnesium sulphate and concentrated *in vacuo* to yield the title product.
¹H-NMR (CD₃OD, 400MHz) δ: 1.42 (s, 9H), 1.63 (m, 1 H), 2.22 (m, 1 H), 2.33 (s, 3H), 2.38 (m, 1 H), 2.51 (m, 1 H), 2.63 (m, 1 H), 2.80 (m, 1H), 4.08 (m, 1 H)

### Preparation 65

### (3S)-1-Methyl-3-pyrrolidinylamine hydrochloride

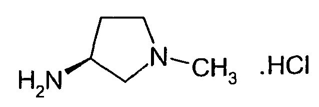

The product of preparation 64 (2.13g, 10.65mmol) was dissolved in a mixture of 30% trifluoroacetic acid (by volume) in dichloromethane (100mL) and the reaction mixture stirred at room temperature for 1 hour. The reaction mixture was concentrated in *vacuo* and the residue taken up in methanol (50mL) and treated with 2M hydrogen chloride in ether (10mL). The solution was concentrated *in vacuo,* redissolved in methanol (50mL) and treated with additional 2M hydrogen chloride in ether (10mL).

The solution was concentrated *in vacuo* and the residue triturated with ether to yield the title product.
¹H-NMR (CD₃OD, 400MHz) δ: 2.23 (m, 1 H), 2.63 (m, 1 H), 3.01 (s, 3H), 3.40-3.92 (m, 4H),4.18(m,1H)

### Example 1

### 1-(2-ethoxyethyl)-N-ethyl-5-(ethylamino)-7-(pyridin-2-ylamino)-1H-pyrazolo[4,3-d]pyrimidine-3-carboxamide

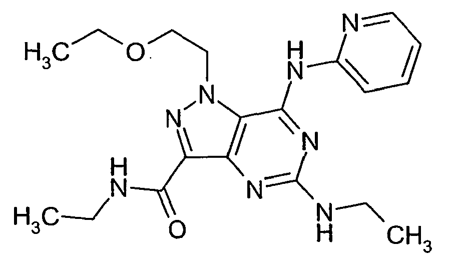

The ester of preparation 30 (80mg, 0.21 mmol) was dissolved in dimethyl sulphoxide (1 mL) and the solution treated with ethylamine (530µL, 1.05mmol) and N-ethyldiisopropylamine (180µL, 0.98mmol). The reaction mixture was heated to 120°C for 18 hours and then treated with additional ethylamine (29µL, 0.53mmol) before being heated for a further 2 hours at 120°C. The reaction mixture was taken up in dichloromethane (100mL) and washed with water (3x150mL). The organics were combined, dried over magnesium sulphate and concentrated *in vacuo.* The crude product was purified by column chromatography on silica gel eluting with dichloroimethane:methanol:ammonia 100:0:0 to 97:2:1 to yield the title product as a yellow oil, 20mg.
¹H-NMR (CD₃OD, 400MHz) δ: 1.07 (t, 3H), 1.27 (m, 6H), 3.41-3.60 (m, 6H), 3.90 (t, 2H), 4.81 (m, 2H), 7.06 (t, 1 H), 7.78 (t, 1 H), 8.28 (m, 1 H), 8.55 (d, 1 H). MS APCI+ m/z 399 [MH]⁺

### Example 2

### N-Methyl-5-(methylamino)-7-(4-methylpyridin-2-ylamino)-1-(2-propoxyethyl)-1H-pyrazolo[4,3-d]pyrimidine-3-carboxamide

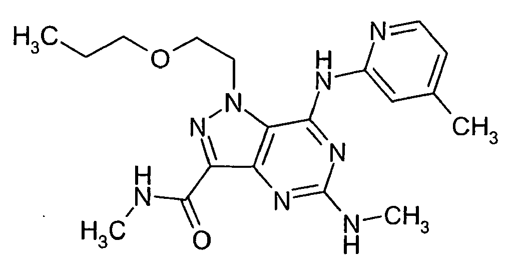

The title compound was prepared by a method similar to that described for example 1 using an 8M solution of methylamine in ethanol and the ester of preparation 28 ¹H-NMR (CD₃OD, 400MHz) δ: 0.77 (t, 3H), 1.46 (m, 2H), 2.54 (s, 3H), 2.93 (s, 3H), 3.10 (s, 3H), 3.41 (m, 2H), 3.93 (m, 2H), 5.03 (m, 2H), 7.13 (m, 1 H), 8.11 (m, 2H). MS ES+ m/z 399 [MH]⁺

### Example 3

### 1-(2-Ethoxyethyl)-N-methyl-5-(methylamino)-7-(4-methylpyridin-2-ylamino)-1H-pyrazolo[4,3-d]pyrimidine-3-carboxamide

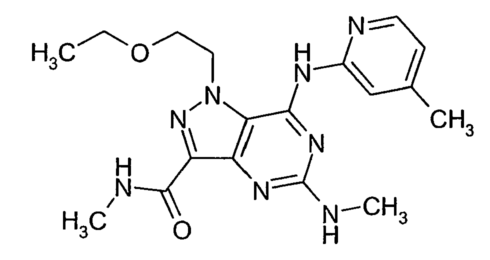

The title compound was prepared by a method similar to that described for example 1 using an 8M solution of methylamine in ethanol and the ester of preparation 25. ¹H-NMR (CD₃OD, 400MHz) δ: 1.18 (t, 3H), 2.40 (s, 3H), 3.00 (s, 3H), 3.05(s, 3H), 3.63 (m, 2H), 3.97 (m, 2H), 4.77 (m, 2H), 6.90 (d, 1 H), 8.20 (d, 1 H), 8.27 (m, 1H),. MS APCI+ m/z 385 [MH]⁺

### Example 4

### [1-(2-ethoxyethyl)-7-(4-methylpyridin-2-ylamino)-5-(pyrrolidin-1-yl)-3-(pyrrolidin-1-carbonyl)-1H-pyrazolo[4,3-d]pyrimidine

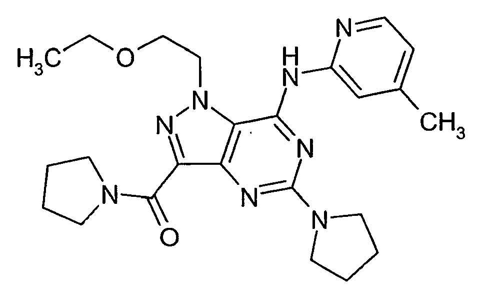

The ester of preparation 25 (100mg, 0.25mmol) was dissolved in a mixture of N-ethyldiisopropylamine (220µL, 1.25mmol), pyrrolidine (60µL, 0.75mmol) and dimethyl sulphoxide (1 mL) and the reaction mixture heated to 120°C for 18 hours. The reaction mixture was concentrated *in vacuo,* the residue was taken up in dichloromethane (100mL) and washed with water (3x100mL). The organics were dried over magnesium sulphate and concentrated *in vacuo.* The residue was purified by column chromatography on silica gel eluting with dichloromethane:methanol 100:0 to 90:10 to yield the title product.
¹H-NMR (CD₃OD, 400MHz) δ: 1.10 (t, 3H), 1.90-2.08 (m, 8H), 2.40 (s, 3H), 3.62 (m, 8H), 3.80 (t, 2H), 3.98 (t, 2H), 4.80 (m, 2H), 6.98 (d, 1 H), 8.18 (d, 1 H), 8.57 (s, 1 H). MS APCI+ m/z 465 [MH]⁺

### Examples 5 to 49

The compounds of Examples 5 to 49 were prepared by the following general method:

The appropriate carboxylic acid of preparations 34, 35, 36, 58, and 59 (1 eq) was dissolved in 1-methyl-2-pyrrolidinone (6mLmmol⁻¹) and the solution treated with N,N'-carbonyldiimidazole (1.1eq) and N-ethyldiisopropylamine (1.1eq). The mixture was stirred at room temperature for 30 minutes and then the appropriate HNR¹⁵R¹⁶ amine (1.1-1.2 eq) added and the reaction mixture stirred at room temperature for 1 hour. The appropriate HNR³R⁴ amine (2.5-3eq) was then added and the reaction mixture stirred at 120°C for 5 hours. The reaction mixture was concentrated in vacuo and the residue purified by HPLC on a Phenomenex Luna^{®} C₁₈ 5µm column, eluting with 0.1 % diethylamine in water:acetonitrile 95:5 to 5:95, or, in the case of examples 14, 20, 21, 22, 23, 27, 28, 33, 36, 37, 42, 44, 46 and 48, by column chromatography on silica gel using an elution gradient of dichloromethane:methanol (100:0 to 95:5) to yield the title product.

| | |
|---|---|
| 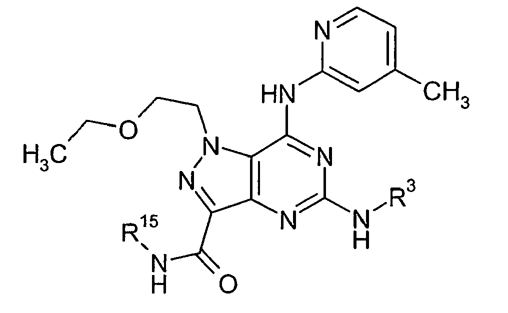 | |

| Ex | |
|---|---|
| 5* | R³ = -CH₃; R¹⁵ = -(CH₂)₂NHCH₃ |
| | ¹H-NMR (CD₃OD, 400MHz) δ: 1.10 (t, 3H), 2.39 (s, 3H), 2.48 (s, 3H), 2.92 (t, 2H), 3.00 (s, 3H), 3.58 (m, 2H), 3.68 (t, 2H), 3.94 (t, 2H), 4.79 (t, 2H), 6.93 (d, 1 H), 8.15 (d, 1 H), 8.42 (m, 1 H). MS ES+ m/z 428 [MH]⁺ |
| 6 | R³ = -CH₃; R¹⁵ = -(CH₂)₂N(CH₃)₂ |
| | ¹H-NMR (CD₃OD, 400MHz) δ: 1.06 (t, 3H), 2.31 (s, 6H), 2.42 (s, 3H), 2.64 (t, 2H), 3.02 (s, 3H), 3.58 (m, 2H), 3.62 (t, 2H), 3.93 (t, 2H), 4.78 (m, 2H), 6.94 (d, 1 H), 8.13 (m, 1 H), 8.43 (s, 1 H). MS ES+ m/z 442 [MH]⁺ |
| 7* | R³ = -CH₃; R¹⁵= 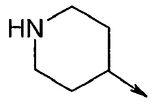 |
| | ¹H-NMR (CD₃OD, 400MHz) δ: 1.09 (t, 3H), 1.67 (m, 2H), 2.16 (m, 2H), 2.41 (s, 3H), 2.88 (m, 2H), 3.00 (s, 3H), 3.20 (m, 2H), 3.58 (m, 2H), 3.92 (t, 2H), 4.13 (m, 1 H), 4.79 (t, 2H), 6.93 (d, 1 H), 8.12 (d, 1 H), 8.42 (m, 1 H). MS ES+ m/z 454.3 [MH]⁺ |
| 8 | R³ = -CH₂CH₃; R¹⁵ = -CH₂CH₃ |
| | ¹H-NMR (CD₃OD, 400MHz) δ: 1.08 (t, 3H), 1.30 (m, 6H), 2.42 (s, 3H), 3.42-3.62 (m, 6H), 3.94 (t, 2H), 4.79 (t, 2H), 6.94 (d, 1 H), 8.14 (d, 1 H), 8.44 (s, 1 H). MS ES+ m/z 413 [MH]⁺ |
| 9* | R³ = -CH₂CH₃; R¹⁵ = -(CH₂)₂NH₂ |
| | ¹H-NMR (CD₃OD, 400MHz) δ: 1.11 (t, 3H), 1.32 (t, 3H), 2.43 (s, 3H), 2.93 (t, 2H), 3.49 (m, 2H), 3.56 (m, 4H), 3.95 (t, 2H), 4.79 (t, 2H), 6.93 (d, 1 H), 8.14 (d, 1 H), 8.42 (m, 1 H). MS ES+ m/z 428 [MH]⁺ |
| 10* | R³ = -CH₂CH₃; R¹⁵ = -(CH₂)₂NHCH₃ |
| | ¹H-NMR (CD₃OD, 400MHz) δ: 1.09 (t, 3H), 1.28 (t, 3H), 2.41 (s, 3H), 2.46 (s, 3H), 2.90 (t, 2H), 3.50 (m, 2H), 3.59 (m, 2H), 3.64 (t, 2H), 3.94 (t, 2H), 4.79 (t, 2H), 6.93 (d, 1H), 8.14 (d, 1 H), 8.42 (m, 1 H). MS ES+ m/z 442 [MH]⁺ |
| 11 | R³ = -CH₂CH₃; R¹⁵ = -(CH₂)₂N(CH₃)₂ |
| | ¹H-NMR (CD₃OD, 400MHz) δ: 1.07 (t, 3H), 1.26 (t, 3H), 2.30 (s, 6H), 2.41 (s, 3H), 2.62 (t, 2H), 3.42-3.70 (m, 6H), 3.92 (t, 2H), 4.78 (m, 2H), 6.93 (d, 1H), 8.14 (m, 1H), 8.42 (m, 1 H). MS APCI+ m/z 456 [MH]⁺ |
| 12* | R³= -CH₂CH₃; R¹⁵= 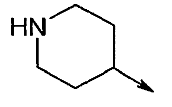 |
| | ¹H-NMR (CD₃OD, 400MHz) δ: 1.12 (t, 3H), 1.28 (t, 3H), 1.64 (m, 2H), 2.14 (m, 2H), 2.43 (s, 3H), 2.88 (m, 2H), 3.20 (m, 2H), 3.47 (m, 2H), 3.60 (m, 2H), 3.95 (t, 2H), 4.15 (m, 1 H), 4.79 (t, 2H), 6.94 (br, 1 H), 8.13 (m, 1 H), 8.43 (m, 1 H). MS ES+ m/z 468 [MH]⁺ |
| *13 | R³ = -CH(CH₃)₂; R¹⁵ = -(CH₂)₂NH₂ |
| | ¹H-NMR (CD₃OD, 400MHz) δ: 1.08 (t, 3H), 1.29 (d, 6H), 2.41 (s, 3H), 2.94 (t, 2H), 3.58 (m, 4H), 3.94 (t, 2H), 4.18 (m, 1 H), 4.79 (t, 2H), 6.93 (d, 1 H), 8.14 (d, 1 H), 8.42 (m, 1 H). MS ES+ m/z 442 [MH]⁺ |

| | |
|---|---|
| 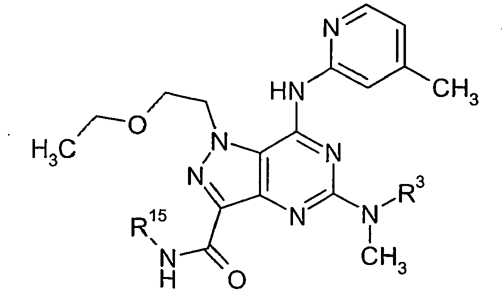 | |

| Ex | |
|---|---|
| 14 | R³ = -CH₃; R¹⁵ = -CH₃ |
| | ¹H-NMR (CD₃OD, 400MHz) δ: 1.10 (t, 3H), 2.41 (s, 3H), 3.04 (s, 3H), 3.24 (s, 6H), 3.60 (m, 2H), 3.92 (t, 2H), 4.79 (t, 2H), 6.95 (d,1 H), 8.18 (d, 1 H), 8.37 (s, 1 H). MS ES+ m/z 399 [MH]⁺ |
| 15^{*} | R³ = -CH₃; R¹⁵ = -(CH₂)₂NH₂ |
| | ¹H-NMR (CD₃OD, 400MHz) δ: 1.10 (t, 3H), 2.40 (s, 3H), 2.94 (t, 2H), 3.28 (s, 6H), 3.60 (m, 4H), 3.92 (t, 2H), 4.79 (t, 2H), 6.94 (d, 1 H), 8.16 (d, 1 H), 8.37 (m, 1 H). MS ES+ m/z 428 [MH]⁺ |
| 16* | R³ = -CH₃; R¹⁵ = -(CH₂)₂NHCH₃ |
| | ¹H-NMR (CD₃OD, 400MHz) δ: 1.08 (t, 3H), 2.39 (s, 3H), 2.45 (s, 3H), 2.88 (t, 2H), 3.26 (s, 6H), 3.59 (m, 2H), 3.64 (t, 2H), 3.93 (t, 2H), 4.79 (t, 2H), 6.94 (d, 1 H), 8.18 (d, 1 H), 8.36 (m, 1 H). MS ES+ m/z 442 [MH]⁺ |
| 17 | R³ = -CH₃; R¹⁵ = -(CH₂)₂N(CH₃)₂ |
| | ¹H-NMR (CDCl₃, 400MHz) δ: 1.05 (t, 3H), 2.32 (m, 6H), 2.35 (s, 3H), 2.64 (m, 2H), 3.24 (s, 6H), 3.60 (m, 2H), 3.72 (m,2H), 3.96 (t, 2H), 4.78 (m, 2H), 6.92 (d, 1 H), 8.18 (d, 1 H), 8.34 (s, 1 H), 9.12 (m, 1 H), 9.78 (s, 1 H). MS APCI+ m/z 456 [MH]⁺ |
| 18* | R³=-CH₃; R¹⁵= 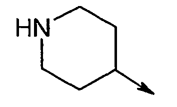 |
| | ¹H-NMR (CD₃OD, 400MHz) δ: 1.10 (t, 3H), 1.60 (m, 2H), 2.12 (m, 2H), 2.40 (s, 3H), 2.82 (m, 2H), 3.15 (m, 2H), 3.28 (s, 6H), 3.60 (m, 2H), 3.93 (t, 2H), 4.10(m, 1 H), 4.79 (t, 2H), 6.96 (d, 1 H), 8.17 (d, 1H), 8.37 (m, 1H). MS ES+ m/z 468 [MH]⁺ |
| 19 | R³ = -CH₃; R¹⁵ = -(CH₂)₂OCH₃ |
| | ¹H-NMR (CD₃OD, 400MHz) δ: 1.11 (t, 3H), 2.40 (s, 3H), 3.28 (s, 6H), 3.42 (s, 3H), 3.60 (m, 4H), 3.67 (t, 2H), 3.93 (t, 2H), 4.79 (t, 2H), 6.93 (d, 1 H), 8.17 (d, 1 H), 8.37 (s, 1 H). MS ES+ m/z 443 [MH]⁺ |
| 20* | R³ = -CH₃; R¹⁵ = -CH₂CO₂H |
| | ¹H-NMR (CD₃OD, 400MHz) δ : 1.10 (t, 3H), 2.52 (s, 3H), 3.30 (s, 6H), 3.57 (q, 2H), 3.99 (t, 2H), 4.17 (s, 3H), 5.05 (t, 2H), 7.18 (d, 1H), 8.02 (s, 1H), 8.18 (d, 1 H). MS APCI+ m/z 443 [MH]⁺ |
| 21* | R³=-CH₃; R¹⁵= 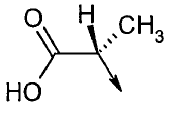 |
| | ¹H-NMR (CD₃OD, 400MHz) δ: 1.10 (t, 3H), 1.57 (d, 3H), 2.51 (s, 3H), 3.30 (s, 6H), 3.48 (q, 2H), 3.99 (t, 2H), 4.63 (q, 1 H), 5.02 (t, 2H), 7.18 (d, 1H), 8.02 (s, 1 H), 8.17 (d, 1 H). MS APCI+ m/z 457 [MH]⁺ |
| 22* | R³=-CH₃; R¹⁵ = 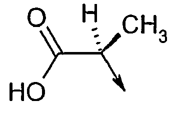 |
| | ¹H-NMR (CD₃OD, 400MHz) δ: 1.10 (t, 3H), 1.57 (d, 3H), 2.51 (s, 3H), 3.30 (s, 6H), 3.57 (q, 2H), 3.99 (t, 2H), 4.62 (q, 1 H), 5.02 (t, 2H), 7.18 (d, 1H), 8.01 (s, 1 H), 8.18 (d, 1 H). MS APCl+ m/z 457 [MH]⁺ |
| 23* | R³ = -CH₃; R¹⁵ =-(CH₂)₂CO₂H |
| | ¹H-NMR (CD₃OD, 400MHz) δ: 1.10 (t, 3H), 2.50 (s, 3H), 2.65 (t, 2H), 3.30 (s, 6H), 3.57 (q, 2H), 3.65 (t, 2H), 3.98 (t, 2H), 4.99 (t, 2H), 7.18 (d, 1 H), 8.01 (s, 1 H), 8.18 (d, 1 H). MS APCI+ m/z 457 [MH]⁺ |
| 24* | R³ = -CH₂CH₃; R¹⁵ = -(CH₂)₂NH₂ |
| | ¹H-NMR (CD₃OD, 400MHz) δ: 1.10 (t, 3H), 1.24 (t, 3H), 2.40 (s, 3H), 2.94 (t, 2H), 3.23 (s, 3H), 3.60 (m, 4H), 3.77 (m, 2H), 3.96 (t, 2H), 4.79 (t, 2H), 6.97 (d, 1 H), 8.17 (d, 1 H), 8.34 (m, 1 H). MS ES+ m/z 442 [MH]⁺ |
| 25* | R³ = -CH(CH₃)₂; R¹⁵ =-(CH₂)₂NH₂ |
| | ¹H-NMR (CD₃OD, 400MHz) δ: 1.12 (t, 3H), 1.27 (d, 6H), 2.40 (s, 3H), 2.95 (t, 2H), 3.07 (s, 3H), 3.60 (m, 4H), 3.93 (t, 2H), 4.79 (t, 2H), 5.09 (m, 1 H), 6.95 (d, 1 H), 8.16 (d, 1 H), 8.30 (m, 1 H). MS ES+ m/z 456 [MH]⁺ |
| 26 | R³= 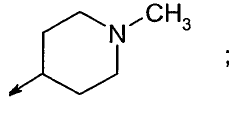 R¹⁵ = -CH₃ |
| | ¹H-NMR (CD₃OD, 400MHz) δ: 1.10 (t, 3H), 1.79 (m, 2H), 1.94 (m, 2H), 2.20 (m, 2H), 2.34 (s, 3H), 2.44 (s, 3H), 3.05 (m, 2H), 3.07 (s, 3H), 3.12 (s, 3H), 3.62 (q, 2H), 3.93 (t, 2H), 4.68 (m, 1 H), 4.80 (t, 2H), 6.97 (d, 1 H), 8.18 (m, 2H). MS ES+ m/z 482 [MH]⁺ |

| 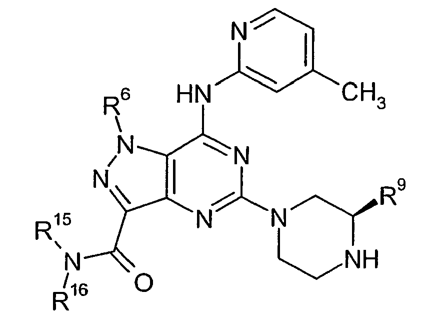 | |
|---|---|
| Ex | |
| 27* | R⁶ =-CH₃; R⁹ = H; R¹⁵ = -CH₃, R¹⁶ = H |
| | ¹H-NMR (CD₃OD, 400MHz) δ: 2.41 (s, 3H), 3.02 (m, 4H), 3.04 (s, 3H), 3.87 (m, 4H), 4.36 (s, 3H), 6.99 (m, 1H), 7.96 (m, 1H), 8.18 (m, 1H). MS APCI+ m/z 382 [MH]⁺ |
| 28 | R⁶ = -CH₃; R⁹ = -CH₃; R¹⁵ = -CH₃, R¹⁶ = H |
| | ¹H-NMR (CD₃OD, 400MHz) δ: 1.18 (d, 3H), 2.43 (s, 3H), 2.70 (m, 2H), 2.87 (m, 2H), 3.04 (s, 3H), 3.08 (m, 1 H), 4.37 (s, 3H), 4.55 (m, 2H), 7.01 (m, 1 H), 7.67 (m, 1 H), 8.19 (m, 1 H). MS APCI+ m/z 396 [MH]⁺ |
| 29^{*} | R⁶ = -(CH₂)₂OCH₂CH₃; R⁹ = H; R¹⁵ = -CH₃, R¹⁶ = H |
| | ¹H-NMR (CD₃OD, 400MHz) δ: 1.12 (t, 3H), 2.40 (s, 3H), 2.96 (t, 4H), 3.06 (s, 3H), 3.62 (q, 2H), 3.84 (t, 4H), 3.93 (t, 2H), 4.80 (t, 2H), 6.96 (d, 1H), 8.05 (m, 2H). MS ES+ m/z 440 [MH]⁺ |
| 30 | R⁶ = -(CH₂)₂OCH₂CH₃; R⁹ = -CH₃; R¹⁵ = -CH₃, R¹⁶ = H |
| | ¹H-NMR (CD₃OD, 400MHz) δ: 1.15 (t, 3H), 1.19 (d, 3H), 2.40 (s, 3H), 2.70 (m, 1 H), 2.76 (m, 2H), 3.06 (s, 3H), 3.08 (m, 3H),3.62 (m, 2H), 3.94 (t, 2H), 4.60 (m, 2H), 4.80 (t, 2H), 6.97 (d, 1 H), 8.18 (m, 2H). MS ES+ m/z 454 [MH]⁺ |
| 31 | R⁶ = -(CH₂)₂OCH₂CH₃; R⁹ = -CH₃; R¹⁵ = -CH₃, R¹⁶ -CH₃ |
| | ¹H-NMR (CD₃OD, 400MHz) δ: 1.14 (t, 3H), 1.18 (d, 3H), 2.42 (s, 3H), 2.64 (m, 1 H), 2.83 (m, 2H), 2.94-3.04 (m, 2H), 3.16 (s, 3H), 3.21 (s, 3H), 3.62 (q, 2H), 3.93 (t, 2H), 4.60 (m, 2H), 4.78 (t, 2H), 6.95 (d, 1H), 8.18 (d, 1 H), 8.20 (s, 1 H). MS ES+ m/z 468 [MH]⁺ |
| 32 | R⁶ = -(CH₂)₂OCH₂CH₃; R⁹ = -CH₃; R¹⁵ = -CH₂CH₃, R¹⁶ = H |
| | ¹H-NMR (DMSO-D₆, 400MHz) δ: 1.02 (m, 6H), 1.18 (t, 3H), 2.34 (s, 3H), 2.53 (t, 2H), 2.70 (m, 2H), 2.84-2.93 (m, 2H), 3.38 (m, 2H), 3.52 (m, 2H), 3.82 (t, 2H), 4.58 (m, 2H), 4.72 (t, 2H), 6.94 (d, 1 H), 8.00 (s, 1H), 8.19 (d, 1 H), 8.53 (m,1 H). MS ES+ m/z 468 [MH]⁺ |

| | |
|---|---|
| 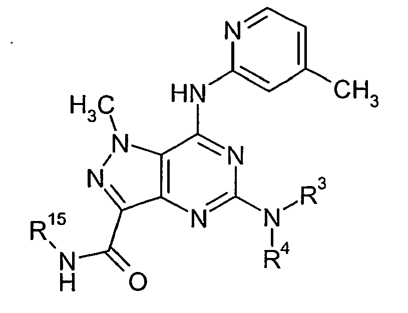 | |

| Ex | |
|---|---|
| 33 | R³ = -CH₃; R⁴ = H; R¹⁵ = -CH₃ |
| | ¹H-NMR (CD₃OD, 400MHz) δ: 2.40 (s, 3H), 2.98 (s, 3H), 3.01 (s, 3H), 4.36 (s, 3H), 6.90 (d, 1H), 7.72 (s, 1H), 8.09 (d, 1H). MS APCI+ m/z 327 [MH]⁺ |
| 34* | R³ = -CH₂CH₃; R⁴ = H; R¹⁵ = 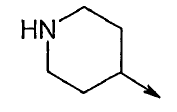 |
| | ¹H-NMR (CD₃OD, 400MHz) δ: 1.31 (t, 3H), 1.59 (m, 2H), 2.07 (m, 2H), 2.40 (s, 3H), 2.80 (m, 2H), 3.11 (m, 2H), 4.08 (m, 1 H), 4.37 (s, 3H), 6.90 (m, 2H), 8.10 (m, 1 H). MS ES+ m/z 410 [MH]⁺ |
| 35* | R³ = -CH(CH₃)₂; R⁴ = H; R¹⁵ = 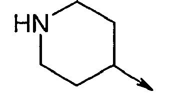 |
| | ¹H-NMR (CD₃OD, 400MHz) δ: 1.28 (d, 6H), 1.59 (m, 2H), 2.11 (m, 2H), 2.41 (s, 3H), 2.80 (m, 2H), 3.30 (m, 1 H), 4.09 (m, 1 H), 4.37 (s, 3H), 6.97 (m, 1 H), 8.17 (m, 2H). MS ES+ m/z 424 [MH]⁺ |
| 36 | R³ = -CH₃; R⁴ = -CH₃; R^{15 =} -CH₃ |
| | ¹H-NMR (DMSO-D₆, 400MHz δ: 2.33 (s, 3H), 2.89 (d, 3H), 3.16 (s, 6H), 4.28 (s, 3H), 6.94 (m, 1 H), 8.19 (d, 1 H), 8.43 (m, 1 H). MS APCI+ m/z 341 [MH]⁺ |
| 37 | R³ = -CH₃; R⁴ = -CH₃; R¹⁵ = -(CH₂)₂N(CH₃)₂ |
| | ¹H-NMR (CD₃OD, 400MHz) δ: 2.35 (s, 3H), 2.42 (t, 2H), 2.69 (m, 6H), 3.18 (s, 6H), 3.75 (t, 2H), 4.32 (s, 3H), 6.93 (m, 2H), 8.09 (m, 1 H). MS APCI+ m/z 398 [MH]⁺ |
| 38 | R³ = -CH₃; R⁴ = -CH₃; R¹⁵ = 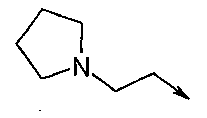 |
| | ¹H-NMR (CD₃OD, 400MHz) δ: 1.83 (m, 4H), 2.39 (s, 3H), 2.67 (m, 4H), 2.80 (t, 2H), 3.21 (s, 6H), 3.64 (t, 2H), 4.34 (s, 3H), 6.98 (m, 1 H), 8.15 (m, 2H). MS ES+ m/z 424 [MH]⁺ |
| 39* | R³= -CH₃; R⁴ = -CH₃; R¹⁵ = 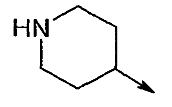 |
| | ¹H-NMR (CD₃OD, 400MHz) δ: 1.57 (m, 2H), 2.10 (m, 2H), 2.40 (s, 3H), 2.82 (m, 2H), 3.13 (m, 2H), 3.23 (s, 6H), 4.08 (m, 1H), 4.35 (s, 3H), 6.96 (m, 1 H), 8.15 (m, 2H). MS ES+ m/z 410 [MH]⁺ |
| 40* | R³ = -CH(CH₃)₂; R⁴ = -CH₃; R¹⁵ = -(CH₂)₂NH₂ |
| | ¹H-NMR (CD₃OD, 400MHz) δ: 1.28 (d, 6H), 2.41 (s, 3H), 2.99 (t, 2H), 3.07 (s, 3H), 3.30 (m, 1 H), 3.63 (t, 2H), 4.37 (s, 3H), 6.97 (m, 1 H), 8.17 (m, 1 H), 8.54 (d, 1 H). MS ES+ m/z 398 [MH]⁺ |
| 41* | R³ = -CH(CH₃)₂; R⁴ = -CH₃; R¹⁵ = 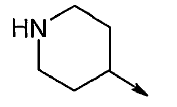 |
| | ¹H-NMR (CD₃OD, 400MHz) δ: 1.33 (d, 6H), 1.60 (m, 2H), 2.11 (m, 2H), 2.40 (m, 3H), 2.82 (m, 2H), 3.14 (m, 2H), 4.09 (m, 1 H), 4.38 (s, 3H), 6.89 (m, 1 H), 8.10 (m, 1H). MS ES+ m/z 438 [MH]⁺ |
| 42 | R³ = 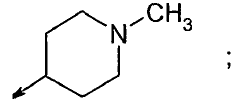 R⁴=-CH_{3;} R¹⁵ = CH₃ |
| | ¹H-NMR (CD₃OD, 400MHz) δ: 1.86 (m, 2H), 1.97 (m, 2H), 2.38 (m, 4H), 2.45 (s, 3H), 3.09 (s, 3H), 3.12 (s, 3H), 3.17 (m, 1 H), 4.36 (s, 3H), 6.57 (s, 1H), 7.00 (m, 1 H), 8.19 (m, 1 H). MS APCI+ m/z 424 [MH]⁺ |

| | |
|---|---|
| 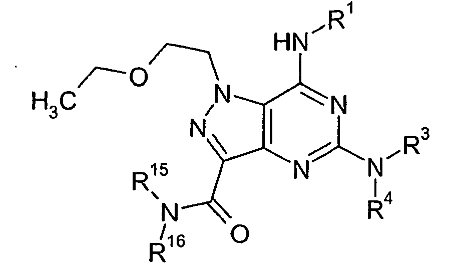 | |

| Ex | |
|---|---|
| 43* | R¹ = 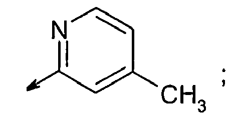 R³= -CH₃; R⁴= H; -NR¹⁵R¹⁶= 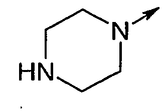 |
| | ¹H-NMR (CD₃OD, 400MHz) δ: 1.10 (t, 3H), 2.42 (s, 3H), 2.93 (m, 4H), 2.98 (s, 3H), 3.60 (m, 2H), 3.64 (m, 2H), 3.78 (m, 2H), 3.92 (t, 2H), 4.78 (m, 2H), 6.97 (m, 1H), 8.15 (m, 1 H), 8.42 (s, 1 H). MS ES+ m/z 440 [MH]⁺ |
| 44 | R¹ = 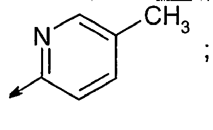 R³ = -CH₃; R⁴ = -CH₃; R¹⁵ = -(CH₂)₂OCH₃; R¹⁶ = H |
| | ¹H-NMR (CD₃OD, 400MHz) δ: 1.10 (t, 3H), 1.30 (t, 3H), 2.34 (s, 3H), 3.42 (s, 3H), 3.50 (q, 2H), 3.60 (m, 4H) 3.69 (m, 2H), 3.96 (m, 2H), 4.99 (m, 2H), 7.64 (m, 1 H), 8.16 (m, 1 H), 8.49 (m, 1 H). MS APCl+ m/z 443 [MH]⁺ |
| 45 | R¹ = 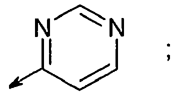 R³ = -CH₃; R⁴ = -CH₃; R¹⁵ = -(CH₂)₂OCH₃; R¹⁶ = H |
| | ¹H-NMR (CD₃OD, 400MHz) δ: 1.21 (t, 3H), 3.34 (s, 3H), 3.34 (s, 6H), 3.69 (m, 6H), 3.96 (t, 2H), 4.81 (t, 2H), 8.39 (d, 1 H), 8.61 (d, 1 H), 8,83 (s, 1H). MS ES+ m/z 430 [MH]⁺ |
| 46 | R¹ = 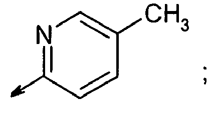 R³ = -CH₂CH₃, R⁴ = H; R¹⁵ = -CH₂CH₃; R¹⁶ = H |
| | ¹H-NMR (CD₃OD, 400MHz) δ: 1.12 (t, 3H), 1.30 (m, 6H), 2.36 (s, 3H), 3.48 (q, 2H), 3.56 (m, 2H), 3.60 (m, 2H) 3.69 (q, 2H), 3.97 (m, 2H), 4.78 (m, 2H), 7.68 (m, 1 H), 8.17 (m, 1 H), 8.50 (m, 1 H). MS APCI+ m/z 413 [MH]⁺ |
| 47 | R¹ = 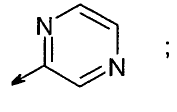 R³ = -CH₂CH₃; R⁴ = H; R¹⁵ = -CH₂CH₃; R¹⁶ = H |
| | ¹H-NMR (CD₃OD, 400MHz) δ: 1.20 (t, 3H), 1.31 (d, 6H), 3.53 (m, 4H), 3.65 (q, 2H), 3.96 (t, 2H) 4.80 (t, 2H), 8.27 (d, 1 H), 8.37 (d, 1 H), 9.71 (s, 1 H). MS APCI+ m/z 400 [MH]⁺ |
| 48 | R¹ = 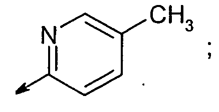 R³ = -CH₂CH₃; R⁴ = H; R¹⁵ = -(CH₂)₂OCH₃; R¹⁶ = H |
| | ¹H-NMR (CD₃OD, 400MHz) δ: 1.12 (t, 3H), 1.30 (t, 3H), 2.32 (s, 3H), 3.42 (s, 3H), 3.49 (q, 2H), 3.60 (m, 4H) 3.69 (q, 2H), 3.96 (m, 2H), 4.79 (m, 2H), 7.62 (m, 1 H), 8.16 (m, 1 H), 8.44 (m, 1 H). MS APCI+ m/z 443 [MH]⁺ |
| 49* | R¹ = 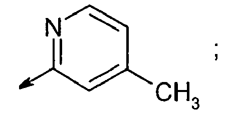 R³ = -CH₂CH₃; R⁴ = -CH₂CH₃; R¹⁵ = -(CH₂)₂NH₂; R¹⁶ = H |
| | ¹H-NMR (CD₃OD, 400MHz) δ: 1.12 (t, 3H), 1.30 (t, 6H), 2.42 (s, 3H), 2.94 (t, 2H), 3.60 (m, 4H), 3.73 (m, 4H), 3.95 (t, 2H), 4.79 (t, 2H), 6.96 (d, 1 H), 8.16 (d, 1 H), 8.38 (m, 1 H). MS ES+ m/z 456 [MH]⁺ |

| | |
|---|---|
| Notes on Examples 5-49 • Examples 5, 6, 7, 14, 26, 27, 28, 29, 30, 33, 36, 42 and 43 used an 8M solution of methylamine in ethanol to provide the HNR¹⁵R¹⁶ and / or HNR³R⁴ amine. • Examples 27 and 29 used *tert*-butyl piperazine-1-carboxylate as the HNR³R⁴ amine. • Examples 18 and 43 used *tert*-butyl piperazine-1-carboxylate as the HNR¹⁵R¹⁶amine. • Examples 7, 12, 34, 35, 39 and 41 used *tert*-butyl 4-aminopiperidine-1-carboxylate as the HNR¹⁵R¹⁶amine. • Examples 9, 13, 15, 24, 25, 40 and 49 used *tert*-butyl (2-aminoethyl)-carbamate as the HNR¹⁵R¹⁶amine. • Examples 5, 10 and 16 used *tert*-butyl *N*-(2-aminoethyl)-*N*-methylcarbamate as the HNR¹⁵R¹⁶amine. • Example 20 used glycine *tert*-butyl ester as the HNR¹⁵R¹⁶ amine. • Example 21 used L-alanine *tert*-butyl ester (Chem. Pharm. Bull., 1978, 26 (3), 803-808) as the HNR¹⁵R¹⁶amine. • Example 22 used D-alanine *tert-*butyl ester (Chem. Pharm. Bull., 1978, 26 (3), 803-808) as the HNR¹⁵R¹⁶ amine. • Example 23 used β-alanine *tert*-butyl ester (ChemBioChem, 2001,2, 171-179, compound 9) as the HNR¹⁵R¹⁶amine. *Prior to column chromatography, these examples were treated with a solution of trifluoroacetic acid in dichloromethane (0.5mL), stirred at room temperature for 6 hours and concentrated *in vacuo.* The residue was then purified by column chromatography on silica gel as described above. | |

### Example 50

### 1-(2-Ethoxyethyl)-5-(ethylamino)-N-methyl-7-(4-methylpyridin-2-ylamino)-1H-pyrazolo[4,3-d]pyrimidine-3-carboxamide

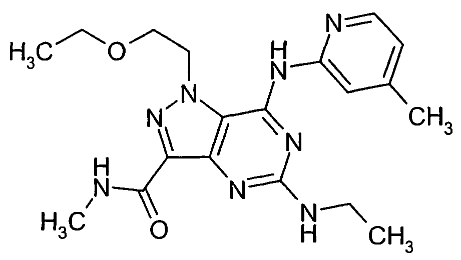

The carboxylic acid of preparation 34 (50mg, 0.13mmol) was dissolved in 1-methyl-2-pyrrolidinone (1 mL) and the solution treated with N,N'-carbonyldiimidazole (24mg, 0.15mmol) and N-ethyldiisopropylamine (25µL, 0.15mmol). The solution was then stirred at room temperature for 45 minutes before being treated with an 8M solution of methylamine in ethanol (19µL) and stirred at room temperature for 1 hour. Ethylamine (20.25mg, 0.45mmol) and additional N-ethyldiisopropylamine (25µL, 0.15mmol) was added and the reaction mixture heated to 120°C for 18 hours. The reaction mixture was concentrated *in vacuo* and the residue purified by HPLC on a Phenomenex Luna® C₁₈ 5µM column eluting with 0.1% diethylamine in water:acetonitrile 90:10 to 5:95 to yield the title product.
¹H-NMR (CD₃OD, 400MHz) δ: 1.08 (t, 3H), 1.29 (t, 3H), 2.43 (s, 3H), 3.02 (s, 3H), 3.49 (m, 2H), 3.60 (m, 2H), 3.94 (t, 2H), 4.79 (t, 2H), 6.95 (d, 1H), 8.16 (d, 1 H), 8.43 (s, 1H). MS ES+ m/z 399 [MH]⁺

### Example 51

### 7-(Cyclopentylamino)-5-(dimethylamino)-1-(2-ethoxyethyl)-N-(2-(methylamino)ethyl)-1H-pyrazolo[4,3-d]pyrimidine-3-carboxamide

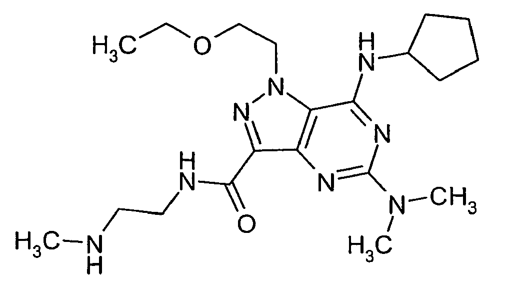

A suspension of the carboxylic acid of preparation 38 (70mg, 0.2mmol) in dimethyl sulphoxide (2mL) was treated with N,N'-carbonyldiimidazole (35.6mg, 0.22mmol) and N-ethyldiisopropylamine (38µL, 0.22mmol) and sealed in a ReactiVial™ for 1 hour. After this time *tert*-butyl *N*-(2-aminoethyl)-*N*-methylcarbamate (58µl, 0.32mmol) and further N,N'-carbonyldiimidazole (18mg, 0.11 mmol) was added and stirring continued for 1 hour. The reaction mixture was dissolved in dichloromethane (30mL) and washed with water (2 x 20mL) then saturated brine (20mL). The organic layer was dried over magnesium sulphate and evapourated to give a colourless oil. The resulting oil was dissolved in dimethyl sulphoxide (2mL) and treated with N-ethyldiisopropylamine (104µL, 0.6mmol) and dimethylamine solution 5.6 M in ethanol (107.2µL, 0.6mmol) before being sealed in a ReactiVial™ and heated to 120°C for 16hours. The reaction mixture was again diluted with dichloromethane (30mL) and washed with water (2 x 20mL) and brine (20mL). The organic solution was dried over magnesium sulphate and concentrated *in vacuo* to give a colourless oil. The resulting oil was re-dissolved in dichloromethane (1 mL) and treated with trifluoroacetic acid (1 mL) before stirring at room temperature for 2hours. The reaction was concentrated *in vacuo* and the resulting oil was purified by column chromatography on silica gel eluting with dichloromethane:methanol:0.88ammonia 90:10:1 to give a white solid.
¹H-NMR (CDCl₃, 400MHz) δ: 1.10 (t, 3H), 1.51 (m, 2H), 1.64 (m, 2H), 1.75 (m, 2H), 2.16 (m, 2H), 2.59 (s, 3H), 3.07 (m, 2H), 3.21 (s, 6H), 3.47 (q, 2H), 3.75 (t, 2H), 3.85 (m, 2H), 4.36 (1, 2H), 4.62 (m, 2H) 6.74 (m, 1 H), 9.03 (m, 1 H). MS APCI+ m/z 419 [MH]⁺

### Example 52

### 7-(Cyclopentylamino)-1-(2-ethoxyethyl)-5-(ethylamino)-N-(2-(methylamino)ethyl)-1H-pyrazolo[4,3-d]pyrimidine-3-carboxamide

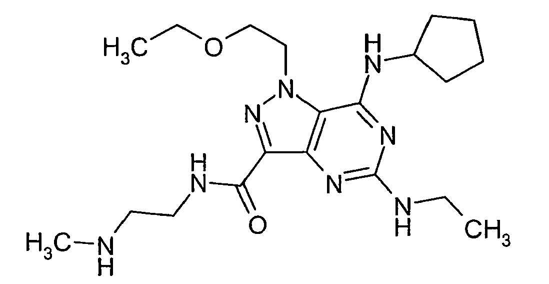

The title compound was prepared by a method similar to that described for example 51 using *tert*-butyl N-(2-aminoethyl)-*N*-methylcarbamate and ethylamine.
¹H-NMR (CDCl₃, 400MHz) δ: 1.13 (t, 3H), 1.24 (t, 3H), 1.60 (m, 2H), 1.68 (m, 2H), 1.80 (m, 2H), 2.15 (m, 2H), 2.46 (s, 3H), 2.87 (t, 2H), 3.42 (q, 2H), 3.53 (q, 2H), 3.63 (t, 2H), 3.86 (t, 2H), 4.43 (m, 1 H), 4.62 (t, 2H). MS APCI+ m/z 419 [MH]⁺

### Example 53

### 7-(Cyclopentylamino)-1-(2-ethoxyethyl)-5-(N-ethyl-N-methylamino)-N-(2-(methylamino)ethyl)-1 H-pyrazolo[4,3-d]pyrimidine-3-carboxamide

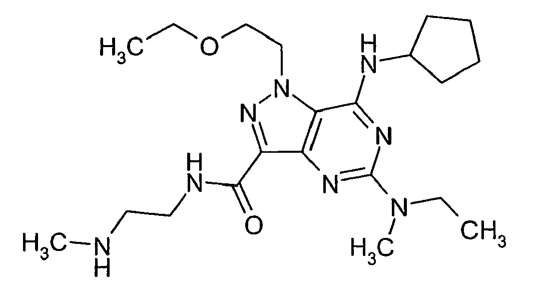

The title compound was prepared by a method similar to that described for example 51 using *tert*-butyl N-(2-aminoethyl)-*N*-methylcarbamate and N-ethyl-methylamine.
¹H-NMR (CD₃OD, 400MHz) δ: 1.13 (t, 3H), 1.19 (t, 3H), 1.60-1.71 (m, 4H), 1.80 (m, 2H), 2.17 (m, 2H), 2.43 (s, 3H), 2.85 (t, 2H) 3.17 (s, 3H), 3.53 (m, 4H), 3.63 (t, 2H), 3.72 (m, 2H), 4.42 (m, 1 H), 4.63 (t, 2H). MS APCI+ m/z 433 [MH]⁺

### Example 54

### 7-(Cyclopentylamino)-1-(2-ethoxyethyl)-N-ethyl-5-(piperazin-1-yl)-1H-pyrazolo[4,3-d]pyrimidine-3-carboxamide

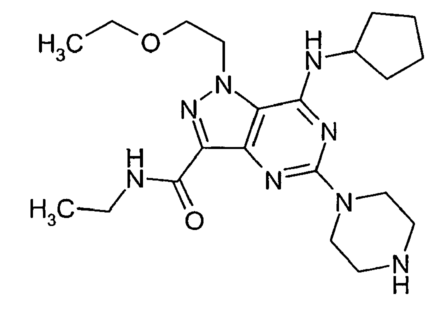

The title compound was prepared by a method similar to that described for example 51 using ethylamine and *tert-*butyl piperazine-1-carboxylate.
¹H-NMR (CD₃OD, 400MHz) δ : 1.11 (t, 3H), 1.27 (t, 3H), 1.66 (m, 4H), 1.80 (m, 2H), 2.18 (m, 2H), 2.93 (m, 4H) 3.53 (m, 2H), 3.81 (m, 4H) 3.85 (m, 2H), 4.41 (m, 1 H), 4.63 (m, 2H). MS ES+ m/z 431 [MH]⁺

### Example 55

### 1-(2-Ethoxyethyl)-N-isopropyl-5-(N-isopropyl-N-methylamino)-N-methyl-7-(4-methylpyridin-2-ylamino)-1H-pyrazolo[4,3-d]pyrimidine-3-carboxamide

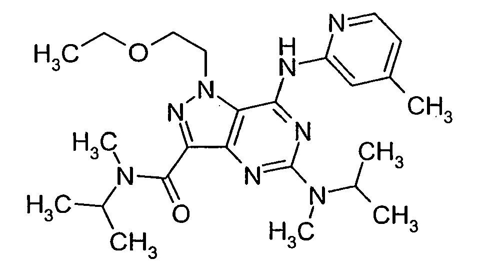

The carboxylic acid of preparation 34 (440mg, 1.17mmol) and isopropylmethylamine (1.21 mL, 11.67mmol) were dissolved in dimethyl sulphoxide (50mL) and the reaction mixture heated to 120°C for 12 hours. The reaction mixture was concentrated in *vacuo* and the residue taken up in sodium carbonate solution (1mL) and concentrated *in vacuo.* The residue was treated with ammonium chloride solution (2mL) and concentrated *in vacuo.* The residue was triturated with acetone (5x30mL) and concentrated *in vacuo.* The residue was purified by column chromatography on silica gel eluting with water:methanol 90:10 to 0:100. The crude product was washed with dichloromethane (2x100mL), dried over magnesium sulphate and concentrated *in vacuo* to yield the title product.
¹H-NMR (CD₃OD, 400MHz) δ: 1.10 (m, 3H), 1.30 (m, 12H), 2.40 (s, 3H), 3.03 (m, 5H), 3.30 (s, 3H), 3.61 (m, 2H), 3.90 (t, 2H), 4.78 (m, 2H), 6.97 (d, 1 H), 8.18 (d, 1 H), 8.35 (s, 1 H). MS APCI+ m/z 469 [MH]⁺

### Examples 56-64

The compounds of Examples 56 to 64 were prepared by the following general method:

The appropriate amide of preparations 39, 40, 41, 42 and 43 (70mg, 0.18mmol) was dissolved in dimethylsulphoxide (1.4mL) and treated with N-ethyldiisopropylamine (0.16mL, 0.9mmol) and the appropriate HNR³R⁴ amine (0.92mmol). The reaction mixture was heated to 100°C for 18 hours before being allowed to cool and being washed with water (3x25mL) and dichloromethane (50mL). The organic layer was separated, dried with magnesium sulphate and concentrated *in vacuo.* The residue was purified by column chromatography on silica gel eluting with dichloromethane:methanol 100:0 to 95:5 to yield the desired product.

| | |
|---|---|
| 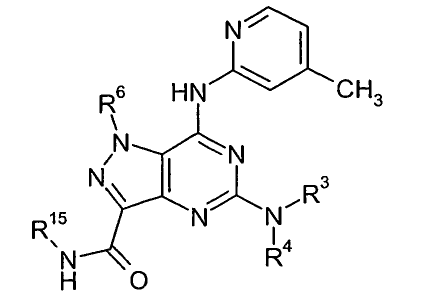 | |

| Ex | |
|---|---|
| 56 | R³ = -CH₃; R⁴ = H; R⁶ = -(CH₂)₂OCH₂CH₃; R¹⁵ = -(CH₂)₂OCH₃ |
| | ¹H-NMR (CD₃OD, 400MHz) δ: 1.10 (t, 3H,), 2.42 (s, 3H), 3.00 (s, 3H), 3.40 (s, 3H), 3.63 (m, 4H), 3.68 (m, 2H), 3.90 (t, 2H), 4.80 (t, 2H), 6.95 (d, 1 H), 8.15 (d, 1 H), 8.45 (s, 1 H). MS APCI+ m/z 429 [MH]⁺ |
| 57 | R³ = -CH₃; R⁴ = -CH₃; R⁶ = -CH(CH₃)₂; R¹⁵ = -(CH₂)₂N(CH₃)₂ |
| | ¹H-NMR (CD₃OD, 400MHz) δ : 0.93 (d, 6H), 2.31 (s, 6H), 2.38 (s, 3H), 2.62 (m, 2H), 3.25 (m, 7H), 3.62 (m, 2H), 4.47 (m, 2H), 6.86 (m, 1 H), 6.94 (m, 1 H), 8.07 (m, 1 H). MS APCI+ m/z 440 [MH]⁺ |
| 58 | R³ = -CH₃; R⁴ = -CH₃; R⁶ = -(CH₂)₂OCH₂CH₃; R¹⁵ = -(CH₂)₂OH |
| | ¹H-NMR (CD₃OD, 400MHz) δ: 1.08 (t, 3H), 2.40 (s, 3H), 3.30 (s, 6H), 3.62 (m, 4H), 3.81 (m, 2H), 3.92 (m, 2H), 4.84 (m, 2H), 6.96 (m, 1 H), 8.18 (m, 1 h), 8.40 (m, 1 H). MS APCI+ m/z 429 [MH]⁺ |
| 59 | R³ = -CH₂CH₃; R⁴ = H; R⁶ = -CH(CH₃)₂; R¹⁵ = -(CH₂)₂N(CH₃)₂ |
| | ¹H-NMR (CD₃OD, 400MHz) δ: 0.93 (d, 6H), 1.29 (t, 3H), 2.36 (s, 6H), 2.40 (s, 3H), 2.64 (m, 3H), 3.48 (m, 2H), 3.62 (m, 2H), 4.58 (m, 2H), 6.84 (m, 1 H), 6.95 (m, 1 H), 8.08 (m, 1 H). MS APCI+ m/z 440 [MH]⁺ |
| 60 | R³ = -CH₂CH₃; R⁴ = H; R⁶ = -(CH₂)₂OCH₂CH₃; R¹⁵ = -(CH₂)₂OCH₃ |
| | ¹H-NMR (CD₃OD, 400MHz) δ: 1.10 (t, 3H), 1.30 (t, 3H), 2.42 (s, 3H), 3.40 (s, 3H), 3.50 (m, 2H), 3.60 (m, 4H), 3.68 (q, 2H), 4.64 (t, 2H), 4.80 (t, 2H), 6.95 (d, 1 H), 8.15 (d, 1 H), 8.45 (s, 1 H). MS APCI+ m/z 443 [MH]⁺ |
| 61 | R³ = -(CH₂)₂OH; R⁴ = -CH₃; R⁶ = -(CH₂)₂OCH₂CH₃; R¹⁵ = -CH₃ |
| | ¹H-NMR (CD₃OD, 400MHz) δ: 1.11 (t, 3H), 2.40 (s, 3H), 3.03 (s, 3H), 3.37 (m, 3H), 3.61 (m, 2H), 3.85 (m, 4H), 3.93 (m, 2H), 4.80 (m, 2H), 6.94 (m, 1 H), 8.16 (m, 1 H), 8.32 (m, 1 H). MS APCI+ m/z 429 [MH]⁺ |
| 62 | R³ = -(CH₂)₂OCH₃; R⁴ = H; R⁶ = -(CH₂)₂OCH₂CH₃; R¹⁵ = -CH₃ |
| | ¹H-NMR (CD₃OD, 400MHz) δ: 1.10 (3H, t), 2.42 (3H, s), 3.00 (3H, s), 3.40 (3H, s), 3.60 (2H, q), 3.65 (4H, m), 3.95 (2H, t), 4.80 (2H, t), 6.95 (1 H, d), 8.15 (1 H, d), 8.40 (1 H, s). MS APCI+ m/z 429 [MH]⁺ |
| 63 | R³ = -(CH₂)₂OCH₃; R⁴= -CH₃; R⁶ = -(CH₂)₂OCH₂CH₃; R¹⁵ = -CH₃ |
| | ¹H-NMR (CD₃OD, 400MHz) δ: 1.12 (t, 3H), 2.40 (s, 3H), 3.04 (s, 3H), 3.28(s, 3H), 3.36 (s, 3H), 3.61 (m, 2H), 3.67 (m, 2H), 3.91 (m, 4H), 4.60 (m, 2H), 6.93 (m, 1 H), 8.15 (m, 1 H), 8.28 (m, 1 H). MS APCI+ m/z 443 [MH]⁺ |
| 64 | -NR³R⁴ = 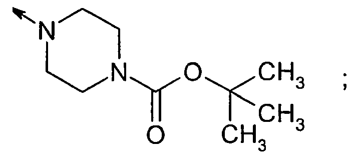 R⁶ = -CH(CH₃)₂; R¹⁵ = -CH₃ |
| | ¹H-NMR (CDCl₃, 400MHz) δ: 1.00(m, 6H), 1.51 (s, 9H), 2.41 (m, 4H), 3.11 (d, 3H), 3.57-3.87(m, 8H), 4.40-4.70(m, 2H), 6.91 (m, 1 H), 8.17(m, 1 H), 8.36(m, 1 H). MS APCI+ m/z 524 [MH]⁺ |

### Example 56 - an 8M solution of methylamine in ethanol was used to provide the HNR³R⁴ amine

### Example 65

### 7-(Cyclopentylamino)-5-(dimethylamino)-N-(2-(dimethylamino)ethyl)-1-(2-ethoxyethyl)-1H-pyrazolo[4,3-d]pyrimidine-3-carboxamide

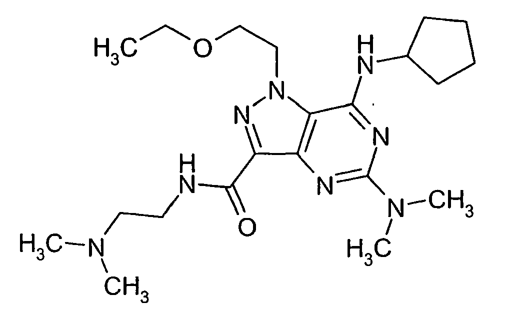

A solution of the amide of preparation 46 (55mg, 0.13mmol) and dimethylamine (5.6M solution in ethanol) (93µl, 0.52mmol) in dimethyl sulphoxide (2mL) was sealed in a ReactiVial™ and heated to 120°C overnight. The reaction mixture was diluted with dichloromethane (20mL) and washed with water (2 x 15mL) then brine (15mL). The organic layer was separated, dried over magnesium sulphate and concentrated to give a colourless oil which crystalised on standing. The solid was purified by column chromatography on silica gel eluting with dichloromethane:methanol:0.88ammonia 97:3:0.25 to give a white solid.
¹H-NMR (CD₃OD, 400MHz) δ: 1.13 (t, 3H), 1.59 - 1.72 (m, 4H), 1.80 (m, 2H), 2.18 (m, 2H), 2.30 (s, 6H), 2.60 (t, 2H), 3.21 (s, 6H), 3.52 (m, 2H), 3.61 (t, 2H), 3.86 (t, 2H), 4.44 (m, 1 H), 4.62 (t, 2H). MS APCI+ m/z 433 [MH]⁺

### Examples 66-71

The following compounds were prepared by a method similar to that described for example 65 using the appropriate HNR³R⁴ amine and the appropriate chloro compound of preparations 46, 47, 48 and 49.

| | |
|---|---|
| 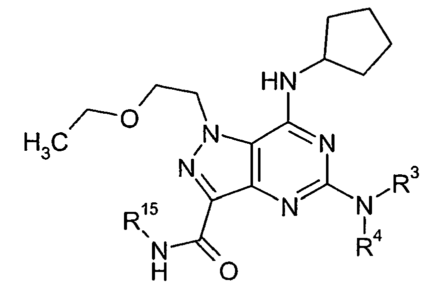 | |

| Ex | |
|---|---|
| 66 | R³ = -CH₂CH₃; R⁴ = H; R¹⁵ = -(CH₂)₂N(CH₃)₂ |
| | ¹H-NMR (CD₃OD, 400MHz) δ : 1.13 (t, 3H), 1.58 (m, 2H), 1.66 (m, 2H), 1.79 (m, 2H), 2.15 (m, 2H), 2.31 (s, 6H), 2.61 (t, 2H), 3.44 (m, 2H), 3.53 (m, 2H), 3.61 (t, 2H), 3.86 (t, 2H), 4.43 (m, 1 H), 4.62 (t, 2H). MS APCI+ m/z 433 [MH]⁺ |
| 67 | R³ = -CH₃; R⁴ = -CH₃; R¹⁵ = -CH₃ |
| | ¹H-NMR (CD₃OD, 400MHz) δ: 1.10 (t, 3H), 1.63 (m, 2H), 1.68 (m, 2H), 1.80 (m, 2H), 2.18 (m, 2H), 3.01 (s, 3H), 3.08 (s, 6H), 3.51 (m, 2H), 3.84 (t, 2H), 4.43 (m, 1 H), 4.61 (t, 2H). MS APCI+ m/z 376 [MH]⁺ |
| 68 | R³ = 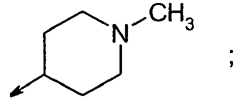 R⁴= -CH₃; R¹⁵ = -CH₃ |
| | ¹H-NMR (CD₃OD, 400MHz) δ: 1.12 (t, 3H), 1.60 - 1.83 (complex, 10H), 1.89 (m, 2H), 2.18 (m, 4H), 2.35 (s, 3H), 3.02 (s, 3H), 3.05 (s, 3H), 3.51 (m, 2H), 3.85 (t, 2H), 4.39 (m, 1 H), 4.61 (t, 2H), 4.69 (m, 1 H). MS APCI+ m/z 459 [MH]⁺ |
| 69 | R³ = -CH₃; R⁴ = -CH₃; R¹⁵ = -(CH₂)₂OCH₃ |
| | ¹H-NMR (CD₃OD, 400MHz) δ: 1.11 (t, 3H), 1.80 (m, 2H), 2.18 (m, 2H), 3.20 (s, 6H), 3.37 (s, 3H), 3.51 (m, 2H), 3.61 (m, 2H), 3.64 (m, 2H), 3.84 (t, 2H), 4.43 (m, 1 H), 4.61 (t, 2H),. MS APCI+ m/z 420 [MH]⁺ |
| | 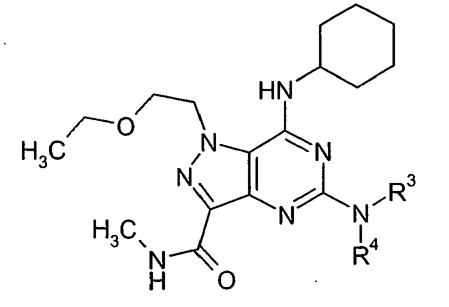 |
| 70 | -NR³R⁴ = 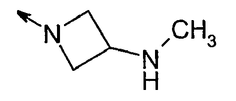 |
| | ¹H-NMR (CD₃OD, 400MHz) δ: 1.13 (t, 3H), 1.21 - 1.50 (m, 5H), 1.69 (m, 1 H), 1.82 (m, 2H), 2.12 (m, 2H), 2.37 (s, 3H), 3.01 (s, 3H), 3.53 (m, 2H), 3.63 (m, 1 H), 3.85 (m, 4H), 4.08 (m, 1 H), 4.26 (t, 2H), 4.63 (t, 2H). MS APCI+ m/z 431 [MH]⁺ |
| 71 | -NR³R⁴ = 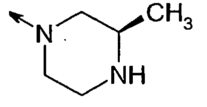 |
| | ¹H-NMR (CD₃OD, 400MHz) δ: 1.15 (m, 6H),1.24-1.53 (m, 5H), 1.73 (m, 1 H), 1.84 (m, 2H), 2.16 (m, 2H), 2.60 (m, 1 H), 2.80 (m, 2H), 2.95 (m, 1 H), 3.04 (m, 6H), 3.56 (m, 2H), 3.86 (m, 2H), 4.03 (m, 1 H), 4.61 (m, 4H). MS APCl+ m/z 445 [MH]⁺ |

Examples 68 and 71 also added N-ethyldiisopropylamine (0.39mmol) to the dimethyl sulphoxide solution

### Example 72

### 7-(Cyclohexylamino)-1-(2-ethoxyethyl)-N-methyl-5-[N-methyl-N-((3S)-1-methylpyrrolidin-3-yl)amino]-1H-pyrazolo[4,3-d]pyrimidine-3-carboxamide

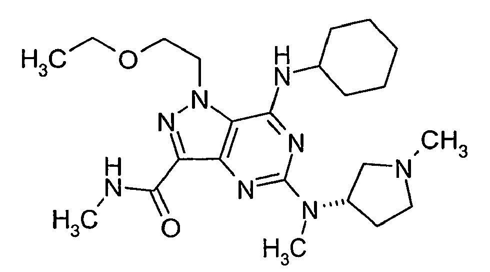

The amide of preparation 49 (100mg, 0.26mmol), the amine of preparation 62 (246mg, 1.30mmol), N-ethyldiisopropylamine (450µL, 2.58mmol) and tetraethylammonium fluoride (39mg, 0.26mmol) were dissolved in 1-methyl-2-pyrrolidinone (1.5mL) and the reaction mixture stirred at 180°C for 1 hour. The reaction mixture was concentrated *in vacuo* and the residue partitioned between water and dichloromethane. The organic layer was dried over magnesium sulphate and concentrated *in vacuo.* The residue was purified by column chromatography on silica gel eluting with dichloromethane:methanol:0.88ammonia 98:2:0.25 to yield the title product as a white solid, 15mg.
¹H-NMR (CD₃OD, 400MHz) δ: 1.11 (t, 3H), 1.22-1.32 (m, 5H), 1.75 (m, 3H), 2.13 (m, 4H), 2.40 (s, 3H), 2.57-2.91 (m, 4H), 3.05 (m, 6H), 3.56 (m, 2H), 3.83 (m, 2H), 4.04 (m, 1 H), 4.60 (m, 2H), 5.56 (m, 1 H). MS APCI+ m/z 459 [MH]⁺

### Example 73

### 1-(2-Ethoxyethyl)-N-methyl-5-[N-methyl-N-((3S)-1-methylpyrrolidin-3-yl)amino]-7-(4-methylpyridin-2-ylamino)-1H-pyrazolo[4,3-d]pyrimidine-3-carboxamide

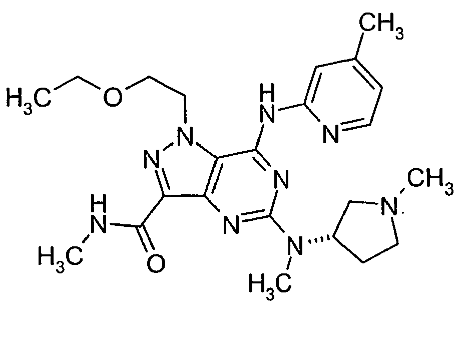

The title compound was prepared by a method similar to that described for example 72 using the amide of preparation 39 and the amine of preparation 63.
¹H-NMR (CD₃OD, 400MHz) δ: 1.11 (t, 3H), 1.90-2.90(m, 12H), 3.05(s, 3H), 3.18(s, 3H), 3.60(q, 2H), 3.93(t, 2H), 4.79(t, 2H), 5.54(m, 1 H), 6.96(d, 1 H), 8.17(d, 1 H), 8.24(m, 1 H). MS APCI+ m/z 468 [MH]⁺

### Example 74

### 1-(2-Ethoxyethyl)-N-methyl-5-((3S)-3-(1-methylpyrrolidin-3-yl)amino-7-(4-methylpridin-2-ylamino)-1H-pyrazolo[4,3-d]pyrimidine-3-carboxamide

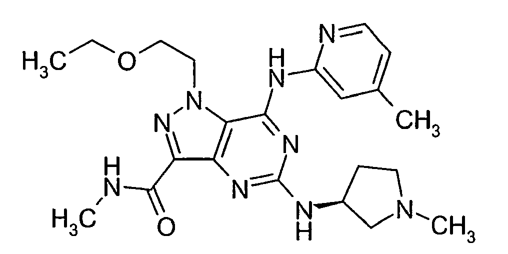

The title compound was prepared by a method similar to that described for example 72 using the amide of preparation 39 and the amine of preparation 65.
¹H-NMR (CD₃OD, 400MHz) δ: 1.08 (t, 3H), 1.96 (m, 1 H), 2.29 (m, 1 H), 2.40 (s, 3H), 2.47 (s, 3H), 3.04 (s, 3H), 3.44 (m, 2H), 3.62 (m, 3H), 3.79 (m, 1H), 3.89 (m, 3H), 4.77 (m, 2H), 6.93 (m, 1 H), 8.14 (m, 1 H), 8.44 (m, 1 H). MS APCI+ m/z 454 [MH]⁺

### Example 75

### 7-(Cyclopentylamino)-5-(dimethylamino)-1-(2-ethoxyethyl)-N-(piperidin-4-yl)-1H-pyrazolo[4,3-d]pyrimidine-3-carboxamide

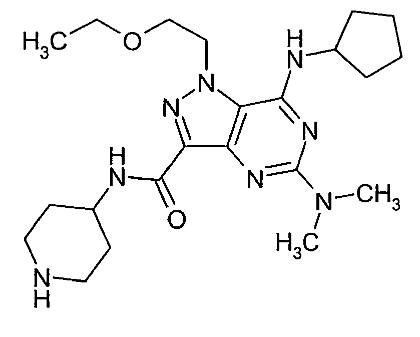

A solution of the amide of preparation 45 (60mg, 0.11 mmol) and dimethylamine (5.6M in ethanol) (80.4µL, 0.45mmol) in dimethyl sulphoxide (2mL) was heated in a sealed ReactiVial™ at 120°C for 4 hours. The reaction mixture was diluted with dichloromethane and washed with water (x2) and brine (x2). The organics were dried over magnesium sulphate and concentrated *in vacuo* to give a yellow oil, which was redissolved in dichloromethane (2mL) and trifluoroacetic acid (2mL) before being stirred at room temperature for 2hours. The reaction was concentrated in *vacuo,* re-dissolved in dichloromethane, washed with sodium carbonate solution, brine and then dried over magnesium sulphate. The solvent was removed *in vacuo* and the resulting oil purified by column chromatography on silica gel eluting with dichloromethane:methanol 98:2 to 95:5 to 90:10:1 to yield the title product.
¹H-NMR (CD₃OD 400MHz) δ: 1.13 (t, 3H), 1.64 (m, 6H), 1.79 (m, 2H), 2.16 (m, 4H), 2.87 (t, 2H), 3.19 (s, 8H), 3.53 (m, 2H), 3.86 (t, 2H), 4.10 (m, 1 H), 4.44 (m, 1 H), 4.63 (t, 2H). MS APCI+ m/z 445 [MH]⁺

### Examples 76-82

The following compounds were prepared by a method similar to that described for example 75 using the appropriate HNR³R⁴ amine and the appropriate chloro compound from preparations 44, 45, 47 and 48.

| | |
|---|---|
| 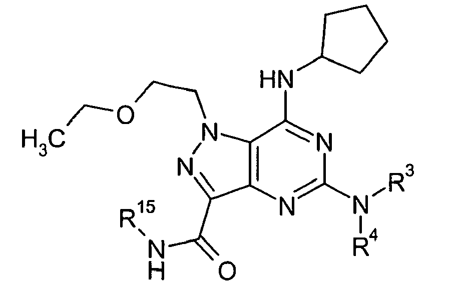 | |

| Ex | |
|---|---|
| 76 | R³ = -CH₂CH₃; R⁴ = H; R¹⁵ = 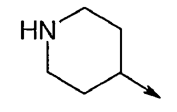 |
| | 1 H-NMR (CD₃OD 400MHz) δ: 1.13 (t, 3H), 1.25 (t, 3H), 1.54-1.71 (m, 6H), 1.79 (m, 2H), 2.05 (m, 2H), 2.15 (m, 2H), 2.76 (t, 2H), 3.09 (m, 2H), 3.40 (m, 2H), 3.53 (m, 2H), 3.86 (t, 2H), 4.07 (m, 1 H), 4.42 (m, 1 H), 4.62 (t, 2H). MS APCI+ m/z 445 [MH]+ |
| 77 | R³ = -CH₃; R⁴= -CH₃; R¹⁵ = -(CH₂)₂NH₂ |
| | ¹H-NMR (CD₃OD, 400MHz) δ: 1.12 (t, 3H), 1.58 - 1.73 (m, 4H), 1.79 (m, 2H), 2.19 (m, 2H), 3.10 (t, 2H), 3.20 (s, 6H), 3.52 (m, 2H), 3.69 (t, 2H), 3.85 (t, 2H), 4.44 (m, 1 H), 4.63 (t, 2H). MS APCI+ m/z 405 [MH]⁺ |
| 78 | R³ = -CH₂CH_{3;} R⁴ = H; R¹⁵ = -(CH₂)₂NH₂ |
| | ¹H-NMR (CD₃OD, 400MHz) δ: 1.13 (t, 3H), 1.23 (t, 3H), 1.55 - 1.71 (m, 4H), 1.80 (m, 2H), 2.15 (m, 2H), 2.93 (t, 2H), 3.42 (m, 2H), 3.53 (m, 2H), 3.58 (t, 2H), 3.86 (t, 2H), 4.43 (m, 1 H), 4.62 (t, 2H). MS APCI+ m/z 405 [MH]⁺ |
| 79 | R³ = -CH(CH₃)₂; R⁴= -CH₃; R¹⁵ = -(CH₂)₂NH₂ |
| | ¹H-NMR (CD₃OD, 400MHz) δ: 1.13 (t, 3H), 1.21 (d, 6H), 1.58 - 1.72 (m, 4H), 1.80 (m, 2H), 2.18 (m, 2H), 2.90 (t, 2H), 3.02 (s, 3H), 3.49 - 3.60 (m, 4H), 3.86 (t, 2H), 4.42 (m, 1 H), 4.63 (t, 2H), 5.11 (m, 1 H). MS APCI+ m/z 433 [MH]⁺ |
| 80 | R³ = -CH₂CH₃; R⁴= -CH₂CH₃; R¹⁵ = -(CH₂)₂NH₂ |
| | ¹H-NMR (CD₃OD, 400MHz) δ: 1.14 (t, 3H), 1.23 (t, 6H), 1.59 - 1.72 (m, 4H), 1.80 (m, 2H), 2.17 (m, 2H), 2.89 (t, 2H), 3.50 - 3.57 (m, 4H), 3.66 (m, 4H), 3.86 (t, 2H), 4.42 (m, 1 H), 4.63 (t, 2H). MS APCl⁺ m/z 433 [MH]⁺ |
| 81 | -NR³R⁴ = 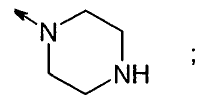 R¹⁵ = -CH₃ |
| | ¹H-NMR (CD₃OD, 400MHz) δ: 1.12 (t, 3H), 1.60 (m, 2H), 1.68 (m, 2H), 1.82 (m, 2H), 2.17 (m, 2H), 2.92 (m, 4H), 3.01 (s, 3H), 3.52 (m, 2H), 3.84 (m, 4H), 3.88 (m, 2H), 4.40 (m, 1 H), 4.61 (m, 2H). MS APCl+ m/z 417 [MH]⁺ |
| 82 | -NR³R⁴= 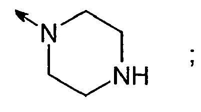 R¹⁵ = -(CH₂)₂OCH₃ |
| | ¹H-NMR (CD₃OD, 400MHz) δ: 1.12 (t, 3H), 1.63 (m, 2H), 1.70 (m, 2H), 1.80 (m, 2H), 2.17 (m, 2H), 2.90 (m, 4H), 3.40 (s, 3H), 3.51 (m, 2H), 3.61 (m, 2H), 3.63 (m, 2H), 3.83 (m, 6H), 4.41 (m, 1 H), 4.63 (t, 2H). MS APCl+ m/z 461 [MH]⁺ |

| | |
|---|---|
| • Examples 81 and 82 used *tert-*butyl piperazine-1-carboxylate as the HNR³R⁴ amine | |

### Example 83

### 1-Isobutyl-N-methyl-7-(4-methylpyridin-2-ylamino)-5-(piperazin-1-yl)-1H-pyrazolo[4,3-d]pyrimidine-3-carboxamide

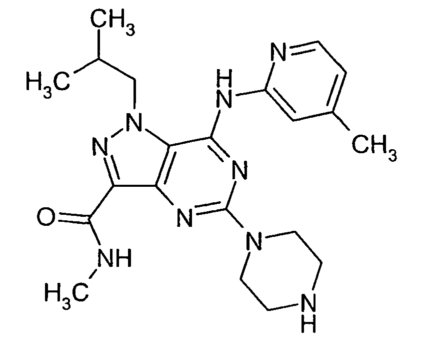

The compound of example 64 (20mg, 0.04mmol) was dissolved in a mixture of trifluoroacetic acid (500µL) and dichloromethane (5mL) and the reaction mixture stirred at room temperature for 18 hours. The reaction mixture was concentrated in *vacuo* and the residue partitioned between ethyl acetate (10mL) and 2M sodium carbonate solution (10mL). The aqueous was extracted with ethyl acetate (10mL), the organics were combined, dried over magnesium sulphate and concentrated in *vacuo.* The residue was purified by column chromatography on silica gel eluting with dichloromethane:methanol:ammonia 95:5:0 to 95:5:0.5 to yield the title product, 10mg.
¹H-NMR (CD₃OD, 400MHz) δ : 0.92(d, 6H), 2.29(m, 1H), 2.40(s, 3H), 2.95(m, 4H), 3.04(s, 3H), 3.81 (m, 4H), 4.51 (m, 2H), 6.97(m, 1H), 7.38(m, 1H), 8.16(d, 1H). MS APCI+ m/z 424 [MH]⁺

### Assay

The compounds of the invention are inhibitors of cyclic guanylate monophosphate (cGMP)-specific phosphodiesterase type 5 (PDE-5 inhibitors). Preferred compounds suitable for use in accordance with the present invention are potent and selective PDE5 inhibitors. *In vitro* PDE inhibitory activities against cyclic guanosine 3',5'-monophosphate (cGMP) and cyclic adenosine 3',5'-monophosphate (cAMP) phosphodiesterases can be determined by measurement of their IC₅₀ values (the concentration of compound required for 50% inhibition of enzyme activity).

The required PDE enzymes can be isolated from a variety of sources, including human corpus cavernosum, human and rabbit platelets, human cardiac ventricle, human skeletal muscle and bovine retina, essentially by a modification of the method of Thompson, WJ et al.; Biochemistry 18(23), 5228-5237, 1979, as described by Ballard SA et al.; J. Urology 159(6), 2164-2171, 1998. In particular, cGMP-specific PDE5 and cGMP-inhibited cAMP PDE3 can be obtained from human corpus cavernosum tissue, human platelets or rabbit platelets; cGMP-stimulated PDE2 was obtained from human corpus cavernosum; calcium/calmodulin (Ca/CAM)-dependent PDE1 from human cardiac ventricle; cAMP-specific PDE4 from human skeletal muscle; and photoreceptor PDE6 from bovine retina. Phosphodiesterases 7-11 can be generated from full length human recombinant clones transfected into SF9 cells.

Assays can be performed either using a modification of the "batch" method of Thompson WJ and Appleman MM; Biochemistry 10(2),311-316, 1971, essentially as described by Ballard SA et al.; J. Urology 159(6), 2164-2171, 1998, or using a scintillation proximity assay for the direct detection of [³H]-labelled AMP/GMP using a modification of the protocol described by Amersham plc under product code TRKQ7090/7100. In summary, for the scintillation proximity assay the effect of PDE inhibitors was investigated by assaying a fixed amount of enzyme in the presence of varying inhibitor concentrations and low substrate, (cGMP or cAMP in a 3:1 ratio unlabelled to [³H]-labeled at a concentration of -1/3 *Kₘ* or less) such that IC₅₀ ≅ *Kᵢ*. The final assay volume was made up to 100µl with assay buffer [20mM Tris-HCl pH 7.4, 5mM MgCl₂, 1mg/ml bovine serum albumin]. Reactions were initiated with enzyme, incubated for 30-60min at 30°C to give <30% substrate turnover and terminated with 50µl yttrium silicate SPA beads (containing 3mM of the respective unlabelled cyclic nucleotide for PDEs 9 and 11). Plates were re-sealed and shaken for 20min, after which the beads were allowed to settle for 30min in the dark and then counted on a TopCount plate reader (Packard, Meriden, CT) Radioactivity units were converted to % activity of an uninhibited control (100%), plotted against inhibitor concentration and inhibitor IC₅₀ values obtained using the 'Fit Curve' Microsoft Excel extension.

All compounds of the invention have an activity against PDE-5 of less than 10,000nM. IC₅₀ values for representative preferred compounds are listed in the table below.

| **Example** | **IC₅₀ (nM)** | | **Example** | **IC₅₀ (nM)** |
|---|---|---|---|---|
| 8 | 0.33 | | 32 | 0.73 |
| 14 | 0.26 | | 50 | 1.31 |
| 16 | 0.40 | | 56 | 1.63 |
| 17 | 0.24 | | 58 | 0.13 |
| 18 | 0.06 | | 60 | 0.57 |
| 19 | 0.09 | | 61 | 0.12 |
| 22 | 0.02 | | 62 | 1.88 |
| 23 | 0.04 | | 71 | 2.95 |
| 29 | 0.50 | | 73 | 0.42 |
| 30 | 1.65 | | | |

## Claims

1. A compound of formula (I) 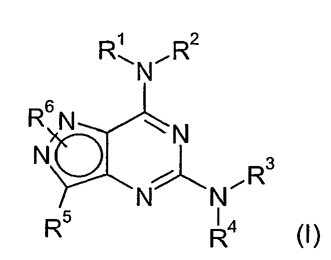 wherein
R¹ is a cyclic group selected from R^{A}, R^{B}, R^{c} and R^{D}, each of which is optionally substituted with one or more R⁷ groups;
R² is hydrogen or C₁-C₂ alkyl;
R³ and R⁴ are each independently C₁-C₈ alkyl, C₂-C₈ alkenyl, C₂-C₈ alkynyl or C₃-C₁₀ cycloalkyl, each of which is optionally substituted with one or more R⁸ groups, or R^{E}, which is optionally substituted with one or more R⁹ groups, or hydrogen;
or -NR³R⁴ forms R^{F}, which is optionally substituted with one or more R¹⁰ groups;
R⁵ is -Y-CONR¹⁵R¹⁶;
R⁶, which may be attached at N¹ or N², is C₁-C₆ alkyl, C₁-C₆ haloalkyl, C₂-C₆ alkenyl or C₂-C₆alkynyl, each of which is optionally substituted by C₁-C₆ alkoxy, C₁-C₆ haloalkoxy or a cyclic group selected from R^{J}, R^{K}, R^{L} and R^{M}, or R⁶ is R^{N}, C₃-C₇ cycloalkyl or C₃-C₇ halocycloalkyl, each of which is optionally substituted by C₁-C₆ alkoxy or C₁-C₆ haloalkoxy, or R⁶ is hydrogen;
R⁷ is halo, C₁-C₆ alkyl, C₁-C₆ haloalkyl, C₂-C₆ alkenyl, C₂-C₆ alkynyl, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹², CONR¹²R¹³ or CN;
R⁸ is halo, phenyl, C₁-C₆ alkoxyphenyl, OR¹², OC(O)R¹², NO₂, NR¹²R¹³, NR¹²C(O)R¹³, NR¹²CO₂R¹⁴, C(O)R¹², CO₂R¹², CONR¹²R¹³, CN, R^{G} or R^{H}, the last two of which are optionally substituted with one or more R⁹ groups;
R⁹ is C₁-C₆ alkyl, C₁-C₆ haloalkyl or CO₂R¹²;
R¹⁰ is halo, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, C₁-C₆ alkyl or C₁-C₆ haloalkyl, the last two of which are optionally substituted by R¹¹;
R¹¹ is phenyl, NR¹²R¹³ or NR¹²CO₂R¹⁴;
R¹² and R¹³ are each independently hydrogen, C₁-C₆ alkyl or C₁-C₆ haloalkyl;
R¹⁴ is C₁-C₆alkyl or C₁-C₆ haloalkyl;
R¹⁵ and R¹⁶ are each independently selected from
hydrogen,
C₁-C₆ haloalkyl,
C₁-C₆ alkyl optionally substituted with
R¹⁷,
-NR¹⁸R¹⁹,
-CO₂R²⁰,
-CONR²¹R²²,
R²³ or
phenyl optionally substituted by
halo,
C₁-C₆ alkyl or
R¹⁷
C₃-C₇ cycloalkyl optionally substituted with
C₁-C₆ alkyl,
R¹⁷ or
-NR¹⁸R¹⁹, and
R²³;
or NR¹⁵R¹⁶ constitutes a 3- to 8-membered ring which may optionally include one or more further heteroatoms selected from nitrogen, oxygen and sulphur, and which may optionally be further substituted with R¹⁷, C₁-C₆ haloalkyl, -CO₂R²⁰, -CONR²¹R²², oxo or C₁-C₆ alkyl optionally substituted by R¹⁷;
R¹⁷ is hydroxy, C₁-C₆ alkoxy, C₁-C₆ (haloalkyl)oxy or C₃-C₇ cycloalkyloxy;
R¹⁸ and R¹⁹ are each independently selected from hydrogen and C₁-C₆ alkyl;
or -NR¹⁸R¹⁹ constitutes an azetidine, pyrrolidine, piperidine or morpholine ring;
R²⁰ is hydrogen or C₁-C₆ alkyl;
R²¹ and R²² are each independently selected from hydrogen, C₁-C₆ alkyl, C₁-C₆ haloalkyl and C₃-C₇ cycloalkyl;
or -NR²¹R²² constitutes a 3- to 8-membered ring which may optionally include one or more further heteroatoms selected from nitrogen, oxygen and sulphur;
R²³ is a saturated 3- to 8-membered ring which includes at least one heteroatom selected from nitrogen, oxygen and sulphur, which ring may optionally be substituted by one or more C₁-C₆ alkyl groups, provided that the group R²³ is joined to the parent molecule by a covalent bond to a carbon atom of said ring;
R^{A} and R^{J} are each independently a C₃-C₁₀ cycloalkyl or C₃-C₁₀ cycloalkenyl group, each of which may be either monocyclic or, when there are an appropriate number of ring atoms, polycyclic and which may be fused to either
(a) a monocyclic aromatic ring selected from a benzene ring and a 5- or 6-membered heteroaromatic ring containing up to three heteroatoms selected from nitrogen, oxygen and sulphur, or
(b) a 5-, 6- or 7-membered heteroalicyclic ring containing up to three heteroatoms selected from nitrogen, oxygen and sulphur;
R^{B} and R^{K} are each independently a phenyl or naphthyl group, each of which may be fused to
(a) a C₅-C₇ cycloalkyl or C₅-C₇ cycloalkenyl ring,
(b) a 5-, 6- or 7-membered heteroalicyclic ring containing up to three heteroatoms selected from nitrogen, oxygen and sulphur, or
(c) a 5- or 6-membered heteroaromatic ring containing up to three heteroatoms selected from nitrogen, oxygen and sulphur;
R^{C}, R^{L} and R^{N} are each independently a monocyclic or, when there are an appropriate number of ring atoms, polycyclic saturated or partly unsaturated ring system containing between 3 and 10 ring atoms, of which at least one is a heteroatom selected from nitrogen, oxygen and sulphur, which ring may be fused to a C₅-C₇ cycloalkyl or C₅-C₇ cycloalkenyl group or a monocyclic aromatic ring selected from a benzene ring and a 5- or 6-membered heteroaromatic ring containing up to three heteroatoms selected from nitrogen, oxygen and sulphur;
R^{D} and R^{M} are each independently a 5- or 6-membered heteroaromatic ring containing up to three heteroatoms independently selected from nitrogen, oxygen and sulphur, which ring may further be fused to
(a) a second 5- or 6-membered heteroaromatic ring containing up to three heteroatoms selected from nitrogen, oxygen and sulphur;
(b) C₅-C₇ cycloalkyl or C₅-C₇ cycloalkenyl ring;
(c) a 5-, 6- or 7-membered heteroalicyclic ring containing up to three heteroatoms selected from nitrogen, oxygen and sulphur; or
(d) a benzene ring;
R^{E}, R^{F} and R^{G} are each independently a monocyclic or, when there are an appropriate number of ring atoms, polycyclic saturated ring system containing between 3 and 10 ring atoms, of which at least one is a heteroatom selected from nitrogen, oxygen and sulphur,
R^{H} is a 5- or 6-membered heteroaromatic ring containing up to three heteroatoms Independently selected from nitrogen, oxygen and sulphur; and
Y is a covalent bond, C₁-C₆ alkylenyl or C₃-C₇ cycloalkylenyl;
a tautomer thereof or a pharmaceutically acceptable salt or solvate of said compound or tautomer.

2. A compound according to claim 1 wherein R¹ is R^{A}, which is optionally substituted with one or more R⁷ groups; and
R^{A} is a C₃-C₁₀ cycloalkyl group, which may be either monocyclic or, when there are an appropriate number of ring atoms, polycyclic, which may be fused to either
(a) a monocyclic aromatic ring selected from a benzene ring and a 5- or 6-membered heteroaromatic ring containing up to three heteroatoms selected from nitrogen, oxygen and sulphur, or
(b) a 5-, 6- or 7-membered heteroalicyclic ring containing up to three heteroatoms selected from nitrogen, oxygen and sulphur.

3. A compound according to claim 1 wherein R¹ is R^{B}, R^{C}, or R^{D} each optionally substituted with one or more R⁷ groups, wherein
R⁵ is phenyl,
R^{c} is a monocyclic saturated or partly unsaturated ring system containing between 5 and 7 ring atoms, of which at least one Is a heteroatom selected from nitrogen, oxygen and sulphur,
R^{D} is furanyl, thienyl, pyrrolyl, pyrazolyl, imidazolyl, isoxazolyl, oxazolyl, isothiazolyl, thiazolyl, oxadiazolyl, pyridyl, pyridazinyl, pyrimidyl or pyrazinyl, and
R⁷ is fluoro, methyl, ethyl, hydroxy, methoxy, propoxy or CONHMe.

4. A compound according to any one of claims 1 to 3 wherein R² is hydrogen or methyl.

5. A compound according to any one of claims 1 to 4 wherein R³ is hydrogen or C,-C₄ alkyl, which is optionally substituted with one or more R⁸ groups, or R³ is azetidinyl, pyrrolidinyl or piperidinyl, each of which is optionally substituted with one or more R⁹ groups, wherein
R⁸ is hydroxy, methoxy, methoxyphenyl, NH₂, NHMe, NMe₂, NHCO₂^{t}Bu, NMeCO₂^{t}Bu, CO₂H, CONHMe, pyrrolidinyl, piperidinyl, morpholinyl or pyrazolyl, the last four of which are optionally substituted with one or more R⁹ groups, and
R⁹ is methyl or CO₂^{t}Bu.

6. A compound according to any one of claims 1 to 5 wherein R⁴ is hydrogen, methyl or ethyl.

7. A compound according to any one of claims 1 to 6 wherein -NR³R⁴ forms R^{F}, which is optionally substituted with one or more R¹⁰ groups, wherein
R^{F} is selected from azetidinyl, pyrrolidinyl, piperidinyl, piperazinyl, morpholinyl, 3-azabicyclo[3.1.0]hex-3-yl, homopiperazinyl, 2,5-diazabicyclo[4.3.0]non-2-yl, 3,8-diazabicyclo[3.2.1]oct-3-yl, 3,8-diazabicyclo[3.2.1]oct-8-yl, 1,4-diazabicyclo[4.3.0]non-4-yl and 1,4-diazabicyclo[3.2.2]non-4-yl, and
R¹⁰ is halo, methyl, ethyl, isopropyl, hydroxy, methoxy, NH₂, NHMe, NMe₂, NHCO₂^{t}Bu, CO₂H, CO₂^{t}Bu, oxo, benzyl, -CH₂NH₂, -CH₂NHMe, CH₂NMe₂ or-CH₂NMeCO₂^{t}Bu.

8. A compound according to any one of claims 1 to 7 wherein
R¹⁵ and R¹⁶ are each independently selected from hydrogen, C₁-C₆ alkyl optionally substituted with R¹⁷, -NR¹⁸R¹⁹, -CO₂R²⁰, -CONR²¹R²², R²³ or phenyl optionally substituted by halo, C₁-C₆ alkyl or R¹⁷, C₃-C₇ cycloalkyl and R²³, or NR¹⁵R¹⁶ constitutes a 5- to 7-membered ring which may optionally include one or more further heteroatoms selected from nitrogen and oxygen, and which may optionally be further substituted with R¹⁷, -CO₂R²⁰, -CONR²¹R²² or C₁-C₆ alkyl optionally substituted by R¹⁷;
R¹⁷ is hydroxy, C₁-C₆ alkoxy or C₃-C₇ cycloalkyloxy;
R²¹ and R²² are each independently selected from hydrogen, C₁-C₆ alkyl, and C₃ C₇ cycloalkyl, or -NR²¹R²² constitutes a 5- to 8-membered ring which may optionally include one or more further heteroatoms selected from nitrogen and oxygen; and
R²³ is a saturated 5- to 7-membered ring which includes at least one heteroatom selected from nitrogen and oxygen, which ring may optionally be substituted by one or more C₁-C₆ alkyl groups.

9. A compound according to any one of claims 1 to 8 wherein R⁶ is positioned on N¹.

10. A compound according to claim 9 wherein R⁶ is hydrogen, methyl, ethyl, isopropyl, isobutyl, methoxyethyl, methoxypropyl, ethoxyethyl, ethoxypropyl, propoxyethyl, 2,2,2-trifluoroethyl, tetrahydrofuranylmethyl, tetrahydropyranylmethyl, tetrahydropyranyl or pyridinylmethyl.

11. A compound according to claim 1 wherein
R¹ is a cyclic group selected from R^{A}, R^{B}, R^{C} and R^{D}, each of which is optionally substituted with one or more R⁷ groups;
R⁷ is halo, C₁-C₆ alkyl, C₁-C₆ haloalkyl, OR¹² or CONR¹²R¹³;
R⁸ is halo, phenyl, C₁-C₆ alkoxyphenyl, OR¹², NR¹²R¹³, NR¹²CO₂R¹⁴, CO₂R¹², CONR¹²R¹³, R^{G} or R^{H}, the last two of which are optionally substituted with one or more R⁹ groups;
R^{A} is a monocyclic C₅-C₇ cycloalkyl group;
R^{B} is phenyl;
R^{C} is a monocyclic saturated ring system containing between 5 and 7 ring atoms, of which at least one is a heteroatom selected from nitrogen, oxygen and sulphur;
R^{D} is a 5-membered heteroaromatic ring containing a heteroatom selected from nitrogen, oxygen and sulphur and optionally up to two further nitrogen atoms in the ring, or a 6-membered heteroaromatic ring including 1, 2 or 3 nitrogen atoms;
R^{E} is a monocyclic saturated ring system containing between 3 and 7 ring atoms containing one nitrogen atom;
R^{F} is a monocyclic or, when there are an appropriate number of ring atoms, polycyclic saturated ring system containing between 3 and 10 ring atoms containing at least one nitrogen atom and optionally one other atom selected from oxygen and sulphur;
R^{G} is a monocyclic saturated ring system containing between 3 and 7 ring atoms, of which at least one is a heteroatom selected from nitrogen, oxygen and sulphur; and
R^{H} is a 5- or 6-membered heteroaromatic ring containing up to two nitrogen atoms.
R³ is hydrogen, C₁-C₄ alkyl, which is optionally substituted with one or more R⁸ groups, or R^{E}, which is optionally substituted with one or more R⁹ groups;
R⁴ is hydrogen, C₁-C₆ alkyl or C₁-C₆ haloalkyl;
or -NR³R⁴ forms R^{F}, which is optionally substituted with one or more R¹⁰ groups;
R⁶ is C₁-C₄ alkyl or C₁-C₄ haloalkyl, each of which is optionally substituted by C,-C₄ alkoxy, C₁-C₄ haloalkoxy or a cyclic group selected from R^{J}, R^{L} and R^{M}, or R⁶ is R^{N} or hydrogen;
R^{J} is cyclopropyl or cyclobutyl;
R^{L} and R^{N} are each independently a monocyclic saturated ring system containing either 5 or 6 ring atoms, of which at least one is a heteroatom selected from nitrogen, oxygen and sulphur;
R^{M} is a 5- or 6-membered heteroaromatic ring containing a heteroatom selected from nitrogen, oxygen and sulphur; and
Y is a covalent bond.

12. A compound according to claim 1 selected from:
1-(2-ethoxyethyl)-*N*-ethyl-5-(ethylamino)-7-(4-methylpyridin-2-ylamino)-1*H-*pyrazolo[4,3-*d*]pyrimidine-3-carboxamide,
5-(dimethylamino)-1-(2-ethoxyethyl)-*N*-methyl-7-(4-methylpyridin-2-ylamino)-1*H-*pyrazolo[4,3-*d*]pyrimidine-3-carboxamide,
5-(dimethylamino)-1-(2-ethoxyethyl)-*N*-(2-(methylamino)ethyl)-7-(4-methylpyridin-2-ylamino)-1H-pyrazolo[4,3-*d*]pyrimidine-3-carboxamide,
5-(dimethylamino)-*N*-(2-(dimethylamino)ethyl)-1-(2-ethoxyethyl)-7-(4-methylpyridin-2-ylamino)-1*H*-pyrazolo[4,3-*d*]pyrimidine-3-carboxamide,
5-(dimethylamino)-1-(2-ethoxyethyl)-7-(4-methylpyridin-2-ylamino)-*N*-(piperidin-4-yl)-1*H*-pyrazolo[4,3-*d*]pyrimidine-3-carboxamide,
5-(dimethylamino)-1-(2-ethoxyethyl)-N-(2-methoxyethyl)-7-(4-methylpyridin-2-ylamino)-1*H*-pyrazolo[4,3-*d*]pyrimidine-3-carboxamide,
(2*R*)-2-{[5-(dimethylamino)-1-(2-ethoxyethyl)-7-(4-methylpyridin-2-ylamino)-1*H-*pyrazolo[4,3-*d*]pyrimidine-3-carbonyl]amino}propionicacid,
3-{[5-(dimethylamino)-1-(2-ethoxyethyl)-7-(4-methylpyridin-2-ylamino)-1*H*-pyrazolo[4,3-*d*]pyrimidine-3-carbonyl]amino}propionicacid,
1-(2-ethoxyethyl)-*N*-methyl-7-(4-methylpyridin-2-ylamino)-5-(piperazin-1-yl)-1*H-*pyrazolo[4,3-*d*]pyrimidine-3-carboxamide,
1-(2-ethoxyethyl)-*N*-methyl-5-((3*R*)-3-methylpiperazin-1-yl)-7-(4-methylpyridin-2-yl-amino)-1H-pyrazolo[4,3-*d*]pyrimidine-3-carboxamide,
1-(2-ethoxyethyl)-N-ethyl-5-((3R)-3-methylpiperazin-1-yl)-7-(4-methylpyridin-2-ylamino)-1*H*-pyrazolo[4,3-*d*]pyrimidine-3-carboxamide,
1-(2-ethoxyethyl)-5-(ethylamino)-*N*-methyl-7-(4-methylpyridin-2-ylamino)-1*H-*pyrazolo[4,3-*d*]pyrimidine-3-carboxamide.
1-(2-ethoxyethyl)-*N*-(2-methoxyethyl)-5-(methylamino)-7-(4-methylpyridin-2-ylamino)-1*H*-pyrazolo[4,3-*d*]pyrimidine-3-carboxamide.
5-(dimethylamino)-1-(2-ethoxyethy))-*N*-(2-hydroxyethyl)-7-(4-methylpyridin-2-ylamino)-1*H*-pyrazolo[4,3-*d*]pyrimidine-3-carboxamide,
1-(2-ethoxyethyl)-5-(ethylamino)-*N*-(2-methoxyethyl)-7-(4-methylpyridin-2-ylamino)-1*H-*pyrazolo[4,3-*d*]pyrimidine-3-carboxamide,
1-(2-ethoxyethyl)-5-(*N*-(2-hydroxyethyl)-*N*-methylamino)-*N*-methyl-7-(4-methylpyridin-2-ylamino)-1*H*-pyrazolo[4,3-*d*]pyrimidine-3-carboxamide,
1-(2-ethoxyethyl)-5-((2-methoxyethyl)amino)-*N*-ethyl-7-(4-methylpyridin-2-ylamino)-1*H*-pyrazolo[4,3-*d*]pyrimidine-3-carboxamide,
7-(cyclohexylamino)-1-(2-ethoxyethyl)-*N*-methyl-5-((3*R*)-3-methylpiperazin-1-yl)-1*H-*pyrazolo[4,3-*d*]pyrimidine-3-carboxamide, and
1-(2-ethoxyethyl)-*N*-methyl-5-[*N*-methyl-*N*-((3S)-1-methylpyrrolidin-3-yl)amino]-7-(4-methylpyridin-2-ylamino)-1*H*-pyrazolo[4,3-*d*]pyrimidine-3-carboxamide
and tautomers thereof and pharmaceutically acceptable salts and solvates of said compound or tautomer.

13. A pharmaceutical composition comprising a compound of formula (I) as claimed in any one of claims 1 to 12, or pharmaceutically acceptable salt or solvate thereof, and a pharmaceutically acceptable diluent or carrier.

14. A compound of formula (I) as claimed in any one of claims 1 to 12, or a pharmaceutically acceptable salt or solvate thereof, for use as a medicament for the treatment of a disease or condition selected from hypertension (including essential hypertension, pulmonary hypertension, secondary hypertension, isolated systolic hypertension, hypertension associated with diabetes, hypertension associated with atherosclerosis, and renovascular hypertension), congestive heart failure, angina (including stable, unstable and variant (Prinzmetal) angina), stroke, coronary artery disease, congestive heart failure, conditions of reduced blood vessel patency (such as post-percutaneous coronary angioplasty), peripheral vascular disease, atherosclerosis, nitrate-induced tolerance, nitrate tolerance, diabetes, impaired glucose tolerance, metabolic syndrome, obesity, sexual dysfunction (including male erectile disorder, impotence, female sexual arousal disorder, clitoral dysfunction, female hypoactive sexual desire disorder, female sexual pain disorder, female sexual orgasmic dysfunction and sexual dysfunction due to spinal cord Injury), premature labour, pre-eclampsia, dysmenorrhea, polycystic ovary syndrome, benign prostatic hyperplasia, bladder outlet obstruction, Incontinence, chronic obstructive pulmonary disease, acute respiratory failure, brorichitis, chronic asthma, allergic asthma, allergic rhinitis, gut motility disorders (including irritable bowel syndrome), Kawasakis syndrome, multiple sclerosis, Alzheimer's disease, psoriasis, skin necrosis, scarring, fibrosis, pain (particularly neuropathic pain), cancer, metastasis, baldness, nutcracker oesophagus, anal fissure and haemorrhoids.

15. Use according to claim 14 wherein the disease or condition is selected from essential hypertension, pulmonary hypertension, secondary hypertension, isolated systolic hypertension, hypertension associated with diabetes, hypertension associated with atherosclerosis, and renovascular hypertension.

## Patentansprüche

1. Verbindung der Formel (I) 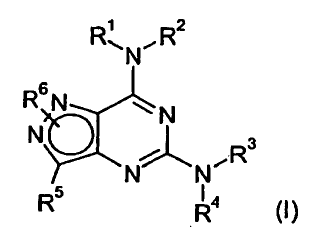 worin R¹ eine cyclische Gruppe, ausgewählt aus R^{A}, R^{B}, R^{C} und R^{D}, von denen jede gegebenenfalls mit einer oder mehreren R⁷-Gruppe(n) substituiert ist, ist;
R² Wasserstoff oder C₁-C₂-Alkyl ist;
R³ und R⁴ jeweils unabhängig C₁-C₈-Alkyl, C₂-C₈-Alkenyl, C₂-C₈-Alkinyl oder C₃-C₁₀-Cycloalkyl, von denen jedes gegebenenfalls substituiert ist mit einer oder mehreren R⁸-Gruppe(n), oder R^{E}, das gegebenenfalls substituiert ist mit einer oder mehreren R⁹-Gruppe(n), oder Wasserstoff sind;
oder -NR³R⁴ R^{F} bildet, das gegebenenfalls substituiert ist mit einer oder mehreren R¹⁰-Gruppe(n);
R⁵ -Y-CONR¹⁵R¹⁶ ist;
R⁶, das an N¹ oder N² gebunden sein kann, C₁-C₆-Alkyl, C₁-C₆-Halogenalkyl, C₂-C₆-Alkenyl oder C₂-C₆-Alkinyl ist, von denen jedes gegebenenfalls substituiert ist mit C₁-C₆-Alkoxy, C₁-C₆-Halogenalkoxy oder einer cyclischen Gruppe, ausgewählt aus R^{J}, R^{K}, R^{L} und R^{M}, oder R⁶ R^{N}, C₃-C₇-Cycloalkyl oder C₃-C₇-Halogencycloalkyl ist, von denen jedes gegebenenfalls mit C₁-C₆-Alkoxy oder C₁-C₆-Halogenalkoxy substituiert ist, oder R⁶ Wasserstoff ist;
R⁷ Halogen, C₁-C₆-Alkyl, C₁-C₆-Halogenalkyl, C₂-C₆-Alkenyl, C₂-C₆-Alkinyl, C₃-C₁₀-Cycloalkyl, C₃-C₁₀-Halogencycloalkyl, Phenyl, OR¹², OC(O)R¹², NO₂, NR¹²R¹³, NR¹²C(O)R¹³, NR¹²CO₂R¹⁴, C(O)R¹², CO₂R¹², CONR¹²R¹³ oder CN ist;
R⁸ Halogen, Phenyl, C₁-C₆-Alkoxyphenyl, OR¹², OC(O)R¹², NO₂ NR¹²R¹³, NR¹²C(O)R¹³, NR¹²CO₂R¹⁴, C(O)R¹², CO₂R¹², CONR¹²R¹³, CN, R^{G} oder R^{H} ist, wobei die letzten zwei von diesen gegebenenfalls mit einer oder mehreren R⁹-Gruppe(n) substituiert sind;
R⁹ C₁-C₆-Alkyl, C₁-C₆-Halogenalkyl oder CO₂R¹² ist;
R¹⁰ Halogen, C₃-C₁₀-Cycloalkyl, C₃-C₁₀-Halogencycloalkyl, Phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, Oxo, C₁-C₆-Alkyl oder C₁-C₆-Halogenalkyl ist, wobei die letzten zwei von diesen gegebenenfalls mit R¹¹ substituiert sind;
R¹¹ Phenyl, NR¹²R¹³ oder NR¹²CO₂R¹⁴ ist;
R¹² und R¹³ jeweils unabhängig Wasserstoff, C₁-C₆-Alkyl oder C₁-C₆-Halogenalkyl sind;
R¹⁴ C₁-C₆-Alkyl oder C₁-C₆-Halogenalkyl ist;
R¹⁵ und R¹⁶ jeweils unabhängig ausgewählt sind aus
Wasserstoff,
C₁-C₆-Halogenalkyl,
C₁-C₆-Alkyl, gegebenenfalls substituiert mit
R¹⁷,
-NR¹⁸R¹⁹,
-CO₂R²⁰,
-CONR²¹R²²,
R²³ oder
Phenyl, gegebenenfalls substituiert mit
Halogen,
C₁-C₆-Alkyl oder
R¹⁷,
C₃-C₇-Cycloalkyl, gegebenenfalls substituiert mit
C₁-C₆-Alkyl,
R¹⁷ oder
-NR¹⁸R¹⁹, und
R²³;
oder NR¹⁵R¹⁶ einen 3- bis 8-gliedrigen Ring bildet, der gegebenenfalls ein oder mehrere weitere Heteroatom(e), ausgewählt aus Stickstoff, Sauerstoff und Schwefel, umfassen kann und der gegebenenfalls außerdem substituiert sein kann mit R¹⁷, C₁-C₆-Halogenalkyl, -CO₂R²⁰, -CONR²¹R²², Oxo oder C₁-C₆-Alkyl, gegebenenfalls substituiert mit R¹⁷;
R¹⁷ Hydroxy, C₁-C₆-Alkoxy, C₁-C₆(Halogenalkyl)oxy oder C₃-C₇-Cycloalkyloxy ist;
R¹⁸ und R¹⁹ jeweils unabhängig ausgewählt sind aus Wasserstoff und C₁-C₆-Alkyl;
oder -NR¹⁸R¹⁹ einen Azetidin-, Pyrrolidin-, Piperidin- oder Morpholinring bildet;
R²⁰ Wasserstoff oder C₁-C₆-Alkyl ist;
R²¹ und R²² jeweils unabhängig ausgewählt sind aus Wasserstoff, C₁-C₆-Alkyl, C₁-C₆-Halogenalkyl und C₃-C₇-Cycloalkyl;
oder -NR²¹R²² einen 3- bis 8-gliedrigen Ring bildet, der gegebenenfalls ein oder mehrere weitere Heteroatom(e), ausgewählt aus Stickstoff, Sauerstoff und Schwefel, umfassen kann;
R²³ ein gesättigter 3- bis 8-gliedriger Ring ist, der wenigstens ein Heteroatom, ausgewählt aus Stickstoff, Sauerstoff und Schwefel, umfasst, wobei der Ring gegebenenfalls mit einer oder mehreren C₁-C₆-Alkylgruppe(n) substituiert sein kann, mit der Maßgabe, dass die Gruppe R²³ durch eine kovalente Bindung an ein Kohlenstoffatom des Rings an das Stammmolekül gebunden ist;
R^{A} und R^{J} jeweils unabhängig eine C₃-C₁₀-Cycloalkyl- oder C₃-C₁₀-Cycloalkenylgruppe sind, von denen jede monocyclisch oder, wenn es eine geeignete Anzahl von Ringatomen gibt, polycyclisch sein kann und die kondensiert sein kann an entweder
(a) einen monocyclischen aromatischen Ring, ausgewählt aus einem Benzolring und einem 5- oder 6-gliedrigen heteroaromatischen Ring, der bis zu drei Heteroatome, ausgewählt aus Stickstoff, Sauerstoff und Schwefel, enthält, oder
(b) einen 5-, 6- oder 7-gliedrigen heteroalicyclischen Ring, der bis zu drei Heteroatome, ausgewählt aus Stickstoff, Sauerstoff und Schwefel, enthält;
R^{B} und R^{K} jeweils unabhängig eine Phenyl- oder Naphthylgruppe sind, von denen jede kondensiert sein kann an
(a) einen C₅-C₇-Cycloalkyl- oder C₅-C₇-Cycloalkenyl-ring,
(b) einen 5-, 6- oder 7-gliedrigen heteroalicyclischen Ring, der bis zu drei Heteroatome, ausgewählt aus Stickstoff, Sauerstoff und Schwefel, enthält, oder
(c) einen 5- oder 6-gliedrigen heteroaromatischen Ring, der bis zu drei Heteroatome, ausgewählt aus Stickstoff, Sauerstoff und Schwefel, enthält;
R^{C}, R^{L} und R^{N} sind jeweils unabhängig ein monocyclisches oder, wenn es eine geeignete Anzahl von Ringatomen gibt, polycyclisches gesättigtes oder teilweise ungesättigtes Ringsystem, das zwischen 3 und 10 Ringatome enthält, von denen wenigstens eines ein Heteroatom, ausgewählt aus Stickstoff, Sauerstoff und Schwefel, ist, wobei der Ring an eine C₅-C₇-Cycloalkyl- oder C₅-C₇-Cycloalkenylgruppe oder einen monocyclischen aromatischen Ring, ausgewählt aus einem Benzolring und einem 5- oder 6-gliedrigen heteroaromatischen Ring, der bis zu drei Heteroatome, ausgewählt aus Stickstoff, Sauerstoff und Schwefel, enthält, kondensiert sein kann;
R^{D} und R^{M} jeweils unabhängig ein 5- oder 6-gliedriger heteroaromatischer Ring, der bis zu drei Heteroatome, unabhängig ausgewählt aus Stickstoff, Sauerstoff und Schwefel, enthält, sind, wobei der Ring außerdem kondensiert sein kann an
(a) einen zweiten 5- oder 6-gliedrigen heteroaromatischen Ring, der bis zu drei Heteroatome, ausgewählt aus Stickstoff, Sauerstoff und Schwefel, enthält;
(b) einen C₅-C₇-Cycloalkyl- oder C₅-C₇-Cycloalkenyl-ring;
(c) einen 5-, 6- oder 7-gliedrigen heteroalicyclischen Ring, der bis zu drei Heteroatome, ausgewählt aus Stickstoff, Sauerstoff und Schwefel, enthält; oder
(d) einen Benzolring;
R^{E}, R^{F} und R^{G} jeweils unabhängig ein monocyclisches oder, wenn es eine geeignete Anzahl von Ringatomen gibt, polycyclisches gesättigtes Ringsystem, das zwischen 3 und 10 Ringatome enthält, von denen wenigstens eines ein Heteroatom, ausgewählt aus Stickstoff, Sauerstoff und Schwefel, ist, sind;
R^{H} ein 5- oder 6-gliedriger heteroaromatischer Ring ist, der bis zu drei Heteroatome, unabhängig ausgewählt aus Stickstoff, Sauerstoff und Schwefel, enthält; und
Y eine kovalente Bindung, C₁-C₈-Alkylenyl oder C₃-C₇-Cycloalkylenyl ist;
ein Tautomer davon oder ein pharmazeutisch verträgliches Salz oder Solvat der Verbindung oder des Tautomers.

2. Verbindung nach Anspruch 1, worin R¹ R^{A} ist, das gegebenenfalls substituiert ist mit einer oder mehreren R⁷-Gruppe(n); und
R^{A} eine C₃-C₁₀-Cycloalkylgruppe ist, die entweder monocyclisch oder, wenn es eine geeignete Anzahl von Ringatomen gibt, polycyclisch sein kann, die kondensiert sein kann entweder an
(a) einen monocyclischen aromatischen Ring, ausgewählt aus einem Benzolring und einem 5- oder 6-gliedrigen heteroaromatischen Ring, der bis zu drei Heteroatome, ausgewählt aus Stickstoff, Sauerstoff und Schwefel, enthält, oder
(b) einen 5-, 6- oder 7-gliedrigen heteroalicyclischen Ring, der bis zu drei Heteroatome, ausgewählt aus Stickstoff, Sauerstoff und Schwefel, enthält.

3. Verbindung nach Anspruch 1, worin R¹ R^{B}, R^{C} oder R^{D} ist, von denen jedes gegebenenfalls mit einer oder mehreren R⁷-Gruppe(n) substituiert ist, worin
R^{B} Phenyl ist,
R^{C} ein monocyclisches gesättigtes oder teilweise ungesättigtes Ringsystem ist, das zwischen 5 und 7 Ringatome enthält, von denen wenigstens eines ein Heteroatom, ausgewählt aus Stickstoff, Sauerstoff und Schwefel, ist,
R^{D} Furanyl, Thienyl, Pyrrolyl, Pyrazolyl, Imidazolyl, Isoxazolyl, Oxazolyl, Isothiazolyl, Thiazolyl, Oxadiazolyl, Pyridyl, Pyridazinyl, Pyrimidyl oder Pyrazinyl ist und
R⁷ Fluor, Methyl, Ethyl, Hydroxy, Methoxy, Propoxy oder CONHMe ist.

4. Verbindung nach einem der Ansprüche 1 bis 3, worin R² Wasserstoff oder Methyl ist.

5. Verbindung nach einem der Ansprüche 1 bis 4, worin R³ Wasserstoff oder C₁-C₄-Alkyl, das gegebenenfalls mit einer oder mehreren R⁸-Gruppe(n) substituiert ist, ist oder R³ Azetidinyl, Pyrrolidinyl oder Piperidinyl ist, von denen jedes gegebenenfalls mit einer oder mehreren R⁹-Gruppe(n) substituiert ist,
R⁸ Hydroxy, Methoxy, Methoxyphenyl, NH₂, NHMe, NMe₂, NHCO₂^{t}Bu, NMeCO₂^{t}Bu, CO₂H, CONHMe, Pyrrolidinyl, Piperidinyl, Morpholinyl oder Pyrazolyl ist, von denen die letzten vier gegebenenfalls mit einer oder mehreren R⁹-Gruppe(n) substituiert sind, und
R⁹ Methyl oder CO₂^{t}Bu ist.

6. Verbindung nach einem der Ansprüche 1 bis 5, worin R⁴ Wasserstoff, Methyl oder Ethyl ist.

7. Verbindung nach einem der Ansprüche 1 bis 6, worin -NR³R⁴R^{F} bildet, das gegebenenfalls mit einer oder mehreren R¹⁰-Gruppe(n) substituiert ist, worin
R^{F} ausgewählt ist aus Azetidinyl, Pyrrolidinyl, Piperidinyl, Piperazinyl, Morpholinyl, 3-Azabicyclo[3.1.0]hex-3-yl, Homopiperazinyl, 2,5-Diazabicyclo[4.3.0]non-2-yl, 3,8-Diazabicyclo[3.2.1]oct-3-yl, 3,8-Diazabicylo[3.2.1]oct-8-yl, 1,4-Diazabicyclo[4.3.0]non-4-yl und 1,4-Diazabicyclo-[3.2.2]non-4-yl und
R¹⁰ Halogen, Methyl, Ethyl, Isopropyl, Hydroxy, Methoxy, NH₂, NHMe, NMe₂, NHCO₂^{t}Bu, CO₂H, CO₂^{t}Bu, Oxo, Benzyl, -CH₂NH₂, -CH₂HNMe, CH₂NMe₂ oder -CH₂NMeCO₂^{t}Bu ist.

8. Verbindung nach einem der Ansprüche 1 bis 7, worin
R¹⁵ und R¹⁶ jeweils unabhängig ausgewählt sind aus Wasserstoff, C₁-C₆-Alkyl, gegebenenfalls substituiert mit R¹⁷, -NR¹⁸R¹⁹, -CO₂R²⁰, -CONR²¹R²², R²³ oder Phenyl, gegebenenfalls substituiert mit Halogen, C₁-C₆-Alkyl oder R¹⁷, C₃-C₇-Cycloalkyl und R²³, oder NR¹⁵R¹⁶ einen 5- bis 7-gliedrigen Ring bildet, der gegebenenfalls ein oder mehrere weitere Heteroatom(e), ausgewählt aus Stickstoff und Sauerstoff, enthalten kann und der gegebenenfalls außerdem substituiert sein kann mit R¹⁷, -CO₂R²⁰, -CONR²¹R²² oder C₁-C₆-Alkyl, das gegebenenfalls mit R¹⁷ substituiert ist;
R¹⁷ Hydroxy, C₁-C₆-Alkoxy oder C₃-C₇-Cycloalkyloxy ist;
R²¹ und R²² jeweils unabhängig ausgewählt sind aus Wasserstoff, C₁-C₆-Alkyl und C₃-C₇-Cycloalkyl oder -NR²¹R²² einen 5- bis 8-gliedrigen Ring bildet, der gegebenenfalls ein oder mehrere weitere Heteroatom(e), ausgewählt aus Stickstoff und Sauerstoff, enthalten kann; und
R²³ ein gesättigter 5- bis 7-gliedriger Ring ist, der wenigstens ein Heteroatom, ausgewählt aus Stickstoff und Sauerstoff, beinhaltet, wobei dieser Ring gegebenenfalls mit einer oder mehreren C₁-C₆-Alkylgruppe(n) substituiert sein kann.

9. Verbindung nach einem der Ansprüche 1 bis 8, worin R⁶ an N¹ positioniert ist.

10. Verbindung nach Anspruch 9, worin R⁶ Wasserstoff, Methyl, Ethyl, Isopropyl, Isobutyl, Methoxyethyl, Methoxypropyl, Ethoxyethyl, Ethoxypropyl, Propoxyethyl, 2,2,2-Trifluorethyl, Tetrahydrofuranylmethyl, Tetrahydropyranylmethyl, Tetrahydropyranyl oder Pyridinylmethyl ist.

11. Verbindung nach Anspruch 1, worin
R¹ eine cyclische Gruppe, ausgewählt aus R^{A}, R^{B}, R^{C} und R^{D}, ist, von denen jede gegebenenfalls mit einer oder mehreren R⁷-Gruppe(n) substituiert ist;
R⁷ Halogen, C₁-C₆-Alkyl, C₁-C₆-Halogenalkyl, OR¹² oder CONR ¹²R¹³ ist;
R⁸ Halogen, Phenyl, C₁-C₆-Alkoxyphenyl, OR¹², NR¹²R¹³, NR¹²CO₂R¹⁴, CO₂R¹², CONR¹²R¹³, R^{G} oder R^{H} ist, wobei die letzten zwei davon gegebenenfalls mit einer oder mehreren R⁹-Gruppe(n) substituiert sind;
R^{A} eine monocyclische C₅-C₇-Cycloalkylgruppe ist;
R^{B} Phenyl ist;
R^{C} ein monocyclisches gesättigtes Ringsystem ist, das zwischen 5 und 7 Ringatome enthält, von denen wenigstens eines ein Heteroatom, ausgewählt aus Stickstoff, Sauerstoff und Schwefel, ist;
R^{D} ein 5-gliedriger heteroaromatischer Ring, der ein Heteroatom, ausgewählt aus Stickstoff, Sauerstoff und Schwefel, und gegebenenfalls bis zu zwei weitere Stickstoffatome im Ring enthält, oder ein 6-gliedriger heteroaromatischer Ring, der 1, 2 oder 3 Stickstoffatome umfasst, ist;
R^{E} ein monocyclisches gesättigtes Ringsystem, das zwischen 3 und 7 Ringatome, die ein Stickstoffatom enthalten, enthält, ist;
R^{F} ein monocyclisches oder, wenn es eine geeignete Anzahl von Ringatomen gibt, polycyclisches gesättigtes Ringsystem, das zwischen 3 und 10 Ringatome, die wenigstens ein Stickstoffatom und gegebenenfalls ein anderes Atom, ausgewählt aus Sauerstoff und Schwefel, enthalten, enthält, ist;
R^{G} ein monocyclisches gesättigtes Ringsystem ist, das zwischen 3 und 7 Ringatome enthält, von denen wenigstens eines ein Heteroatom, ausgewählt aus Stickstoff, Sauerstoff und Schwefel, ist; und
R^{H} ein 5- oder 6-gliedriger heteroaromatischer Ring ist, der bis zu zwei Stickstoffatome enthält;
R³ Wasserstoff, C₁-C₄-Alkyl, das gegebenenfalls mit einer oder mehreren R⁸-Gruppe(n) substituiert ist, oder R^{E}, das gegebenenfalls mit einer oder mehreren R⁹-Gruppe(n) substituiert ist, ist;
R⁴ Wasserstoff, C₁-C₆-Alkyl oder C₁-C₆-Halogenalkyl ist;
oder -NR³R⁴ R^{F} bildet, das gegebenenfalls mit einer oder mehreren R¹⁰-Gruppe(n) substituiert ist;
R⁶ C₁-C₄-Alkyl oder C₁-C₄-Halogenalkyl ist, von denen jedes gegebenenfalls mit C₁-C₄-Alkoxy, C₁-C₄-Halogenalkoxy oder einer cyclischen Gruppe, ausgewählt aus R^{J}, R^{L} und R^{M}, substituiert ist, oder R⁶ R^{N} oder Wasserstoff ist;
R^{J} Cyclopropyl oder Cyclobutyl ist;
R^{L} und R^{M} jeweils unabhängig ein monocyclisches gesättigtes Ringsystem sind, das entweder 5 oder 6 Ringatome enthält, von denen wenigstens eines ein Heteroatom, ausgewählt aus Stickstoff, Sauerstoff und Schwefel, ist;
R^{M} ein 5- oder 6-gliedriger heteroaromatischer Ring ist, der ein Heteroatom, ausgewählt aus Stickstoff, Sauerstoff und Schwefel, enthält; und
Y eine kovalente Bindung ist.

12. Verbindung nach Anspruch 1, ausgewählt aus:
1-(2-Ethoxyethyl)-N-ethyl-5-(ethylamino)-7-(4-methylpyridin-2-ylamino)-1H-pyrazolo[4,3-d]pyrimidin-3-carboxamid,
5-(Dimethylamino)-1-(2-ethoxyethyl)-N-methyl-7-(4-methylpyridin-2-ylamino)-1H-pyrazolo[4,3-d]pyrimidin-3-carboxamid,
5-(Dimethylamino)-1-(2-ethoxyethyl)-N-(2-(methylamino)-ethyl)-7-(4-methylpyridin-2-ylamino)-1H-pyrazolo[4,3-d]pyrimidin-3-carboxamid,
5-(Dimethylamino)-N-(2-(dimethylamino)ethyl)-1-(2-ethoxyethyl)-7-(4-methylpyridin-2-ylamino)-1H-pyrazolo[4,3-d]-pyrimidin-3-carboxamid,
5-(Dimethylamino)-1-(2-ethoxyethyl)-7-(4-methylpyridin-2-ylamino)-N-(piperidin-4-yl)-1H-pyrazolo[4,3-d]pyrimidin-3-carboxamid,
5-(Dimethylamino)-1-(2-ethoxyethyl)-N-(2-methoxyethyl)-7-(4-methylpyridin-2-ylamino)-1H-pyrazolo[4,3-d]pyrimidin-3-carboxamid,
(2R)-2-{[5-(Dimethylamino)-1-(2-ethoxyethyl)-7-(4-methylpyridin-2-ylamino)-1H-pyrazolo[4,3-d]pyrimidin-3-carbonyl]amino}propionsäure;
3-{[5-(Dimethylamino)-1-(2-ethoxyethyl)-7-(4-methylpyridin-2-ylamino)-1H-pyrazolo[4,3-d]pyrimidin-3-carbonyl]-amino}propionsäure,
1-(2-Ethoxyethyl)-N-methyl-7-(4-methylpyridin-2-ylamino)-5-(piperazin-1-yl)-1H-pyrazolo[4,3-d]pyrimidin-3-carboxamid,
1-(2-Ethoxyethyl)-N-methyl-5-((3R)-3-methylpiperazin-1-yl)-7-(4-methylpyridin-2-ylamino)-1H-pyrazolo[4,3-d]pyrimidin-3-carboxamid,
1-(2-Ethoxyethyl)-N-ethyl-5-((3R)-3-methylpiperazin-1-yl)-7-(4-methylpyridin-2-ylamino)-1H-pyrazolo[4,3-d]pyrimidin-3-carboxamid,
1-(2-Ethoxyethyl)-5-(ethylamino)-N-methyl-7-(4-methylpyridin-2-ylamino)-1H-pyrazolo[4,3-d]pyrimidin-3-carboxamid,
1-(2-Ethoxyethyl)-N-(2-methoxyethyl)-5-(methylamino)-7-(4-methylpyridin-2-ylamino)-1H-pyrazolo[4,3-d]pyrimidin-3-carboxamid,
5-(Dimethylamino)-1-(2-ethoxyethyl)-N-(2-hydroxyethyl)-7-(4-methylpyridin-2-ylamino)-1H-pyrazolo[4,3-d]pyrimidin-3-carboxamid,
1-(2-Ethoxyethyl)-5-(ethylamino)-N-(2-methoxyethyl)-7-(4-methylpyridin-2-ylamino)-1H-pyrazolo[4,3-d]pyrimidin-3-carboxamid,
1-(2-Ethoxyethyl)-5-(N-(2-hydroxyethyl)-N-methylamino)-N-methyl-7-(4-methylpyridin-2-ylamino)-1H-pyrazolo[4,3-d]-pyrimidin-3-carboxamid,
1-(2-Ethoxyethyl)-5-((2-methoxyethyl)amino)-N-methyl-7-(4-methylpyridin-2-ylamino)-1H-pyrazolo[4,3-d]pyrimidin-3-carboxamid,
7-(Cyclohexylamino)-1-(2-ethoxyethyl)-N-methyl-5-((3R)-3-methylpiperazin-1-yl)-1H-pyrazolo[4,3-d]pyrimidin-3-carboxamid und
1-(2-Ethoxyethyl)-N-methyl-5-[N-methyl-N-((3S)-1-methylpyrrolidin-3-yl)amino]-7-(4-methylpyridin-2-ylamino)-1H-pyrazolo[4,3-d]pyrimidin-3-carboxamid
und Tautomeren davon und pharmazeutisch verträglichen Salzen und Solvaten der Verbindung oder des Tautomers.

13. Pharmazeutische Zusammensetzung, umfassend eine Verbindung der Formel (I), wie sie in einem der Ansprüche 1 bis 12 beansprucht ist, oder ein pharmazeutisch verträgliches Salz oder Solvat davon und ein pharmazeutisch verträgliches Verdünnungsmittel oder einen pharmazeutisch verträglichen Träger.

14. Verbindung der Formel (I), wie sie in einem der Ansprüche 1 bis 12 beansprucht wird, oder ein pharmazeutisch verträgliches Salz oder Solvat davon zur Verwendung als Medikament für die Behandlung einer Erkrankung oder eines Zustandes, ausgewählt aus Hypertonie (einschließlich essentieller Hypertonie, pulmonaler Hypertonie, Sekundärhypertonie, isoliertem systolischem Hochdruck, Hypertonie, assoziiert mit Diabetes, Hypertonie, assoziiert mit Atherosklerose, und renovaskulärer Hypertonie), kongestiver Herzinsuffizienz, Angina (einschließlich stabiler, instabiler und varianter (Prinzmetal-)Angina), Schlaganfall, Koronararterienerkrankung, kongestiver Herzinsuffizienz, Zuständen reduzierter Blutgefäßdurchgängigkeit (z.B. nach perkutaner Koronarangioplastie), Erkrankung der peripheren Gefäße, Atherosklerose, Nitrat-induzierter Toleranz, Nitrattoleranz, Diabetes, verschlechterter Glucosetoleranz, Stoffwechselsyndrom, Adipositas, Sexualdysfunktion (einschließlich männlicher Erektionsstörung, Impotenz, Störung der weiblichen sexuellen Erregbarkeit, Klitorisdysfunktion, weibliche hypoaktive Störung des Verlangens nach Sex, weiblicher Sexschmerzstörung, weiblicher sexueller Orgasmus-Dysfunktion und sexueller Dysfunktion, bedingt durch Rückenmarksverletzung), Frühgeburt, Pre-Eklampsie, Dysmenorrhöe, polycystischem Ovarialsyndrom, benigner Prostatahyperplasie, Blasenobstruktion, Inkontinenz, chronischer obstruktiver Lungenerkrankung, respiratorischem Versagen, Bronchitis, chronischem Asthma, allergischem Asthma, allergischer Rhinitis, Motilitätsstörungen des Darms (einschließlich Reizdarmsyndrom), Kawasaki-Syndrom, multipler Sklerose, Alzheimer-Erkrankung, Psoriasis, Hautnekrose, Narbenbildung, Fibrose, Schmerzen (insbesondere neuropathischer Schmerz), Krebs, Metastasen, Kahlköpfigkeit, Nussknacker-Ösophagus, Analfissur und Hämorrhoiden.

15. Verwendung nach Anspruch 14, wobei die Erkrankung oder der Zustand ausgewählt aus ist essentieller Hypertonie, pulmonaler Hypertonie, Sekundärhypertonie, isoliertem systolischem Hochdruck, Hypertonie, assoziiert mit Diabetes, Hypertonie, assoziiert mit Atherosklerose, und renovaskulärer Hypertonie.

## Revendications

1. Composé de formule (I) 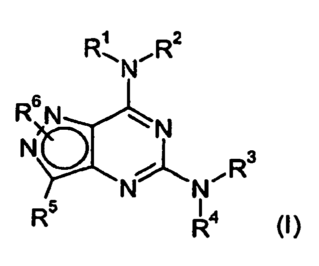 dans laquelle
R¹ représente un groupe cyclique choisi entre des groupes R^{A}, R^{B}, R^{C} et R^{D}, chacun étant facultativement substitué avec un ou plusieurs groupes R⁷ ;
R² représente un atome d'hydrogène ou un groupe alkyle en C₁ ou C₂ ;
R³ et R⁴ représentent chacun indépendamment un groupe alkyle en C₁ à C₈, alcényle en C₂ à C₈, alcynyle en C₂ à C₈
ou cycloalkyle en C₃ à C₁₀, chacun étant facultativement substitué avec un ou plusieurs groupes R⁸, ou un groupe R^{E} qui est facultativement substitué avec un ou plusieurs groupes R⁹ ou un atome d'hydrogène ;
ou bien -NR³R⁴ forme un groupe R^{F} qui est facultativement substitué avec un ou plusieurs groupes R¹⁰ ;
R⁵ représente un groupe -Y-CONR¹⁵R¹⁶ ;
R⁶, qui peut être fixé à N¹ ou à N², représente un groupe alkyle en C₁ à C₆, halogénalkyle en C₁ à C₆, alcényle en C₂ à C₆ ou alcynyle en C₂ à C₆, chacun étant facultativement substitué avec un groupe alkoxy en C₁ à C₆, halogénalkoxy en C₁ à C₆ ou un groupe cyclique choisi entre R^{J}, R^{K}, R^{L} et R^{M}, ou bien R⁶ représente un groupe R^{N}, cycloalkyle en C₃ à C₇ ou halogénocycloalkyle en C₃ à C₇, chacun de ces groupes étant facultativement substitué avec un substituant alkoxy en C₁ à C₆ ou halogénalkoxy en C₁ à C₆, ou bien R⁶ représente un atome d'hydrogène ;
R⁷ représente un groupe halogéno, alkyle en C₁ à C₆, halogénalkyle en C₁ à C₆, alcényle en C₂ à C₆, alcynyle en C₂ à C₆, cycloalkyle en C₃ à C₁₀, halogénocycloalkyle en C₃ à C₁₀, phényle, OR¹², OC(O)R¹², NO₂, NR¹²R¹³, NR¹²C(O) R¹³, NR¹²CO₂R¹⁴, C(O)R¹², CO₂R¹², CONR¹²R¹³ ou CN ;
R⁸ représente un groupe halogéno, (alkoxy en C₁ à C₆) phényle, OR¹², OC(O)R¹², NO₂, NR¹²R¹³, NR¹²C(O)R¹³, NR¹²CO₂R¹⁴, C(O)R¹², CO₂R¹², CONR¹²R¹³, CN, R^{G} ou R^{H}, ces deux derniers groupes étant facultativement substitués avec un ou plusieurs groupes R⁹ ;
R⁹ représente un groupe alkyle en C₁ à C₆, halogénalkyle en C₁ à C₆ ou CO₂R¹² ;
R¹⁰ représente un groupe halogéno, cycloalkyle en C₃ à C₁₀, halogénocycloalkyle en C₃ à C₁₀, phényle, OR¹², OC(O)R¹², NO₂, NR¹²R¹³, NR¹²C(O)R¹³, NR¹²CO₂R¹⁴ , C(O)R¹², CO₂R¹³, CONR¹²R¹³, CN, oxo, alkyle en C₁ à C₆ ou halogénalkyle en C₁ à C₆, ces deux derniers groupes étant facultativement substitués avec R¹¹;
R¹¹ représente un groupe phényle, NR¹²R¹³ ou NR¹²CO₂R¹⁴ _{;}
R¹² et R¹³ représentent chacun indépendamment un atome d'hydrogène, un groupe alkyle en C₁ à C₆ ou halogénalkyle en C₁ à C₆ ;
R¹⁴ représente un groupe alkyle en C₁ à C₆ ou halogénalkyle en C₁ à C₆ ;
R¹⁵ et R¹⁶ sont choisis chacun indépendamment entre :
un atome d'hydrogène,
un groupe halogénalkyle en C₁ à C₆,
un groupe alkyle en C₁ à C₆ facultativement substitué avec un substituant
R¹⁷,
-NR¹⁸R¹⁹,
-CO₂R²⁰,
-CONR²¹R²²,
R²³ ou
phényle facultativement substitué avec un substituant
halogéno,
alkyle en C₁ à C₆, ou
R¹⁷,
cycloalkyle en C₃ à C₇ facultativement substitué avec un substituant
alkyle en C₁ à C₆,
R¹⁷ ou
-NR¹⁸R¹⁹ et
R²³;
ou bien NR¹⁵R¹⁶ représente un noyau tri- à octogonal qui peut comprendre facultativement un ou plusieurs hétéroatomes supplémentaires choisis entre l'azote, l'oxygène et le soufre, et qui peut être en outre facultativement substitué avec un substituant R¹⁷, halogénalkyle en C₁ à C₆, -CO₂R²⁰, -CONR²¹R²², oxo ou alkyle en C₁ à C₆ facultativement substitué avec R¹⁷;
R¹⁷ représente un groupe hydroxy, alkoxy en C₁ à C₆, (halogénalkyl)oxy en C₁ à C₆ ou cycloalkyloxy en C₃ à C₇ ;
R¹⁸ et R¹⁹ sont choisis chacun indépendamment entre un atome d'hydrogène et un groupe alkyle en C₁ à C₆ ;
ou bien -NR¹⁸R¹⁹ représente un noyau azétidine, pyrrolidine, pipéridine ou morpholine ;
R²⁰ représente un atome d'hydrogène ou un groupe alkyle en C₁ à C₆ ;
R²¹ et R²² sont choisis chacun indépendamment entre un atome d'hydrogène, des groupes alkyle en C₁ à C₆, halogénalkyle en C₁ à C₆ et cycloalkyle en C₃ à C₇ ;
ou bien -NR²¹R²² représente un noyau tri- à octogonal qui peut comprendre facultativement un ou plusieurs hétéroatomes supplémentaires choisis entre l'azote, l'oxygène et le soufre ;
R²³ représente un noyau tri- à octogonal saturé qui comprend au moins un hétéroatome choisi entre l'azote, l'oxygène et le soufre, noyau qui peut être facultativement substitué avec un ou plusieurs groupes alkyle en C₁ à C₆, sous réserve que le groupe R²³ soit joint à la molécule parentale par une liaison covalente à un atome de carbone dudit noyau ;
R^{A} et R^{J} représentent chacun indépendamment un groupe cycloalkyle en C₃ à C₁₀ ou cycloalcényle en C₃ à C₁₀, chacun de ces groupes pouvant être monocyclique ou, lorsqu'il existe un nombre approprié d'atomes dans le noyau, polycyclique et pouvant être condensé à
(a) un noyau aromatique monocyclique choisi entre un noyau benzénique et un noyau hétéroaromatique penta- ou hexagonal contenant jusqu'à trois hétéroatomes choisis entre l'azote, l'oxygène et le soufre, ou
(b) un noyau hétéroalicyclique penta-, hexa- ou heptagonal contenant jusqu'à trois hétéroatomes choisis entre l'azote, l'oxygène et le soufre ;
R^{B} et R^{K} représentent chacun indépendamment un groupe phényle ou naphtyle, chacun pouvant être facultativement condensé à
(a) un noyau cycloalkyle en C₅ à C₇ ou cycloalcényle en C₅ à C₇,
(b) un noyau hétéroalicyclique penta-, hexa- ou pentagonal contenant jusqu'à trois hétéroatomes choisis entre l'azote, l'oxygène et le soufre, ou
(c) un noyau hétéroaromatique penta- ou hexagonal contenant jusqu'à trois hétéroatomes choisis entre l'azote, l'oxygène et le soufre ;
R^{C}, R^{L} et R^{N} représentent chacun indépendamment un système de noyau monocyclique ou, lorsqu'il existe un nombre approprié d'atomes dans le noyau, polycyclique saturé ou partiellement insaturé contenant 3 à 10 atomes dans le noyau, dont au moins l'un est un hétéroatome choisi entre l'azote, l'oxygène et le soufre, noyau qui peut être condensé à un groupe cycloalkyle en C₅ à C₇ ou cycloalcényle en C₅ à C₇ ou un noyau aromatique monocyclique choisi entre un noyau benzénique et un noyau hétéroaromatique penta- ou hexagonal contenant jusqu'à trois hétéroatomes choisis entre l'azote, l'oxygène et le soufre ;
R^{D} et R^{M} représentent chacun indépendamment un noyau hétéroaromatique penta- ou hexagonal contenant jusqu'à trois hétéroatomes choisis indépendamment entre l'azote, l'oxygène ou le soufre, noyau qui peut être en outre condensé à
(a) un second noyau hétéroaromatique penta- ou hexagonal contenant jusqu'à trois hétéroatomes choisis entre l'azote, l'oxygène et le soufre ;
(b) un noyau cycloalkyle en C₅ à C₇ ou cycloalcényle en C₅ à C₇ ;
(c) un noyau hétéroalicyclique penta-, hexa- ou heptagonal contenant jusqu'à trois hétéroatomes choisis entre l'azote, l'oxygène et le soufre ; ou
(d) un noyau benzénique ;
R^{E}, R^{F} et R^{G} représentent chacun indépendamment un système de noyau saturé monocyclique ou, lorsqu'il existe un nombre approprié d'atomes dans le noyau, polycyclique contenant 3 à 10 atomes dans le noyau, dont au moins l'un est un hétéroatome choisi entre l'azote, l'oxygène et le soufre ;
R^{H} représente un noyau hétéroaromatique penta- ou hexagonal contenant jusqu'à trois hétéroatomes choisis indépendamment entre l'azote, l'oxygène et le soufre ; et
Y représente une liaison covalente, un groupe alkylényle en C₁ à C₆ ou cycloalkylényle en C₃ à C₇ ;
une de ses formes tautomères ou un sel ou produit de solvatation pharmaceutiquement acceptable dudit composé ou de ladite forme tautomère.

2. Composé suivant la revendication 1, dans lequel R¹ représente un groupe R^{A}, qui est facultativement substitué avec un ou plusieurs groupes R⁷ ; et
R^{A} représente un groupe cycloalkyle en C₃ à C₁₀, qui peut être monocyclique ou, lorsqu'il existe un nombre approprié dans le noyau, polycyclique, qui peut être condensé à
(a) un noyau aromatique monocyclique choisi entre un noyau benzénique et un noyau hétéroaromatique penta- ou hexagonal contenant jusqu'à trois hétéroatomes choisis entre l'azote, l'oxygène et le soufre ; ou
(b) un noyau hétéroalicyclique penta-, hexa- ou heptagonal contenant jusqu'à trois hétéroatomes choisis entre l'azote, l'oxygène et le soufre.

3. Composé suivant la revendication 1, dans lequel R¹ représente un groupe R^{B}, R^{C} ou R^{D}, chacun facultativement substitué avec un ou plusieurs groupes R⁷, où
R^{B} représente un groupe phényle,
R^{C} représente un système de noyau monocyclique saturé ou partiellement insaturé contenant 5 à 7 atomes dans le noyau, dont au moins l'un est un hétéroatome choisi entre l'azote, l'oxygène et le soufre,
R^{D} représente un groupe furannyle, thiényle, pyrrolyle, pyrazolyle, imidazolyle, isoxazolyle, oxazolyle, isothiazolyle, thiazolyle, oxadiazolyle, pyridyle, pyridazinyle, pyrimidyle ou pyrazinyle, et
R⁷ représente un groupe fluoro, méthyle, éthyle, hydroxy, méthoxy, propoxy ou CONHMe.

4. Composé suivant l'une quelconque des revendications 1 à 3, dans lequel R² représente un atome d'hydrogène ou un groupe méthyle.

5. Composé suivant l'une quelconque des revendications 1 à 4, dans lequel R³ représente un atome d'hydrogène ou un groupe alkyle en C₁ à C₄, qui est facultativement substitué avec un ou plusieurs groupes R⁸, ou bien R³ représente un groupe azétidinyle, pyrrolidinyle ou pipéridinyle, chacun étant facultativement substitué avec un ou plusieurs groupes R⁹, où
R⁸ représente un groupe hydroxy, méthoxy, méthoxyphényle, NH₂, NHMe, NMe₂, NHCO₂^{t}Bu, NMeCO₂^{t}Bu, CO₂H, CONHMe, pyrrolidinyle, pipéridinyle, morpholinyle ou pyrazolyle, les quatre derniers étant facultativement substitués avec un ou plusieurs groupes R⁹, et
R⁹ représente un groupe méthyle ou CO₂^{t}Bu.

6. Composé suivant l'une quelconque des revendications 1 à 5, dans lequel R⁴ représente un atome d'hydrogène, un groupe méthyle ou éthyle.

7. Composé suivant l'une quelconque des revendications 1 à 6, dans lequel -NR³R⁴ forme un groupe R^{F} qui est facultativement substitué avec un ou plusieurs groupes R¹⁰, où
R^{F} est choisi entre des groupes azétidinyle, pyrrolidinyle, pipéridinyle, pipérazinyle, morpholinyle, 3-azabicyclo[3.1.0]hex-3-yle, homopipérazinyle, 2,5-diazabicyclo-[4,3.0]non-2-yle, 3,8-diazabicyclo[3.2.1]oct-3-yle, 3,8-diazabicyclo[3.2.1]oct-8-yle, 1,4-diazabicyclo[4,3.0]non-4-yle et 1,4-diazabicyclo[3.2.2]non-4-yle, et
R¹⁰ représente un groupe halogéno, méthyle, éthyle, isopropyle, hydroxy, méthoxy, NH₂, NHMe, NMe₂, NHCO₂^{t}Bu, CO₂H CO₂^{t}Bu, oxo, benzyle, -CH₂NH₂-, -CH₂NHMe, CH₂NMe₂ ou -CH₂NMeCO₂^{t}Bu.

8. Composé suivant l'une quelconque des revendications 1 à 7, dans lequel
R¹⁵ et R¹⁶ sont choisis chacun indépendamment entre un atome d'hydrogène, des groupes alkyle en C₁ à C₆ facultativement substitué avec R¹⁷, -NR¹⁸R¹⁹, -CO₂R²⁰, -CONR²¹R²², R²³ ou phényle facultativement substitué avec un substituant halogéno, alkyle en C₁ à C₆ ou R¹⁷, cycloalkyle en C₃ à C₇ et R²³, ou bien NR¹⁵R¹⁶ représente un noyau penta- à heptagonal qui peut comprendre facultativement un ou plusieurs hétéroatomes supplémentaires choisis entre l'azote et l'oxygène, et qui peut être en outre facultativement substitué avec un substituant R¹⁷, -CO₂R²⁰, -CONR²¹R²² ou alkyle en C₁ à C₆ facultativement substitué avec R¹⁷ ;
R¹⁷ représente un groupe hydroxy, alkoxy en C₁ à C₆ ou cycloalkyloxy en C₃ à C₇ ;
R²¹ et R²² sont choisis chacun indépendamment entre un atome d'hydrogène, des groupes alkyle en C₁ à C₆ et cycloalkyle en C₃ à C₇, ou bien -NR²¹R²² représente un noyau penta- à octogonal qui peut comprendre facultativement un ou plusieurs hétéroatomes supplémentaires choisis entre l'azote et l'oxygène ; et
R²³ représente un noyau penta- à heptagonal saturé qui comprend au moins un hétéroatome choisi entre l'azote et l'oxygène, noyau qui peut être facultativement substitué avec un ou plusieurs groupes alkyle en C₁ à C₆.

9. Composé suivant l'une quelconque des revendications 1 à 8, dans lequel R⁶ est positionné sur N¹.

10. Composé suivant la revendication 9, dans lequel R⁶ représente un atome d'hydrogène, un groupe méthyle, éthyle, isopropyle, isobutyle, méthoxyéthyle, méthoxypropyle, éthoxyéthyle, éthoxypropyle, propoxyéthyle, 2,2,2-trifluoréthyle, tétrahydrofurannylméthyle, tétrahydropyrannylméthyle, tétrahydropyrannyle ou pyridinylméthyle.

11. Composé suivant la revendication 1, dans lequel R¹ représente un groupe cyclique choisi entre R^{A}, R^{B}, R^{C} et R^{D} chacun étant facultativement substitué avec un ou plusieurs groupes R⁷;
R⁷ représente un groupe halogéno, alkyle en C₁ à C₆, halogénalkyle en C₁ à C₆, OR¹² ou CONR¹²R¹³ ;
R⁸ représente un groupe halogéno, phényle, (alkoxy en C₁ à C₆) phényle, OR¹² , NR¹²R¹³ , NR¹²CO₂R¹⁴ , CO₂R¹², CONR¹²R¹³ , R^{G}
ou R^{H}, les deux derniers étant facultativement substitués avec un ou plusieurs groupes R⁹;
R^{A} représente un groupe cycloalkyle en C₅ à C₇ monocyclique ;
R^{B} représente un groupe phényle ;
R^{C} représente un système de noyau monocyclique saturé contenant 5 à 7 atomes dans le noyau, dont au moins l'un est un hétéroatome choisi entre l'azote, l'oxygène et le soufre ;
R^{D} représente un noyau hétéroaromatique pentagonal contenant un hétéroatome choisi entre l'azote, l'oxygène et le soufre et facultativement jusqu'à deux atomes d'azote supplémentaires dans le noyau, ou un noyau hétéroaromatique hexagonal comprenant 1, 2 ou 3 atomes d'azote ;
R^{E} représente un système de noyau monocyclique saturé contenant 3 à 7 atomes dans le noyau, contenant un atome d'azote ;
R^{F} représente un système de noyau saturé monocyclique ou, lorsqu'il existe un nombre approprié d'atomes dans le noyau, polycyclique contenant 3 à 10 atomes dans le noyau, contenant au moins un atome d'azote, et facultativement un autre atome choisi entre l'oxygène et le soufre ;
R^{G} représente un système de noyau monocyclique saturé contenant 3 à 7 atomes dans le noyau, dont au moins l'un est un hétéroatome choisi entre l'azote, l'oxygène et le soufre ; et
R^{H} représente un noyau hétéroaromatique penta- ou hexagonal contenant jusqu'à deux atomes d'azote ;
R³ représente un atome d'hydrogène, un groupe alkyle en C₁ à C₄ qui est facultativement substitué avec un ou plusieurs groupes R⁸, ou bien un groupe R^{E} qui est facultativement substitué avec un ou plusieurs groupes R⁹ ;
R⁴ représente un atome d'hydrogène, un groupe alkyle en C₁ à C₆ ou halogénalkyle en C₁ à C₆ ;
ou bien -NR³R⁴ forme un groupe R^{F}, qui est facultativement substitué avec un ou plusieurs groupes R¹⁰ ;
R⁶ représente un groupe alkyle en C₁ à C₄ ou halogénalkyle en C₁ à C₄, chacun étant facultativement substitué avec un groupe alkoxy en C₁ à C₄, halogénalkoxy en C₁ à C₄ ou un groupe cyclique choisi entre R^{J}, R^{L} et R^{M}, ou bien R⁶ représente un groupe R^{N} ou un atome d'hydrogène ;
R^{J} représente un groupe cyclopropyle ou cyclobutyle ;
R^{L} et R^{N} représentent chacun indépendamment un système de noyau saturé monocyclique contenant 5 ou 6 atomes dans le noyau, dont au moins l'un est un hétéroatome choisi entre l'azote, l'oxygène et le soufre ;
R^{M} représente un noyau hétéroaromatique penta- ou hexagonal contenant un hétéroatome choisi entre l'azote, l'oxygène et le soufre ; et
Y représente une liaison covalente.

12. Composé suivant la revendication 1, choisi entre les suivants:
1-(2-éthoxyéthyl)-*N*-éthyl-5-(éthylamino)-7-(4-méthylpyridine-2-ylamino)-1*H*-pyrazolo[4,3-d]pyrimidine-3-carboxamide,
5-(diméthylamino)-1-(2-éthoxyéthyl) -N-méthyl-7- (4-méthyl-pyridine-2-ylamino)-1*H*-pyrazolo[4,3-*d*]pyrimidine-3-carboxamide,
5-(diméthylamino)-1-(2-éthoxyéthyl)-*N*-(2-(méthylamino)-éthyl)-7-(4-méthylpyridine-2-ylamino)-1H-pyrazolo[4,3-d]pyrimidine-3-carboxamide,
5-(diméthylamino)-*N*- (2- (diméthylamino)éthyl) -1- (2-éthoxyéthyl)-7-(4-méthylpyridine-2-ylamino)-1*H*-pyrazolo[4,3-*d*]-pyrimidine-3-carboxamide,
5- (diméthylamino)-1-(2-éthoxyéthyl)-7-(4-méthylpyridine-2-ylamino)-*N*-(pipéridine-4-yl)-1*H*-pyrazolo[4,3-*d*]pyrimidine-3-carboxamide,
5-(diméthylamino)-1-(2-éthoxyéthyl)-*N*-(2-(méthoxyéthyl)-7-(4-méthylpyridine-2-ylamino)-1*H*-pyrazolo[4,3-d]pyrimidine-3-carboxamide,
acide (2*R*)-2-{[5-(diméthylamino)-1-(2-éthoxyéthyl)-7-(4-méthylpyridine-2-ylamino)-1*H*-pyrazolo[4,3-*d*]pyrimidine-3-carbonyl]amino}propionique,
acide 3-{[5-(diméthylamino)-1-(2-éthoxyéthyl)-7-(4-méthyl-pyridine-2-ylamino)-1*H*-pyrazolo[4,3-d]pyrimidine-3-carbonyl]-amino}propionique,
1-(2-éthoxyéthyl)-*N*-méthyl-7-(4-méthylpyridine-2-yl-amino)-5-(pipérazine-1-yl)-1*H*-pyrazolo[4,3-d]pyrimidine-3-carboxamide,
1-(2-éthoxyéthyl)-*N*-méthyl-5-((3*R*)-3-méthylpipérazine-1-yl)-7-(4-méthylpyridine-2-ylamino)-1*H*-pyrazolo[4,3-*d*]-pyrimidine-3-carboxamide,
1-(2-éthoxyéthyl)-*N*-éthyl-5-((3R)-3-méthylpipérazine-1-yl)-7-(4-méthylpyridine-2-ylamino)-1*H*-pyrazolo[4,3-*d*]-pyrimidine-3-carboxamide,
1-(2-éthoxyéthyl)-5-(éthylamino)-*N*-méthyl-7-(4-méthyl-pyridine-2-ylamino)-1*H*-pyrazolo[4,3-*d*]pyrimidine-3-carboxamide,
1-(2-éthoxyéthyl)-*N*-(2-méthoxyéthyl)-5-(méthylamino)-7-(4-méthylpyridine-2-ylamino)-1*H*-pyrazolo [4,3-*d*] pyrimidine-3-carboxamide,
5-(diméthylamino)-1-(2-éthoxyéthyl)-*N-*(2-hydroxyéthyl)-7-(4-méthylpyridine-2-ylamino)-1*H*-pyrazolo [4,3-*d*] pyrimidine-3-carboxamide,
1-(2-éthoxyéthyl)-5-(éthylamino) -N-(2-méthoxyéthyl)-7-(4-méthylpyridine-2-ylamino)-1*H*-pyrazolo[4,3-*d*]pyrimidine-3-carboxamide,
1-(2-éthoxyéthyl)-5-(N-(2-hydroxyéthyl)-*N*-méthylamino)-*N*-méthyl-7-(4-méthylpyridine-2-ylamino)-1*H*-pyrazolo[4,3-*d*]-pyrimidine-3-carboxamide,
1-(2-éthoxyéthyl)-5- ((2-méthoxyéthyl) amino) -N-méthyl-7-(4-méthylpyridine-2-ylamino) -1*H*-pyrazolo[4,3-*d*]pyrimidine-3-carboxamide,
7-(cyclohexylamino)-1-(2-éthoxyéthyl)-*N*-méthyl-5- ((3R) -3-méthylpipérazine-1-yl)-1*H*-pyrazolo[4,3-*d*]pyrimidine-3-carboxamide, et
1-(2-éthoxyéthyl)-*N*-méthyl-5-[N-méthyl-N-((3*S*)-1-méthyl-pyrrolidine-3-yl)amino]-7-(4-méthylpyridine-2-ylamino)-1*H-*pyrazolo[4,3-d]pyrimidine-3-carboxamide,
et ses formes tautomères, ainsi que les sels et produits de solvatation pharmaceutiquement acceptables dudit composé ou desdites formes tautomères.

13. Composition pharmaceutique comprenant un composé de formule (I) suivant l'une quelconque des revendications 1 à 12, ou un de ses sels ou produits de solvatation pharmaceutiquement acceptables, et un diluant ou support pharmaceutiquement acceptable.

14. Composé de formule (I) suivant l'une quelconque des revendications 1 à 12, ou un de ses sels ou produits de solvatation pharmaceutiquement acceptables, destiné à être utilisé comme médicament pour le traitement d'une maladie ou affection choisie entre l'hypertension (comprenant l'hypertension essentielle, l'hypertension pulmonaire, l'hypertension secondaire, l'hypertension systolique isolée, l'hypertension associée au diabète, l'hypertension associée à l'athérosclérose et l'hypertension réno-vasculaire), l'insuffisance cardiaque congestive, l'angine de poitrine (comprenant l'angor stable, l'angor instable et l'angor variant (de Prinzmetal)), un ictus, une maladie des artères coronaires, l'insuffisance cardiaque congestive, des états de patence réduite des vaisseaux sanguins (tels que l'angioplastie coronarienne post-percutanée), une maladie vasculaire périphérique, l'athérosclérose, la tolérance induite par les nitrates, la tolérance aux nitrates, le diabète, la tolérance entravée au glucose, le syndrome métabolique, l'obésité, un dysfonctionnement sexuel (comprenant un trouble de l'érection masculine, l'impuissance, un trouble de l'éveil sexuel féminin, un dysfonctionnement clitoridien, un trouble de désir sexuel hypoactif féminin, un trouble de douleur sexuelle féminine, un dysfonctionnement orgasmique sexuel, un dysfonctionnement sexuel dû à une lésion de la moelle épinière), l'accouchement prématuré, la prééclampsie, la dysménorrhée, le syndrome d'ovaires polykystiques, l'hyperplasie prostatique bénigne, une obstruction de la sortie de la vessie, l'incontinence, une maladie pulmonaire obstructive chronique, l'insuffisance respiratoire aiguë, la bronchite, l'asthme chronique, l'asthme allergique, la rhinite allergique, des troubles de la motilité intestinale (comprenant le syndrome du côlon irritable), le syndrome de Kawasaki, la sclérose en plaques, la maladie d'Alzheimer, le psoriasis, la nécrose cutanée, la cicatrisation, la fibrose, la douleur (en particulier la douleur neuropathique), le cancer, une métastase, la calvitie, l'oesophage en casse-noix, la fissure anale et les hémorroïdes.

15. Utilisation suivant la revendication 14, dans laquelle la maladie ou l'affection est choisie entre l'hypertension essentielle, l'hypertension pulmonaire, l'hypertension secondaire, l'hypertension systolique isolée, l'hypertension associée au diabète, l'hypertension associée à l'athérosclérose et l'hypertension réno-vasculaire.
